(19) Europäisches Patentamt
European Patent Office
Office européen des brevets

(11) **EP 4 390 580 A1**

(12) **EUROPEAN PATENT APPLICATION**
published in accordance with Art. 153(4) EPC

(43) Date of publication:
**26.06.2024 Bulletin 2024/26**

(21) Application number: **22885298.4**

(22) Date of filing: **11.08.2022**

(51) International Patent Classification (IPC):
**G05B 15/02** (2006.01)   **G05B 19/418** (2006.01)

(52) Cooperative Patent Classification (CPC):
**G05B 15/02; G05B 19/418**

(86) International application number:
**PCT/CN2022/111863**

(87) International publication number:
**WO 2023/071398 (04.05.2023 Gazette 2023/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.10.2021 CN 202111243352**

(71) Applicant: **Huawei Technologies Co., Ltd.
Shenzhen, Guangdong 518129 (CN)**

(72) Inventors:
• **DONG, Wei
Shenzhen, Guangdong 518129 (CN)**
• **XU, Haowei
Shenzhen, Guangdong 518129 (CN)**
• **LIU, Zhen
Shenzhen, Guangdong 518129 (CN)**
• **GAO, Xiaoqiang
Shenzhen, Guangdong 518129 (CN)**

(74) Representative: **Körber, Martin Hans
Mitscherlich PartmbB
Patent- und Rechtsanwälte
Karlstraße 7
80333 München (DE)**

(54) **AUTOMATIC CONTROL METHOD BASED ON HUMAN BODY PERCEPTION, ELECTRONIC DEVICE, AND SYSTEM**

(57)   A human sensing-based automatic control method, an electronic device, and a system are provided, so that when a user approaches an IoT device, the IoT device automatically performs an operation, to improve user experience. The system includes a hub device (200), a first electronic device (100), and a second electronic device (300). The first electronic device (100) includes an ultra-wide band module (150) and a millimeter-wave radar module (160), and is configured to measure a human body position and a position of the second electronic device (300) in a house. The hub device (200) determines, based on the human body position and the position of the second electronic device (300) that are measured by the first electronic device (100), a most suitable second electronic device (300) for performing functions such as alarm clock ringing, smart doorbell reminding or incoming call notification, and answering a call from a user in the house. In addition, during registration and control of the IoT device, the hub device (200) further automatically fills room information for the IoT device based on the human body position and the position of the second electronic device (300) that are measured by the first electronic device (100), and filters, during control, another IoT device in a room in which a control device is located.

FIG. 30

**Description**

This application claims priority to Chinese Patent Application No. 202111243352.6, filed with the China National Intellectual Property Administration on October 25, 2021 and entitled "HUMAN SENSING-BASED AUTOMATIC CONTROL METHOD, ELECTRONIC DEVICE, AND SYSTEM", which is incorporated herein by reference in its entirety.

**TECHNICAL FIELD**

This application relates to the field of automatic control, and in particular, to a human sensing-based automatic control method, an electronic device, and a system.

**BACKGROUND**

With rapid development of smart homes, more users install internet of things (Internet of Things, IoT) devices (which may also be referred to as IoT devices, smart devices, or the like) in places such as homes or offices, and even install IoT devices throughout a house (which may also be referred to as whole-house intelligence, an all-in-one smart home, a smart manager, or the like). The users can experience convenience brought by the IoT devices. However, in the conventional technology, the user needs to carry a mobile device (for example, a smartphone or a wearable device) to implement automatic control of the IoT device through communication and sensing between the mobile device and the IoT device, and the like. The user sometimes forgets to carry the mobile device in places such as the home or the office, and cannot implement automatic control of the IoT device. As a result, inconvenience is brought to the user, and user experience is affected.

**SUMMARY**

Embodiments of this application provide a human sensing-based automatic control method, an electronic device, and a system, so that when a user approaches an IoT device, the IoT device automatically performs an operation, to improve user experience.
The following technical solutions are used in embodiments of this application, to achieve the foregoing objective.
According to a first aspect, this application provides a human sensing-based distributed alarm clock system, including a hub device, a first electronic device, and R second electronic devices. Any two of the hub device, the first electronic device, and any one of the R second electronic devices communicate in a wired communication or wireless communication manner. The first electronic device includes an ultra-wide band module and a millimeter-wave radar module, and is configured to measure a human body position and positions of the R second electronic devices. The R second electronic devices include a first device and a second device. R is a positive integer greater than or equal to 1.
The first device sends alarm start information of a first alarm clock to the hub device, and the alarm start information includes an alarm start moment. After receiving the alarm start information of the first alarm clock, the hub device determines the second device from the R second electronic devices based on a human body position that is measured by the first electronic device within first preset duration before the alarm start moment of the first alarm clock and the positions of the R second electronic devices. The hub device sends, to the second device, a first message for performing the first alarm clock. The second device performs a task of the first alarm clock in response to receiving the first message.
It can be learned that, for a same alarm clock (for example, the first alarm clock) set by a user, if the user is located at different positions every day or within different time periods of a day, the hub device may indicate, based on a current position, different devices to perform an alarm clock task, to provide a short-distance reminder for the user. In addition, the user does not need to be required to operate/wear a specific device, to bring immersive alarm clock experience to the user.
In addition, if one or some devices are offline in a whole-house scenario, the hub device may alternatively select another online device to perform an alarm clock task, to avoid a case in which the alarm clock task cannot be performed because an individual device is offline.
According to the first aspect, that the first device sends alarm start information of a first alarm clock to the hub device includes: The first device sends the alarm start information of the first alarm clock to the hub device within second preset duration before the alarm start moment of the first alarm clock.
To be specific, in some embodiments of this application, the first device is responsible for alarm start triggering logic, that is, detecting whether an alarm start condition of an alarm clock is met. The alarm start condition of the alarm clock is, for example, that a current time point is an alarm start moment or is close to an alarm start moment. In addition, when detecting that the alarm start condition of the alarm clock is met, the first device reports alarm start information of the alarm clock to the hub device. In this case, when the hub device receives the alarm start information sent by the first device, the current time point is already the alarm start moment or is close to the alarm start moment. In this case, after determining the second device, the hub device directly indicates the second device to perform an alarm clock task.

In some other embodiments of this application, the first device is not responsible for alarm start triggering logic, and the hub device is responsible for the alarm start triggering logic. In this case, when detecting that an alarm start condition of an alarm clock is met, for example, after a current time point is an alarm start moment or is close to an alarm start moment, the hub device indicates the second device to perform an alarm clock task.

According to the first aspect or any implementation of the first aspect, the alarm start information further includes ringing duration. The hub device sends, to the second device after the ringing duration starting after the hub device sends, to the second device, the first message for performing the first alarm clock, a second message for stopping performing the first alarm clock. The second device stops, in response to receiving the second message, performing the task of the first alarm clock.

To be specific, the hub device is responsible for controlling the ringing duration of the first alarm clock, and when the ringing duration expires, the hub device indicates the second device to disable ringing.

According to the first aspect or any implementation of the first aspect, when the second device performs the task of the first alarm clock, the second device receives a first operation of disabling the first alarm clock by the user. The second device stops, in response to receiving the first operation, performing the task of the first alarm clock.

To be specific, the user can directly disable the alarm clock on a device (namely, the second device) that performs the alarm clock task. The device that performs the alarm clock task is located near the user, and therefore this helps the user quickly and conveniently disable the alarm clock, thereby not only reminding the user but also avoiding excessively disturbing the user. In other words, a distributed alarm clock can effectively balance a function of reminding the user and do-not-disturb for the user, to improve experience of using the alarm clock by the user.

According to the first aspect or any implementation of the first aspect, after stopping performing the task of the first alarm clock, the second device sends, to the hub device, a third message for disabling the first alarm clock.

It can be learned that an operation of disabling the alarm clock by the user is also reported to the hub device, or reported to the first device by using the hub device, so that the hub device or the first device updates a status of the alarm clock in real time.

According to the first aspect or any implementation of the first aspect, the hub device updates an execution status of the first alarm clock in response to receiving the third message.

According to the first aspect or any implementation of the first aspect, the alarm start information further includes a re-ringing interval. The R second electronic devices further include a third device. When the second device performs the task of the first alarm clock, the second device receives a second operation of performing alarm clock re-ringing by the user. In response to receiving the second operation, the second device stops performing the task of the first alarm clock, and sends a fourth message for alarm clock re-ringing to the hub device. After receiving the fourth message, the hub device calculates a re-ringing moment of the first alarm clock based on the re-ringing interval. The hub device determines the third device from the R second electronic devices based on a human body position that is measured by the first electronic device within first preset duration before the re-ringing moment of the first alarm clock and the positions of the R second electronic devices. The hub device sends, to the third device, a fifth message for performing the first alarm clock. The third device performs the task of the first alarm clock in response to receiving the fifth message.

To be specific, the user can directly perform a re-ringing operation on a device (namely, the second device) that performs the alarm clock task. The device that performs the alarm clock task is located near the user, and therefore this helps the user quickly and conveniently disable the alarm clock. In addition, when the alarm clock task needs to be performed a plurality of times for the same alarm clock, according to a solution of a distributed alarm clock provided in this embodiment of this application, different devices are intelligently switched, based on a change of a position of the user, to perform the alarm clock task each time, so as to provide a short-distance reminder for the user at any time. In addition, in a process of switching the devices to perform the alarm clock task, the user does not need to be required to operate/wear the specific device, to bring immersive alarm clock experience to the user.

According to the first aspect or any implementation of the first aspect, the alarm start information further includes ringing duration. The R second electronic devices further include a fourth device. The hub device determines, based on a real-time human body position that is measured by the first electronic device and the positions of the R second electronic devices, the fourth device from the R second electronic devices within the ringing duration starting after the hub device sends, to the second device, the first message for performing the first alarm clock. The hub device sends, to the second device, a sixth message for stopping performing the first alarm clock, and sends, to the fourth device, a seventh message for performing the first alarm clock. The second device stops, in response to receiving the sixth message, performing the task of the first alarm clock. The fourth device performs the task of the first alarm clock in response to receiving the seventh message.

To be specific, when the alarm clock task is performed once for the same alarm clock, the different devices can be also switched, based on a position of the user, to perform the current alarm clock task. It can be learned that, in a process of switching the alarm clock, no operation of the user is required, and the hub device intelligently switches the different devices, based on a movement track of a human body, to continue the alarm clock task, to bring immersive alarm clock experience to the user.

According to the first aspect or any implementation of the first aspect, the hub device sends, to the fourth device after the ringing duration starting after the first message for performing the first alarm clock is sent to the second device, an eighth message for stopping performing the first alarm clock. The fourth device stops, in response to receiving the eighth message, performing the task of the first alarm clock.

According to a second aspect, this application provides a human sensing-based distributed alarm clock execution method, applied to a hub device. The hub device separately communicates with a first electronic device and R second electronic devices in a wired communication or wireless communication manner. The first electronic device includes an ultra-wide band module and a millimeter-wave radar module, and is configured to measure a human body position and positions of the R second electronic devices. The R second electronic devices include a first device and a second device. R is a positive integer greater than or equal to 1. The hub device receives alarm start information of a first alarm clock sent by the first device, and the alarm start information includes an alarm start moment. The hub device determines the second device from the R second electronic devices based on a human body position that is measured by the first electronic device within first preset duration before the alarm start moment of the first alarm clock and the positions of the R second electronic devices. The hub device sends, to the second device, a first message for performing the first alarm clock.

According to the second aspect, the ringing information further includes ringing duration. The hub device sends, to the second device after the ringing duration starting after the hub device sends, to the second device, the first message for performing the first alarm clock, a second message for stopping performing the first alarm clock.

According to the second aspect or any implementation of the second aspect, the hub device receives a third message that is for disabling the first alarm clock and that is sent by the second device, and the hub device updates an execution status of the first alarm clock.

According to the second aspect or any implementation of the second aspect, the alarm start information further includes a re-ringing interval. The R second electronic devices further include a third device. The hub device receives a fourth message for alarm clock re-ringing sent by the second device. The hub device calculates a re-ringing moment of the first alarm clock based on the re-ringing interval. The hub device determines the third device from the R second electronic devices based on a human body position that is measured by the first electronic device within first preset duration before the re-ringing moment of the first alarm clock and the positions of the R second electronic devices. The hub device sends, to the third device, a fifth message for performing the first alarm clock.

According to the second aspect or any implementation of the second aspect, the ringing information further includes ringing duration. The R second electronic devices further include a fourth device. The hub device determines, based on a real-time human body position that is measured by the first electronic device and the positions of the R second electronic devices, the fourth device from the R second electronic devices within the ringing duration starting after the hub device sends, to the second device, the first message for performing the first alarm clock. The hub device sends, to the second device, a sixth message for stopping performing the first alarm clock, and sends, to the fourth device, a seventh message for performing the first alarm clock.

According to the second aspect or any implementation of the second aspect, the hub device sends, to the fourth device after the ringing duration starting after the first message for performing the first alarm clock is sent to the second device, an eighth message for stopping performing the first alarm clock.

For technical effects that can be achieved by the second aspect and any implementation of the second aspect, refer to the descriptions of the technical effects in the first aspect and any implementation of the first aspect. Details are not described herein again.

According to a third aspect, this application provides a human sensing-based device registration system, including a hub device, a first electronic device, R second electronic devices, and a server. The first electronic device includes an ultra-wide band module and a millimeter-wave radar module, and is configured to measure a human body position and positions of the R second electronic devices. The R second electronic devices include a first device and a second device. R is a positive integer greater than or equal to 1. The first device receives a first operation of adding the second device. The first device sends network configuration information to the second device, and sends a first registration request for the second device to the hub device. After receiving the first registration request for the second device, the hub device determines room information or area information of the second device based on the human body position and the positions of the R second electronic devices that are measured by the first electronic device. The hub device sends a second registration request for the second device to the server. The second registration request includes the room information or the area information of the second device. The server registers the second device based on the second registration request.

It may be understood that, if network configuration and registration are performed on the second device by using the hub device, the second device and the hub device are devices in a same home, and therefore the hub device can determine home information of the hub device as home information of the second device.

It can be learned that, according to the device network configuration and registration method provided in this embodiment of this application, home information and room information can be automatically added to a device on which network configuration is performed, and a user does not need to manually enter the room information and the room information. This simplifies an operation procedure of the user, and shortens operation duration of the user, thereby improving network configuration efficiency of the device, and improving use experience of the device.

In addition, the home information and the room information are automatically added to the device on which network configuration is performed. This can also avoid a case in which the user adds an incorrect home or room because the user maintains devices in a plurality of homes or the user maintains a large quantity of devices, thereby improving accuracy of adding the home information and the room information to the device.

According to the third aspect, that the hub device determines room information or area information of the second device based on the human body position and the positions of the R second electronic devices that are measured by the first electronic device includes: The hub device determines, as the room information or the area information of the second device, a room or an area in which the human body position measured by the first electronic device is located; or the first device includes an ultra-wide band module, and the hub device determines the room information or the area information of the second device based on a room or an area in which a position that is of the first device and that is measured by the first electronic device is located.

In some examples of this embodiment, the hub device detects a human body position in a whole house based on a millimeter-wave radar in a whole-house sensing system, and determines the human body position as a position of a user of the first device. It may be understood that, when the user of the first device performs network configuration and registration on the second device, the user of the first device is usually located near the second device, that is, the user of the first device and the second device are located in a same room. Therefore, the hub device obtains information about a room in which the user of the first device is located, namely, the room information of the second device.

In some other examples of this embodiment, when the hub device detects, based on a whole-house sensing system, that positions of a plurality of human bodies are included in a whole-house scenario, that is, detects a plurality of users, the hub device may alternatively recognize a user of the first device based on a feature of each detected user, obtain information about a room in which the user of the first device is located, and determine the information as the room information of the second device.

In some other embodiments, the hub device may detect, based on a UWB system in a whole-house sensing system, information about a room in which the first device is located, and determine the room information of the first device as the room information of the second device.

According to a fourth aspect, this application provides a human sensing-based device registration method, applied to a hub device. The hub device separately communicates with a first electronic device, R second electronic devices, and a server in a wired communication or wireless communication manner. The first electronic device includes an ultra-wide band module and a millimeter-wave radar module, and is configured to measure a human body position and positions of the R second electronic devices. The R second electronic devices include a first device and a second device. R is a positive integer greater than or equal to 1. The hub device receives a first registration request for the second device sent by the first device. The hub device determines room information or area information of the second device based on the human body position and the positions of the R second electronic devices that are measured by the first electronic device. The hub device sends a second registration request for the second device to the server. The second registration request includes the room information or the area information of the second device.

According to the fourth aspect, that the hub device determines room information or area information of the second device based on the human body position and the positions of the R second electronic devices that are measured by the first electronic device includes: The hub device determines, as the room information or the area information of the second device, a room or an area in which the human body position measured by the first electronic device is located; or the first device includes an ultra-wide band module, and the hub device determines the room information or the area information of the second device based on a room or an area in which a position that is of the first device and that is measured by the first electronic device is located.

For technical effects that can be achieved by the fourth aspect and any implementation of the fourth aspect, refer to the descriptions of the technical effects in the third aspect and any implementation of the third aspect. Details are not described herein again.

According to a fifth aspect, this application provides a human sensing-based device control system, including a hub device, a first electronic device, R second electronic devices, and a server. The first electronic device includes an ultra-wide band module and a millimeter-wave radar module, and is configured to measure a human body position and positions of the R second electronic devices. The R second electronic devices include a first device. R is a positive integer greater than or equal to 1. The first device sends a first request to the hub device. The first request is for searching for device list information controlled by the first device. After receiving the first request, the hub device determines room information of the first device based on the human body position and the positions of the R second electronic devices that are measured by the first electronic device. The hub device sends a second request to the server. The second request is for requesting device list information of a room in which the first device is located. After receiving the second request, the server returns, to the hub device, the device list information of the room in which the first device is located. After receiving the device list information that is of the room in which the first device is located and that is returned by the server, the first device displays a device list of the room in which the first device is located.

In this embodiment of this application, the hub device automatically obtains, through screening based on a whole-house sensing system, a device in the room in which the first device (namely, a control device) is located, and therefore a procedure of manually selecting the room by the user does not need to be performed. It may be understood that, when a user wants to operate a device, the user and the device are usually located in a same room. Therefore, in this embodiment of this application, the first device automatically obtains, through screening, the device in the room in which the first device is located. This satisfies an operation intention of the user, simplifies an operation procedure of the user, and improves user experience.

According to the fifth aspect, before the first device sends the first request to the hub device, the first device receives a first operation of starting a smart home application or enabling a device search function by the user.

According to a sixth aspect, this application provides a human sensing-based device control method, applied to a hub device. The hub device separately communicates with a first electronic device, R second electronic devices, and a server in a wired communication or wireless communication manner. The first electronic device includes an ultra-wide band module and a millimeter-wave radar module, and is configured to measure a human body position and positions of the R second electronic devices. The R second electronic devices include a first device. R is a positive integer greater than or equal to 1. The hub device receives a first request sent by the first device. The first request is for searching for device list information controlled by the first device. The hub device determines room information of the first device based on the human body position and the positions of the R second electronic devices that are measured by the first electronic device. The hub device sends a second request to the server. The second request is for requesting device list information of a room in which the first device is located. The hub device receives the device list information that is of the room in which the first device is located and that is returned by the server. The hub device returns, to the first device, the device list information of the room in which the first device is located.

For technical effects that can be achieved in the sixth aspect, refer to the descriptions of the technical effects in the fifth aspect and any implementation of the fifth aspect. Details are not described herein again.

According to a seventh aspect, this application provides a human sensing-based intelligent reminding system, including a hub device, a first electronic device, and R second electronic devices. The first electronic device includes an ultra-wide band module and a millimeter-wave radar module, and is configured to measure a human body position and positions of the R second electronic devices. The R second electronic devices include a first device and a second device. R is a positive integer greater than or equal to 1. The first device sends a first message of a first reminder to the hub device. After receiving the first message of the first reminder, the hub device determines the second device from the R second electronic devices based on the human body position and the positions of the R second electronic devices that are measured by the first electronic device. The hub device sends a second message of the first reminder to the second device. The second device performs the first reminder after receiving the second message.

It can be learned that, when the first reminder is triggered on the first device, the hub device detects a human body position based on a whole-house sensing system, and then selects an appropriate device, for example, the second device, based on the detected human body position, to collaborate with the first device. It can be learned that, in one aspect, the second device is selected based on the detected human body position, and therefore there is a user located near the second device. In this case, the user can receive the first reminder in time, which also facilitates processing by the user. In another aspect, the hub device selects the second device from all devices in a whole house. Therefore, when a device in the whole house is disconnected from the hub device or a signal is poor, the hub device may select another device to collaborate with the first device.

According to the seventh aspect, before the first device sends the first message of the first reminder to the hub device, the first device receives an operation of triggering a doorbell by the user. The first message of the first reminder is for triggering a doorbell reminder.

According to the seventh aspect or any implementation of the seventh aspect, that the first device sends a first message of a first reminder to the hub device includes: The first device sends the first message of the first reminder to the hub device by using a server. The first message of the first reminder is for calling any one of the R second electronic devices.

In conclusion, compared with that in the conventional technology, when a device (for example, a large-screen device or a smart speaker) in a home network receives an incoming call, only the device rings. In this case, if the user is far away from the device, the user may miss the reminder, or the user needs to quickly move near the device to process the incoming call, resulting in poor user experience. In addition, if the device is offline, the current call fails.

However, in this embodiment of this application, when receiving a call to the device in the home network, the hub device can determine, based on the human body position detected by the whole-house sensing system, that there is no person around the called device, and may choose to switch the call to another appropriate device, that is located near a person, for ringing and the call with the first device. It can be learned that, in one aspect, the second device is selected based on the detected human body position, and therefore there is a user located near the second device. In this case, the user can receive the ringing reminder in time, which also facilitates processing by the user. In another aspect, the hub device selects the second device from all the devices in the whole house. Therefore, when the device in the whole house is disconnected from the hub device or the signal is poor, the hub device may select the another device for ringing and the call with the first device.

According to the seventh aspect or any implementation of the seventh aspect, that the hub device determines the second device from the R second electronic devices based on the human body position and the positions of the R second electronic devices that are measured by the first electronic device includes: The hub device determines, as the second device from the R second electronic devices based on the human body position and the positions of the R second electronic devices that are measured by the first electronic device, a device that is closest to the human body position; or when there are a plurality of detected human body positions, the hub device determines, as the second device from the R second electronic devices based on the human body positions and the positions of the R second electronic devices that are measured by the first electronic device, a device that is closest to a central position of the plurality of human body positions; or the hub device determines, as the second device from the R second electronic devices based on the human body position and the positions of the R second electronic devices that are measured by the first electronic device, a device that is in a room in which there is a person and that is closest to the first device; or the hub device determines, as the second device from the R second electronic devices based on the human body position and the positions of the R second electronic devices that are measured by the first electronic device, a device in a room in which no person rests.

In this way, several methods for determining the second device from the R second electronic devices are provided.

According to an eighth aspect, this application provides a human sensing-based intelligent reminding method, applied to a hub device. The hub device separately communicates with a first electronic device and R second electronic devices in a wired communication or wireless communication manner. The first electronic device includes an ultra-wide band module and a millimeter-wave radar module, and is configured to measure a human body position and positions of the R second electronic devices. The R second electronic devices include a first device and a second device. R is a positive integer greater than or equal to 1. After receiving a first message that is of a first reminder and that is sent by the first device, the hub device determines the second device from the R second electronic devices based on the human body position and the positions of the R second electronic devices that are measured by the first electronic device. The hub device sends a second message of the first reminder to the second device. The second message indicates the second device to perform the first reminder.

According to the eighth aspect, the first message of the first reminder is for triggering a doorbell reminder.

According to the eighth aspect or any implementation of the eighth aspect, that the hub device receives the first message that is of the first reminder and that is sent by the first device includes: The hub device receives the first message that is of the first reminder and that is sent by the first device by using a server. The first message of the first reminder is for calling any one of the R second electronic devices.

According to the eighth aspect or any implementation of the eighth aspect, that the hub device determines the second device from the R second electronic devices based on the human body position and the positions of the R second electronic devices that are measured by the first electronic device includes: The hub device determines, as the second device from the R second electronic devices based on the human body position and the positions of the R second electronic devices that are measured by the first electronic device, a device that is closest to the human body position; or when there are a plurality of detected human body positions, the hub device determines, as the second device from the R second electronic devices based on the human body positions and the positions of the R second electronic devices that are measured by the first electronic device, a device that is closest to a central position of the plurality of human body positions; or the hub device determines, as the second device from the R second electronic devices based on the human body position and the positions of the R second electronic devices that are measured by the first electronic device, a device that is in a room in which there is a person and that is closest to the first device; or the hub device determines, as the second device from the R second electronic devices based on the human body position and the positions of the R second electronic devices that are measured by the first electronic device, a device in a room in which no person rests.

For technical effects that can be achieved by the eighth aspect or any implementation of the eighth aspect, refer to the descriptions of the technical effects in the seventh aspect or any implementation of the seventh aspect. Details are not described herein again.

According to a ninth aspect, this application provides a human sensing-based indoor calling system, including a hub device, a first electronic device, R second electronic devices, and a server. The first electronic device includes an ultra-wide band module and a millimeter-wave radar module, and is configured to measure a human body position and positions of the R second electronic devices. The R second electronic devices include a first device and a second device. R is a positive integer greater than or equal to 1. The first device receives a first speech of a first user, and the first speech is for calling a second user. The first device sends the first speech of the first user to the server by using the hub device. The server performs speech semantic recognition on the first speech of the first user, and recognizes the first speech as a first calling instruction. The server sends the first calling instruction to the hub device. After receiving the first calling instruction, the hub device determines, as the second device from the R second electronic devices based on the human body position and the positions of the R second electronic devices that are measured by the first electronic device, a device located near the second user. The hub device forwards, to the second device, a second speech or video of the first user sent by the first device, and forwards, to the first device, a third speech or video of the second user sent by the second device.

In conclusion, when a user calls another user in a home, the user can send a speech command to the hub device by using a nearby first device having a speech function. Then, the hub device can recognize, based on a whole-house sensing system, a position of the called user, and automatically determine, based on a position of another user, a nearby second device having the speech function. The second device and the first device jointly perform a local call between family members in the home. It can be learned that, in the technical solution provided in this embodiment of this application, the local call between the family members is no longer limited to a specific device on which social software or call software is installed. In addition, the hub device selects a nearby device for the called user based on the whole-house sensing system, to prevent the called user from missing the current call, and help the called user directly answer the call with the calling user. In addition, according to the technical solution provided in this embodiment of this application, the local call between the family members is implemented, and the server is prevented from forwarding multimedia data. This improves transmission efficiency of the multimedia data, and improves call experience of the user.

According to the ninth aspect, when detecting that a call end condition is met, the hub device sends a call end message to the first device and/or the second device. The call end condition includes that the call end message sent by the first device or the second device is received or the hub device receives no new speech or video after preset duration starting after a speech or a video of the user is forwarded.

It can be learned that either party in a call can actively end the call, or the hub device can actively end a call.

According to the ninth aspect or any implementation of the ninth aspect, when the first speech does not include information about the second user, that the hub device determines, from the R second electronic devices based on the human body position and the positions of the R second electronic devices that are measured by the first electronic device, that the second user is located near the second device includes: The hub device determines, based on the human body position that is measured by the first electronic device, the detected human body position as a position of the second user, and determines, as the second user from the R second electronic devices based on the positions of the R second electronic devices, a device located near the second user.

In some embodiments, when a calling instruction received by the hub device includes no identifier of a specific called family member (that is, does not include information about the second user), the hub device may first obtain one or more human body positions in a whole house based on the whole-house sensing system. Then, a device having the speech function near the one or more human body positions is recognized as the second device based on a UWB system. When the one or more human body positions are located in different rooms, one second device is determined in each room. When two or more human body positions in the one or more human body positions are located in a same room, one second device is determined in the room. For example, a device closest to one or more human body positions may be selected and determined as the second device. Currently, the second device may alternatively be determined according to another policy. In other words, a specific method for selecting, as the second device, one device from a plurality of devices that have a speech function in a room is not specifically limited.

According to a tenth aspect, a human sensing-based indoor calling method is provided, applied to a hub device. The hub device separately communicates with a first electronic device, R second electronic devices, and a server in a wired communication or wireless communication manner. The first electronic device includes an ultra-wide band module and a millimeter-wave radar module, and is configured to measure a human body position and positions of the R second electronic devices. The R second electronic devices include a first device and a second device. R is a positive integer greater than or equal to 1. The hub device receives a first calling instruction sent by the server. The first calling instruction is obtained by the server based on a first speech sent by the first device. The first speech is used by a first user to call a second user. The hub device determines, as the second device from the R second electronic devices based on the human body position and the positions of the R second electronic devices that are measured by the first electronic device, a device located near the second user. The hub device forwards, to the second device, a second speech or video of the first user sent by the first device, and forwards, to the first device, a third speech or video of the second user sent by the second device.

According to the tenth aspect, when detecting that a call end condition is met, the hub device sends a call end message to the first device and/or the second device. The call end condition includes that the call end message sent by the first device or the second device is received or the hub device receives no new speech or video after preset duration starting after a speech or a video of a user is forwarded.

According to the tenth aspect or any implementation of the tenth aspect, when the first speech does not include information about the second user, that the hub device determines, from the R second electronic devices based on the human body position and the positions of the R second electronic devices that are measured by the first electronic device, that the second user is located near the second device includes: The hub device determines, based on the human body position that is measured by the first electronic device, the detected human body position as a position of the second user, and determines, as the second user from the R second electronic devices based on the positions of the R second electronic devices, a device located near the second user.

For technical effects that can be achieved by the tenth aspect and any implementation of the tenth aspect, refer to the descriptions of the technical effects in the ninth aspect and any implementation of the ninth aspect. Details are not described herein again.

According to an eleventh aspect, this application provides an electronic device, including a processor, a memory, and a communication module. The memory and the communication module are coupled to the processor. The memory is configured to store computer program code. The computer program code includes computer instructions. When the processor reads the computer instructions from the memory, the electronic device is enabled to perform the method according to the second aspect, the fourth aspect, the sixth aspect, the eighth aspect, the tenth aspect, or any implementation of the aspects.

According to a twelfth aspect, this application provides a computer-readable storage medium. The computer-readable storage medium includes a computer program. When the computer program is run on an electronic device, the electronic device is enabled to perform the method according to the second aspect, the fourth aspect, the sixth aspect, the eighth aspect, the tenth aspect, or any implementation of the aspects.

According to a thirteenth aspect, this application provides a computer program product. When the computer program product runs on an electronic device, the electronic device is enabled to perform the method according to the second aspect, the fourth aspect, the sixth aspect, the eighth aspect, the tenth aspect, or any implementation of the aspects.

For technical effects that can be achieved by the electronic device, the computer-readable medium, and the computer program product according to the eleventh aspect to the thirteenth aspect, refer to the descriptions of the technical effects in the second aspect, the fourth aspect, the sixth aspect, the eighth aspect, the tenth aspect, and any implementation of the aspects. Details are not described herein again.

## BRIEF DESCRIPTION OF DRAWINGS

FIG. 1 is a schematic diagram of a scenario of a human sensing-based automatic control method according to an embodiment of this application;

FIG. 2 is a schematic diagram of a structure of a first electronic device in an automatic control method according to an embodiment of this application;

FIG. 3 is a schematic diagram of a structure of a hub device in an automatic control method according to an embodiment of this application;

FIG. 4 is a schematic diagram of a structure of a second electronic device in an automatic control method according to an embodiment of this application;

FIG. 5A is a schematic diagram of a structure of an ultra-wide band (Ultra-Wide Band, UWB) module in a first electronic device according to this application;

FIG. 5B is a schematic diagram of a structure of a millimeter-wave radar module in a first electronic device according to an embodiment of this application;

FIG. 6 is a schematic diagram of a structure of a UWB module in a second electronic device according to an embodiment of this application;

FIG. 7(a) to FIG. 7(e) are a schematic diagram of several antenna distributions of a UWB module in a first electronic device according to an embodiment of this application;

FIG. 8 is a schematic diagram of several antenna distributions of a millimeter-wave radar module in a first electronic device according to an embodiment of this application;

FIG. 9 is a schematic diagram of several manners of establishing a first coordinate system when a UWB module of a first electronic device includes three antennas according to an embodiment of this application;

FIG. 10 is a schematic diagram of a manner of establishing a second coordinate system according to an embodiment of this application;

FIG. 11 is a schematic diagram of a manner of establishing a third coordinate system according to an embodiment of this application;

FIG. 12(a) to FIG. 12(f) are a schematic diagram of a principle of calculating coordinates of a second electronic device in a first coordinate system according to an embodiment of this application;

FIG. 13(a) to FIG. 13(c) are a schematic diagram of several manners of marking a second electronic device according to an embodiment of this application;

FIG. 14 is a schematic diagram of a manner of marking a spatial area according to an embodiment of this application;

FIG. 15 is a schematic diagram of a pitch $\varphi_e^b$, a yaw $\psi_e^b$, and a roll $\theta_e^b$ of a second coordinate system relative to a first coordinate system according to an embodiment of this application;

FIG. 16 is a schematic diagram of a pitch $\varphi_e^t$, a yaw $\psi_e^t$, and a roll $\theta_e^t$ of a third coordinate system relative to a first coordinate system according to an embodiment of this application;

FIG. 17 is a schematic diagram of a manner of establishing a fourth coordinate system according to an embodiment of this application;

FIG. 18 is a schematic diagram of a principle of determining a distance and a radial speed of a reflection point by a millimeter-wave radar according to an embodiment of this application;

FIG. 19(a) to FIG. 19(c)-2 are a schematic diagram of a principle of determining a signal coming direction of a reflected signal from a reflection point by a millimeter-wave radar according to an embodiment of this application;

FIG. 20 is a schematic diagram of a principle of determining coordinates of a user by a first electronic device in a fourth coordinate system according to an embodiment of this application;

FIG. 21 is a schematic diagram of a method for determining coordinates of a user by a first electronic device in a fourth coordinate system according to an embodiment of this application;

FIG. 22 is a schematic diagram of a method for obtaining a breath frequency and a heartbeat frequency of a user by a millimeter-wave radar according to an embodiment of this application;

FIG. 23 is a schematic diagram of a method for determining a human body attitude of a user by a millimeter-wave radar according to an embodiment of this application;

FIG. 24 is a schematic diagram of conversion between a first coordinate system and a fourth coordinate system on a first electronic device according to an embodiment of this application;

FIG. 25 is a schematic diagram of an example of establishing a whole-house coordinate system (a fifth coordinate system), a first coordinate system, and a sixth coordinate system (a geographical coordinate system) according to an embodiment of this application;

FIG. 26 is a schematic diagram of a flowchart and a principle of a human sensing-based automatic control method in a whole-house scenario according to an embodiment of this application;

FIG. 27(a) and FIG. 27(c) are a schematic diagram of a method for correcting an installation error of a first electronic device according to an embodiment of this application;

FIG. 28 is a schematic diagram of a principle of an ICP algorithm according to an embodiment of this application;

FIG. 29 is a schematic diagram of area division and a user interface according to an embodiment of this application;

FIG. 30 is a schematic flowchart of a method for implementing a distributed alarm clock according to an embodiment of this application;

FIG. 31 is a schematic flowchart of another method for implementing a distributed alarm clock according to an embodiment of this application;

FIG. 32 is a schematic flowchart of still another method for implementing a distributed alarm clock according to an embodiment of this application;

FIG. 33 is a schematic flowchart of still another method for implementing a distributed alarm clock according to an embodiment of this application;

FIG. 34 is a schematic diagram of some user interfaces related to a method for implementing a distributed alarm clock according to an embodiment of this application;

FIG. 35 is a schematic diagram of a method for implementing a distributed alarm clock according to an embodiment of this application;

FIG. 36 is a schematic diagram of a user interface related to a method for implementing a distributed alarm clock according to an embodiment of this application;

FIG. 37 is a schematic flowchart of an IoT device registration method according to an embodiment of this application;

FIG. 38A and FIG. 38B are a schematic flowchart of an IoT device control method according to an embodiment of this application;

FIG. 39A and FIG. 39B are a schematic diagram of some user interfaces related to an IoT device control method according to an embodiment of this application;

FIG. 40 is a schematic flowchart of a reminding method of a smart doorbell according to an embodiment of this application;

FIG. 41 is a schematic flowchart of an incoming call notification method according to an embodiment of this application;

FIG. 42A and FIG. 42B are a schematic diagram of some user interfaces related to an incoming call notification method according to an embodiment of this application;

FIG. 43 is a schematic diagram of a user interface related to an incoming call notification method according to an embodiment of this application; and

FIG. 44A and FIG. 44B are a schematic flowchart of a method for calling a user in a house according to an embodiment of this application.

## DESCRIPTION OF EMBODIMENTS

The following describes the technical solutions in embodiments of this application with reference to the accompanying drawings in embodiments of this application.

In the descriptions of embodiments of this application, terms used in the following embodiments are merely intended for a purpose of describing specific embodiments, but are not intended to limit this application. The terms "a", "the", "the foregoing", "this", and "the one" of singular forms used in this specification and the appended claims of this application are also intended to include forms such as "one or more", unless otherwise specified in the context clearly. It should be further understood that, in the following embodiments of this application, "at least one" and "one or more" mean one or more than two (including two). The term "and/or" is for describing an association relationship between associated objects and indicates that three relationships may exist. For example, A and/or B may indicate the following cases: Only A exists, both A and B exist, and only B exists, where A and B each may be singular or plural. The character "/" generally indicates an "or" relationship between associated objects.

Reference to "an embodiment", "some embodiments", or the like described in this specification indicates that one or more embodiments of this application include a specific feature, structure, or characteristic described with reference to the embodiment. Therefore, statements such as "in an embodiment", "in some embodiments", "in some other embodiments", and "in other embodiments" that appear in different places in this specification do not necessarily mean referring to a same embodiment. Instead, the statements mean "one or more but not all of embodiments", unless otherwise specifically emphasized in another manner. The terms "include", "comprise", "have", and variants of the terms all mean "include but are not limited to", unless otherwise specifically emphasized in other ways. The term "connection" includes a direct connection and an indirect connection, unless otherwise stated. "First" and "second" are merely intended for a purpose of description, and shall not be understood as an indication or implication of relative importance or implicit indication of a quantity of indicated technical features.

In embodiments of this application, the word "example", "for example", or the like is for representing giving an example, an illustration, or a description. Any embodiment or design scheme described as an "example" or "for example" in embodiments of this application should not be explained as being more preferred or having more advantages than another embodiment or design scheme. Exactly, use of the term "example", "for example", or the like is intended to present a related concept in a specific manner.

## 1. Description of overall scenario

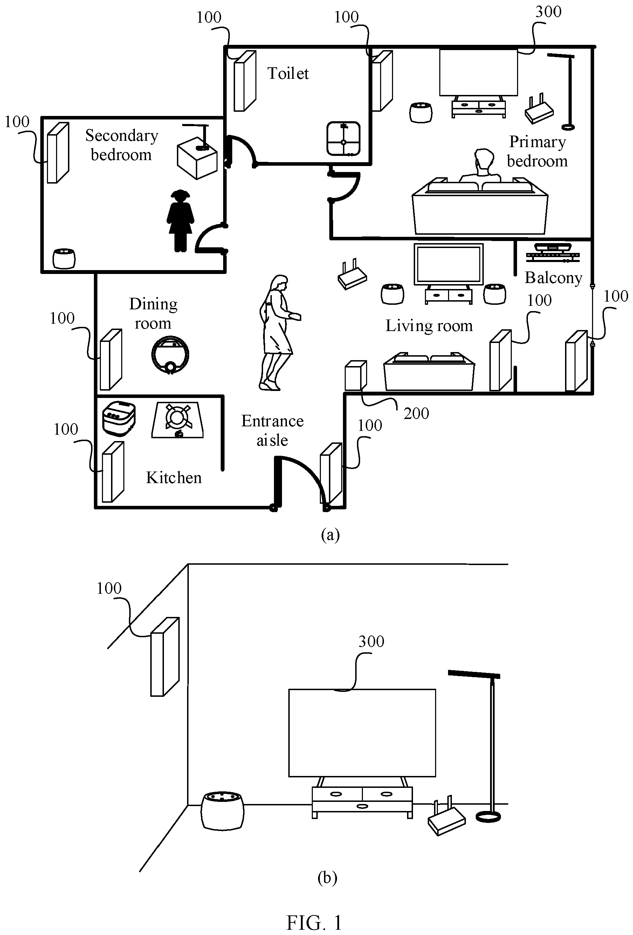

For example, FIG. 1 is a schematic diagram of a scenario of a human sensing-based automatic control method according to an embodiment of this application. As shown in (a) in FIG. 1, a whole house includes an entrance aisle, a kitchen, a dining room, a living room, a balcony, a primary bedroom, a secondary bedroom, a toilet, and the like. At least one first electronic device is disposed in the whole house. For example, each room or area includes at least one first electronic device. A second electronic device (for example, an IoT device) is further disposed in the whole house. Specifically, an electric rice cooker or an electric pressure cooker, a gas device, and the like are disposed in the kitchen. A speaker (for example, a smart speaker), a television (for example, a smart television, also referred to as a smart screen, a large screen, or the like), a routing device, and the like are disposed in the living room. A clothes hanger (for example, a smart clothes hanger) is disposed on the balcony. A floor-sweeping robot and the like are disposed in the dining room. A television (for example, a smart television), a speaker (for example, a smart speaker), a floor lamp (for example, a smart floor lamp), a routing device, and the like are disposed in the primary bedroom. A desk lamp (for example, a smart desk lamp), a speaker (for example, a smart speaker), and the like are disposed in the secondary bedroom. A body fat scale and the like are disposed in the toilet.

The human sensing-based automatic control method provided in embodiments of this application includes a human sensing-based automatic control method in a whole-house scenario, and further includes a human sensing-based automatic control method in a single room or area. The human sensing-based automatic control method provided in embodiments of this application is applied to a communication system. Correspondingly, the communication system includes a human sensing-based communication system in the whole-house scenario (which may also be referred to as a whole-house intelligent system), and a human sensing-based communication system in the single room or area (which may also be referred to as a room intelligent system or an area intelligent system). The communication system includes at least one first electronic device 100 and at least one second electronic device 300. In addition, the communication system may further include a hub device 200.

The first electronic device 100 is configured to locate the second electronic device 300 and/or a user. The first electronic device 100 may include a sensor. In an example, the first electronic device 100 includes an ultra-wide band (Ultra-Wide Band, UWB) module and a millimeter-wave radar module. The UWB module is configured to locate the second electronic device 300, and the millimeter-wave radar module is configured to locate the user.

A UWB technology is a radio communication technology that does not use a carrier modulated signal, but uses an energy pulse sequence below a nanosecond level or a microsecond level, and extends a pulse to a frequency range through orthogonal frequency division modulation or direct sequencing. UWB has features such as a wide spectrum, high precision, low power consumption, a strong anti-multipath capability, high security, and low system complexity, is widely used in short-range and high-speed wireless communication, and especially has a great advantage in the field of indoor locating. Generally, locating accuracy of a UWB system may reach a centimeter level. The UWB system includes a UWB base station and a UWB tag. The UWB base station determines a position (coordinates) of the UWB tag by detecting a distance between the UWB tag and the UWB base station and a signal coming direction of the UWB tag, that is, locates the UWB tag. Locating by using UWB is performed based on a UWB coordinate system (also referred to as a first coordinate system).

In an example, the second electronic device includes a UWB module. The UWB module of the first electronic device 100 implements a function of the UWB base station. The UWB module of the second electronic device implements a function of the UWB tag. The UWB module of the second electronic device is located by using the UWB module of the first electronic device 100, so that the first electronic device 100 can locate the second electronic device.

In another example, some second electronic devices include no UWB module. Usually, the second electronic device including the UWB module is a mobile device (for example, a smartphone or a remote control). Consequently, the second electronic device including the UWB module may be used to mark the second electronic device including no UWB module, so that the first electronic device 100 locates the second electronic device including no UWB module. A specific marking method is described in detail later.

The millimeter-wave radar operates in a millimeter-wave (millimeter-wave) band, is mainly configured to detect a moving object, and has an operating frequency band distributed in a 30 to 300 GHz frequency domain (a wavelength is 1 to 10 mm). The millimeter-wave radar continuously transmits (radiates) a specific form of radio magnetic signal when operating, receives an electromagnetic echo signal reflected by an object, and determines spatial information of the object by comparing a difference between the transmitted signal and the received signal. The millimeter-wave radar has features of a small size and high spatial resolution, is deployed indoors, and may be configured to detect information about a position, a physiological feature (for example, a breath frequency or a heartbeat frequency), an identity category (for example, an adult or a child), and a human body attitude (for example, standing, sitting, or lying) of a human body (the user) in a whole house. Consequently, by using the integrated millimeter-wave radar module, the first electronic device 100 can locate the user, and even detect the information such as the physiological feature, the identity category, and the human body attitude of the user. A specific method is described in detail later.

Locating by using the millimeter-wave radar is implemented based on a millimeter-wave radar coordinate system (also referred to as a second coordinate system). Coordinates respectively located in the second coordinate system and the first coordinate system need to be converted or unified to a same coordinate system. A specific coordinate system conversion method is described in detail later.

It should be noted that, in this embodiment of this application, an example in which the first electronic device 100 includes the UWB module and the millimeter-wave radar is used for description. In some other embodiments, the first electronic device 100 may include only the UWB module or the millimeter-wave radar. The first electronic device 100 including the UWB module is configured to locate the second electronic device 300. The first electronic device 100 including the millimeter-wave radar is configured to locate the user. The two types of first electronic devices 100 cooperate with each other to implement the human sensing-based automatic control method provided in embodiments of this application. This is not limited in embodiments of this application.

The first electronic device 100 may obtain a position of the second electronic device 300 (that is, locate the second electronic device), and may further obtain a position of a user in a room or an area (that is, locate the user). The whole house includes at least one room or area. If only one first electronic device is disposed in the whole house, signal attenuation or the like may be caused due to a reason, for example, wall blockage. In this case, the first electronic device cannot cover all areas in the whole house. Therefore, a plurality of first electronic devices are generally disposed in the whole house. For example, one first electronic device is disposed in each piece of relatively independent space (for example, the living room, a bedroom, a study, the balcony, the toilet, the kitchen, or an aisle), and is configured to locate a second electronic device and a user in the independent space. In this way, a second electronic device or a user at any position in the whole house can be detected by the first electronic device.

For example, as shown in (a) in FIG. 1, a signal receiving and sending range of the first electronic device disposed in the entrance aisle may cover the entrance aisle. A signal receiving and sending range of the first electronic device disposed in the kitchen may cover the kitchen. A signal sending and receiving range of the first electronic device disposed in the living room may cover the living room. A signal sending and receiving range of the first electronic device disposed in the dining room may cover the dining room. A signal sending and receiving range of the first electronic device disposed on the balcony may cover the balcony. A signal receiving and sending range of a first electronic device disposed in the primary bedroom may cover the primary bedroom. A signal sending and receiving range of the first electronic device disposed in the toilet may cover the toilet. A signal receiving and sending range of a first electronic device disposed in the secondary bedroom may cover the secondary bedroom. In an example, as shown in (b) in FIG. 1, the first electronic device may be disposed on a wall of the room or the area. In an example, the first electronic device may be disposed on a ceiling or the like of the room or the area. In this way, blockage of a signal by an object, for example, furniture in the whole house, can be reduced, and reduction of detection accuracy of the first electronic device due to blockage of the signal can be avoided. In an example, the first electronic device may be disposed on the ground or the like of the room or the area. Optionally, the first electronic device 100 may exist independently, or may be integrated with the second electronic device. This is not limited in this application. For example, the first electronic device 100 and a smart air conditioner are integrated into one device.

Optionally, some rooms or areas may not need to be equipped with a first electronic device. In other words, not all rooms or areas each are equipped with at least one first electronic device. For example, the dining room may be equipped with no first electronic device. One first electronic device may be shared in the entrance aisle and the dining room, or one first electronic device may be shared in the dining room and the living room.

It should be noted that, although the smart television is only shown as the second electronic device 300 in (a) in FIG. 1, a person skilled in the art should know that the second electronic device 300 includes but is not limited to the smart television, a smart speaker, a smart lamp (for example, a ceiling lamp, a smart desk lamp, or an aromatherapy lamp), a floor-sweeping robot, a body fat scale, a smart clothes hanger, a smart electric rice cooker, an air purifier, a humidifier, a desktop computer, a routing device, a smart socket, a water dispenser, a smart refrigerator, a smart air conditioner, a smart switch, a smart lock, and the like. It should be noted that, alternatively, the second electronic device 300 may not be a smart home device, but a portable device, for example, a personal computer (person computer, PC), a tablet computer, a mobile phone, or a smart remote control. A specific form of the second electronic device 300 is not limited in embodiments of this application.

The second electronic device 300 and the first electronic device 100 may be connected to the hub device 200 in a wired (for example, power line communication (power line communication, PLC)) manner and/or a wireless (for example, wireless fidelity (wireless fidelity, Wi-Fi) or Bluetooth) manner. It may be understood that the second electronic device 300 and the first electronic device 100 may be connected to the hub device 200 in a same manner or different manners. For example, both the second electronic device 300 and the first electronic device 100 are connected to the hub device 200 in the wireless manner. Alternatively, the second electronic device 300 is connected to the hub device 200 in the wireless manner, and the first electronic device 100 is connected to the hub device 200 in the wired manner. Alternatively, devices such as the smart speaker, the smart television, the body fat scale, and the floor-sweeping robot in the second electronic device 300 are connected to the hub device 200 in the wireless (for example, Wi-Fi) manner. Devices such as the smart desk lamp, the smart clothes hanger, and the smart lock in the second electronic device 300 are connected to the hub device 200 in the wired (for example, a PLC) manner. Preferably, the first electronic device 100 communicates with the second electronic device 300 in the wireless manner.

In an example, the first electronic device 100 may upload, to the hub device 200 in the wired or wireless manner, at least one of position information of the second electronic device 300 and the information such as the position, the physiological feature, the identity category, and the human body attitude of the user, that are obtained through detection.

The hub device 200, also referred to as a hub, a central control system, a host, or the like, is configured to receive the information sent by the first electronic device 100. Optionally, the hub device 200 is further configured to: construct a whole-house map, establish a whole-house coordinate system, and unify, to the whole-house coordinate system for unified measurement, position information obtained by all the first electronic devices 100. In this way, position information of the second electronic device 300 or the user obtained by all the first electronic devices 100 through detection can be unified to the whole-house coordinate system, and a specific position of the second electronic device 300 or the user in the whole house can be determined. The hub device 200 further notifies or controls the second electronic device 300 based on the received information (including but not limited to the position information). Correspondingly, conversion between coordinate systems is also mentioned, and is described in detail later.

In an implementation, the hub device 200 receives the information that is sent by the first electronic device 100 and that includes the position information of the second electronic device 300 and at least one of the position, the physiological feature, the identity category, the human body attitude, and the like of the user. The hub device 200 notifies or controls, based on the position information of the second electronic device 300 and at least one of the position, the physiological feature, the identity category, the human body attitude, and the like of the user, the second electronic device 300 to execute a preset instruction. For example, when the user wakes up a smart speaker by a voice, the hub device 200 notifies or controls, based on positions of a plurality of smart speakers in the whole house, one or more smart speakers, that are closest to the user, to wake up. For example, when the user moves from one room to another room in the whole house, the hub device 200 controls a smart speaker in the room, from which the user leaves, to stop playing an audio, and controls a smart speaker in the room, that the user enters, to start playing (for example, continuing to play) the audio. For another example, the hub device 200 controls, based on distances between two smart speakers (the two smart speakers respectively play a left-channel audio and a right-channel audio of a same audio) and the user, playing volume of the smart speakers, so that volume of the left-channel audio received by the user is consistent with that of the right-channel audio received by the user. For another example, the user watches a video (for example, the video includes violent content) in a room by using a smart television, and it is detected that a child user enters the room, and the hub device 200 controls the smart television to stop playing the video. For another example, the hub device 200 notifies or controls, based on a position (for example, a distance or an orientation) of the user relative to a smart television, the smart television to start playing or stop playing a video.

Optionally, as shown in (a) in FIG. 1, at least one hub device 200 is disposed in the whole house. A first electronic device in each room or each area may send, to the hub device 200, all detected position information of a user and position information of one or more second electronic devices in the room or the area. The hub device 200 obtains detection data (including but not limited to the position information) of each room or each area in the whole house, so that when a preset condition is met, the hub device 200 can notify or control a corresponding second electronic device in a corresponding room or a corresponding area.

Optionally, a hub device (not shown in the figure) may be alternatively disposed in each room or each area in the whole house. The first electronic device in each room or each area may send, to the hub device in the room or the area, detected position information of a user and detected position information of one or more second electronic devices in the room or the area. The hub device in the current room or the current area is then sent to the hub device 200 in the whole house. The hub device 200 in the whole house obtains the detection data of each room or each area in the whole house, so that when a preset condition is met, a hub device 200 in a corresponding room or a corresponding area may be notified or controlled. The hub device in the corresponding room or the corresponding area then notifies or controls a corresponding second electronic device.

Optionally, the hub device in each room or each area and the hub device in the whole house may exist independently, or may be integrated with the first electronic device or the second electronic device into one device, or may be integrated with the first electronic device and the second electronic device into one device. This is not limited in this application.

Optionally, some rooms or areas may not need to be equipped with a hub device. In other words, not all rooms or areas each are equipped with at least one hub device. For example, the dining room may be equipped with no hub device. A hub device is shared in the dining room and the entrance aisle, or a hub device is shared in the dining room and the living room.

Optionally, the hub device 200 in the whole house may further assume a function of a hub device in a room or an area. For example, the hub device 200 in the whole house may further assume a function of a hub device in the living room. In an example, a hub device is disposed in each room or each area other than a room or an area (for example, the living room). When communicating with the second electronic device in each room or each area other than the room or the area (for example, the living room), the hub device 200 in the whole house still performs communication through the hub device in each room or each area. However, when communicating with the second electronic device in the room or the area (for example, the living room), the hub device 200 in the whole house no longer performs communication through the hub device in the room or the area (for example, the living room).

In an example, the communication system further includes a routing device (for example, a router). The routing device is configured to: connect to a local area network or an internet, and uses a specific protocol to select and set a path for sending a signal. For example, one or more routers are deployed in the whole house to constitute a local area network, or to access the local area network or the internet. The second electronic device 300 or the first electronic device 100 accesses the router, and performs data transmission with a device in the local area network or a device in the internet through a Wi-Fi channel established by the router. In an implementation, the hub device 200 may be integrated with the routing device into one device. For example, the hub device 200 and the routing device are integrated into a routing device, that is, the routing device has a function of the hub device 200. The routing device may be one or more routing devices in a parent-child routing device, or may be an independent routing device.

In an example, the communication system further includes a gateway (Gateway). The gateway is also referred to as an inter-network connector or a protocol converter. In an implementation, the gateway is configured to forward, to the routing device or the hub device 200, the information from the first electronic device 100. In another implementation, the function of the hub device 200 may be implemented by the gateway.

In an example, the communication system further includes a server (for example, a cloud server). The hub device 200, the routing device, or the gateway may send, to the server, the received information from the first electronic device 100. Further, the hub device 200, the routing device, or the gateway may further send, to the server, control information of the hub device 200 for the second electronic device 300. Further, the hub device 200, the routing device, or the gateway may further upload, to the server, various types of information generated in a running process of the second electronic device 300, to provide the various types of information for the user for viewing.

In an example, the communication system further includes one or more input devices (for example, the input device is a control panel). For example, a human-machine interaction interface of the communication system is displayed on the control panel. The user may view, on the man-machine interaction interface, information (for example, connection information of each device in the communication system) about the communication system, running information of the second electronic device 300, the control information of the hub device 200 for the second electronic device 300, and/or the like. The user may further input a control instruction on the man-machine interaction interface in a manner, for example, tapping a screen or a voice, to control a device in the communication system.

The foregoing content is merely some descriptions of the human sensing-based automatic control method provided in embodiments of this application. It should be noted that, in the examples or optional manners described above, any content may be freely combined, and combined content also falls within the scope of this application.

**2. Description of a hardware structure of an electronic device**

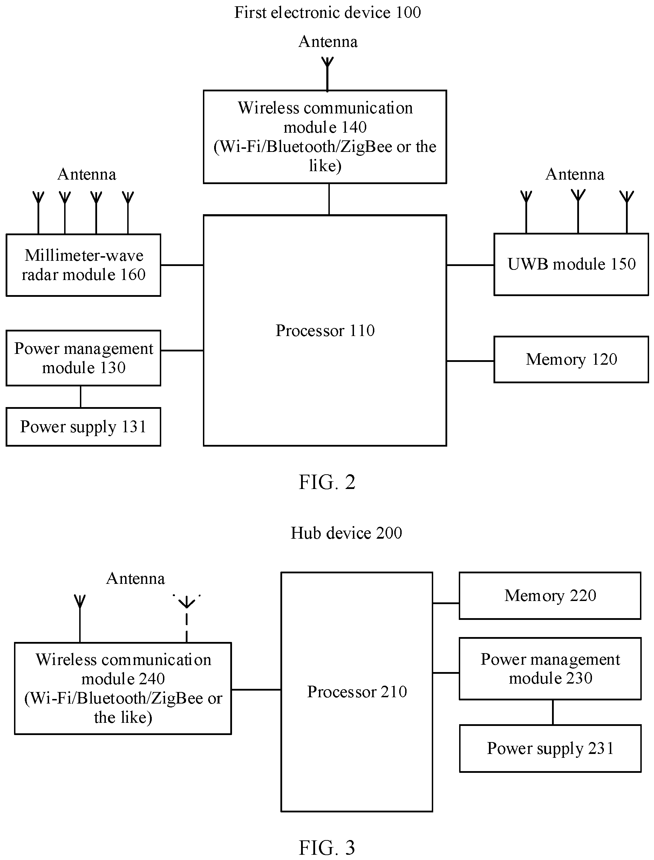

For example, FIG. 2 is a schematic diagram of a structure of a first electronic device 100.

As shown in FIG. 2, the first electronic device 100 may include a processor 110, a memory 120, a power management module 130, a power supply 131, a wireless communication module 140, a UWB module 150, a millimeter-wave radar module 160, and the like.

It may be understood that the structure shown in FIG. 2 constitutes no specific limitation on the first electronic device 100. In some other embodiments of this application, the first electronic device 100 may include more or fewer components than those shown in the figure, or combine some of the components, or split some of the components, or may have different arrangements of the components. The components shown in the figure may be implemented by using hardware, software, or a combination of software and hardware.

The processor 110 may include one or more processing units. Different processing units may be independent components, or may be integrated into one or more processors. For example, the processor 110 is a central processing unit (central processing unit, CPU), or an application-specific integrated circuit (application-specific integrated circuit, ASIC), or may be configured as one or more integrated circuits implementing embodiments of this application, for example, one or more microprocessors (digital signal processors, DSPs) or one or more field programmable gate arrays (field programmable gate arrays, FPGAs).

The memory 120 may be configured to store computer-executable program code. The executable program code includes instructions. For example, the memory 120 may further store data processed by the processor 110. In addition, the memory 120 may include a high-speed random access memory, and may further include a non-volatile memory, for example, at least one magnetic disk storage component, a flash memory component, or a universal flash storage (universal flash storage, UFS). The processor 110 runs the instructions stored in the memory 120 and/or instructions stored in a memory disposed in the processor, to perform various function applications of the first electronic device 100 and data processing.

The power management module 130 is configured to receive an input from the power supply 131. The power supply 131 may be a battery, or may be a mains supply. The power management module 130 receives power supplying from the battery and/or the mains supply, and supplies power to components of the first electronic device 100, such as the processor 110, the memory 120, the wireless communication module 140, the UWB module 150, and the millimeter-wave radar module 160.

The wireless communication module 140 may provide a solution that is applied to the first electronic device 100 and includes wireless communication such as a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), near field communication (near field communication, NFC), an infrared (infrared, IR) technology, and ZigBee (ZigBee). The wireless communication module 140 may be one or more components integrating at least one communication processing module. The wireless communication module 140 receives an electromagnetic wave through an antenna, performs frequency modulation and filtering processing on an electromagnetic wave signal, and sends a processed signal to the processor 110. The wireless communication module 140 may further receive a to-be-sent signal from the processor 110, perform frequency modulation and amplification on the to-be-sent signal, and convert a processed signal into an electromagnetic wave for radiation through the antenna. It should be noted that quantities of antennas of the wireless communication module 140, the UWB module 150, and the millimeter-wave radar module 160 in FIG. 2 are merely examples for description. It may be understood that the communication module 140, the UWB module 150, and the millimeter-wave radar module 160 each may include more or fewer antennas. This is not limited in embodiments of this application.

The UWB module 150 may provide a solution that is applied to the first electronic device 100 and includes wireless communication based on a UWB technology. For example, the UWB module 150 is configured to implement a function of the foregoing UWB base station. In an embodiment of this application, the UWB base station may locate a UWB tag. Specifically, duration within which a UWB signal flies in the air may be calculated with reference to some locating algorithms by detecting the UWB signal. A distance between the UWB tag and the UWB base station is obtained by multiplying the duration by a rate (for example, a light speed) at which the UWB signal is transmitted in the air. In an embodiment of this application, the UWB base station may further determine a direction of the UWB tag relative to the UWB base station (namely, a signal coming direction of the UWB tag) based on a phase difference obtained when the UWB signal sent by the UWB tag reaches different antennas of the UWB base station. The signal coming direction includes a horizontal coming direction and a vertical coming direction.

For example, FIG. 3 is a schematic diagram of a structure of a hub device 200.

As shown in FIG. 3, the hub device 200 may include a processor 210, a memory 220, a power management module 230, a power supply 231, a wireless communication module 240, and the like.

It may be understood that the structure shown in FIG. 3 constitutes no specific limitation on the hub device 200. In some other embodiments of this application, the hub device 200 may include more or fewer components than those shown in the figure, or combine some of the components, or split of some of the components, or may have different arrangements of the components. The components shown in the figure may be implemented by using hardware, software, or a combination of software and hardware.

The processor 210 may include one or more processing units. Different processing units may be independent components, or may be integrated into one or more processors. For example, the processor 210 is a central processing unit (central processing unit, CPU), or an application-specific integrated circuit (application-specific integrated circuit, ASIC), or may be configured as one or more integrated circuits implementing embodiments of this application, for example, one or more microprocessors (digital signal processors, DSPs) or one or more field programmable gate arrays (field programmable gate arrays, FPGAs).

The memory 220 may be configured to store computer-executable program code. The executable program code includes instructions. For example, the memory 220 may further store data processed by the processor 210. In addition, the memory 220 may include a high-speed random access memory, and may further include a non-volatile memory, for example, at least one magnetic disk storage component, a flash memory component, or a universal flash storage (universal flash storage, UFS). The processor 210 runs the instructions stored in the memory 220 and/or instructions stored in a memory disposed in the processor, to perform various function applications of the hub device 200 and data processing.

The power management module 230 is configured to receive an input from the power supply 231. The power supply 231 may be a battery, or may be a mains supply. The power management module 230 receives power supplying from the battery and/or the mains supply, and supplies power to components of the hub device 200, such as the processor 210, the memory 220, and the wireless communication module 240.

The wireless communication module 240 may provide a solution that is applied to the hub device 200 and includes wireless communication such as a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), near field communication (near field communication, NFC), an infrared (infrared, IR) technology, and ZigBee (ZigBee). The wireless communication module 240 may be one or more components integrating at least one communication processing module. The wireless communication module 240 receives an electromagnetic wave through an antenna, performs frequency modulation and filtering processing on an electromagnetic wave signal, and sends a processed signal to the processor 210. The wireless communication module 240 may further receive a to-be-sent signal from the processor 210, perform frequency modulation and amplification on the to-be-sent signal, and convert a processed signal into an electromagnetic wave for radiation through the antenna.

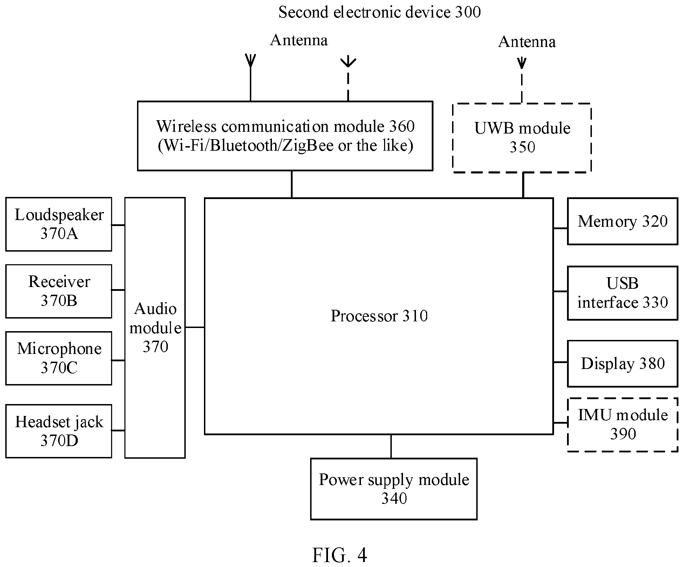

For example, FIG. 4 is a schematic diagram of a structure of a second electronic device 300.

As shown in FIG. 4, the second electronic device 300 may include a processor 310, a memory 320, a universal serial bus (universal serial bus, USB) interface 330, a power supply module 340, a UWB module 350, a wireless communication module 360, and the like. Optionally, the second electronic device 300 may further include an audio module 370, a loudspeaker 370A, a receiver 370B, a microphone 370C, a headset jack 370D, a display 380, a sensor module 390, and the like.

It may be understood that the structure shown in FIG. 4 constitutes no specific limitation on the second electronic device 300. In some other embodiments of this application, the second electronic device 300 may include more or fewer components than those shown in the figure, or combine some of the components, or split some of the components, or may have different arrangements of the components. The components shown in the figure may be implemented by using hardware, software, or a combination of software and hardware. In addition, an interface connection relationship between the modules that is shown in FIG. 4 is merely an example for description, and constitutes no limitation on the structure of the second electronic device 300. In some other embodiments of this application, the second electronic device 300 may alternatively use an interface connection manner different from that in FIG. 4, or use a combination of a plurality of interface connection manners.

The processor 310 may include one or more processing units. For example, the processor 310 may include an application processor (application processor, AP), a modem processor, a graphics processing unit (graphics processing unit, GPU), an image signal processor (image signal processor, ISP), and a video codec, and a digital signal processor (digital signal processor, DSP). Different processing units may be independent components, or may be integrated into one or more processors.

The memory 320 may be configured to store computer-executable program code. The executable program code includes instructions. For example, the memory 320 may further store data processed by the processor 310. In addition, the memory 320 may include a high-speed random access memory, and may further include a non-volatile memory, for example, at least one magnetic disk storage component, a flash memory component, or a universal flash storage (universal flash storage, UFS). The processor 310 runs the instructions stored in the memory 320 and/or instructions stored in a memory disposed in the processor, to perform various function applications of the second electronic device 300 and data processing.

The USB interface 330 is an interface that conforms to a USB standard specification, and may be specifically a mini USB interface, a micro USB interface, a USB Type-C interface, or the like. The USB interface 330 may be configured to connect to a charger to charge the second electronic device 300, or may be configured to transmit data between the second electronic device 300 and a peripheral device.

The power supply module 340 is configured to supply power to components of the second electronic device 300, for example, the processor 310 and the memory 320.

The UWB module 350 may provide a solution that is applied to the second electronic device 300 and includes wireless communication based on a UWB technology. For example, the UWB module 350 is configured to implement a function of the foregoing UWB tag.

The wireless communication module 360 may provide a solution that is applied to the second electronic device 300 and includes wireless communication such as a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), near field communication (near field communication, NFC), an infrared (infrared, IR) technology, and ZigBee (ZigBee). The wireless communication module 360 may be one or more components integrating at least one communication processing module. The wireless communication module 360 receives an electromagnetic wave through an antenna, performs frequency modulation and filtering processing on an electromagnetic wave signal, and sends a processed signal to the processor 310. The wireless communication module 360 may further receive a to-be-sent signal from the processor 310, perform frequency modulation and amplification on the to-be-sent signal, and convert a processed signal into an electromagnetic wave for radiation through the antenna. The wireless communication module 360 and the UWB module 350 may be integrated together or disposed separately. This is not limited in this application.

The second electronic device 300 may implement an audio function by using the audio module 370, the loudspeaker 370A, the receiver 370B, the microphone 370C, the headset jack 370D, the application processor, and the like, for example, audio playing or recording.

The audio module 370 is configured to convert digital audio information into an analog audio signal output, and is further configured to convert an analog audio input into a digital audio signal. The audio module 370 may be further configured to encode and decode an audio signal. In some embodiments, the audio module 370 may be disposed in the processor 310, or some functional modules of the audio module 370 are disposed in the processor 310.

The loudspeaker 370A, also referred to as a "speaker", is configured to convert an audio electrical signal into a sound signal. The second electronic device 300 may be for listening to an audio through the loudspeaker 370A.

The receiver 370B, also referred to as an "earpiece", is configured to convert an audio electrical signal into a sound signal.

The microphone 370C, also referred to as a "mike" or a "mic", is configured to convert a sound signal into an electrical signal. A user may make a sound near the microphone 370C through the mouth of the user, to input a sound signal to the microphone 370C.

The headset jack 370D is configured to connect to a wired headset. The headset jack 370D may be a USB interface 330, or may be a 3.5 mm open mobile terminal platform (open mobile terminal platform, OMTP) standard interface or a cellular telecommunications industry association of the USA (cellular telecommunications industry association of the USA, CTIA) standard interface.

The display 380 is configured to display an image, a video, and the like. The display 380 includes a display panel. The display panel may be a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flexible light-emitting diode, FLED), a mini-LED, a micro-LED, a micro-OLED, a quantum dot light-emitting diode (quantum dot light-emitting diode, QLED), or the like.

Optionally, the sensor module 390 includes an inertial measurement unit (inertial measurement unit, IMU) module and the like. The IMU module may include a gyroscope, an accelerometer, and the like. The gyroscope and the accelerometer may be configured to determine a motion attitude of the second electronic device 300. In some embodiments, angular velocities of the second electronic device 300 around three axes may be determined by using the gyroscope. The accelerometer may be configured to detect magnitude of accelerations of the second electronic device 300 in various directions (generally on three axes). When the second electronic device 300 is still, magnitude and a direction of gravity may be detected. In an embodiment of this application, a device attitude of the second electronic device 300 may be obtained based on the angular speed and the acceleration that are measured by the IMU module. Optionally, some second electronic devices each may include an IMU module, and some second electronic devices each include no IMU module.

Optionally, the second electronic device 300 further includes a filter (for example, a Kalman filter). For example, an output of the IMU module and the output of the UWB module 350 may be superimposed, and a signal obtained after the output of the MU module and the output of the UWB module 350 are superimposed may be input to the Kalman filter for filtering, to reduce an error.

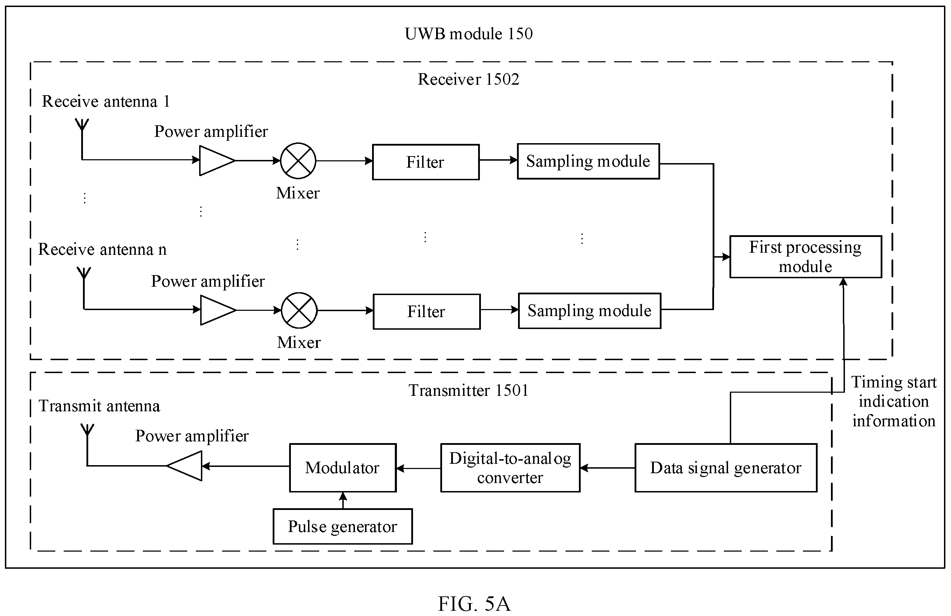

For example, FIG. 5A shows a structure of the UWB module in the first electronic device according to an embodiment of this application. As shown in FIG. 5A, the UWB module 150 includes a transmitter 1501 and a receiver 1502. The transmitter 1501 and the receiver 1502 may run independently. The transmitter 1501 includes a data signal generator, a pulse generator, a modulator, a digital-to-analog converter, a power amplifier, a transmit antenna, and the like. The data signal generator is configured to generate a data signal, and is further configured to send timing start indication information to the receiver 1502 when starting to generate the data signal. The pulse generator is configured to generate cyclical pulse signals. The digital-to-analog converter is configured to convert a digital signal into an analog signal. A data signal that needs to be sent is modulated by the modulator to the pulse signal generated by the pulse generator, and undergoes power amplification performed by the power amplifier. Then, a UWB signal is transmitted through the transmit antenna. The receiver 1502 includes a receive antenna, a mixer, a filter, a sampling module, a first processing module, and the like. Any receive antenna receives the UWB signal (for example, in a form of a pulse sequence). The received UWB signal is mixed by the mixer, filtered and amplified by the filter, and undergoes analog-to-digital conversion performed by the sampling module, to obtain a baseband digital signal. The first processing module is configured to process the baseband digital signal, to detect the UWB signal. For example, the first processing module calculates time of flight (time of flight, ToF) of the UWB signal based on the timing start indication information and a moment at which the pulse sequence is received, and calculates, based on the ToF and a rate (for example, a light speed) at which the UWB signal is transmitted in the air, a distance between the first electronic device 100 and the second electronic device 300 including the UWB module 350. For another example, the first processing module calculates, based on a phase difference between pulse sequences received by a plurality of receive antennas, a signal coming direction of the second electronic device 300 including the UWB module 350. It should be noted that, in FIG. 5A, a pulse sequence received by each receive antenna passes through a power amplifier, a mixer, a filter, and a sampling module that are in a group, which indicates a procedure of processing the pulse sequence. Optionally, the receiver 1502 may include only a power amplifier, a mixer, a filter, and a sampling module that are in one group. In an implementation, one antenna may implement a function of one transmit antenna in the transmitter 1501 and a function of one receive antenna in the receiver 1502, that is, the transmit antenna and the receive antenna are integrated into a same antenna.

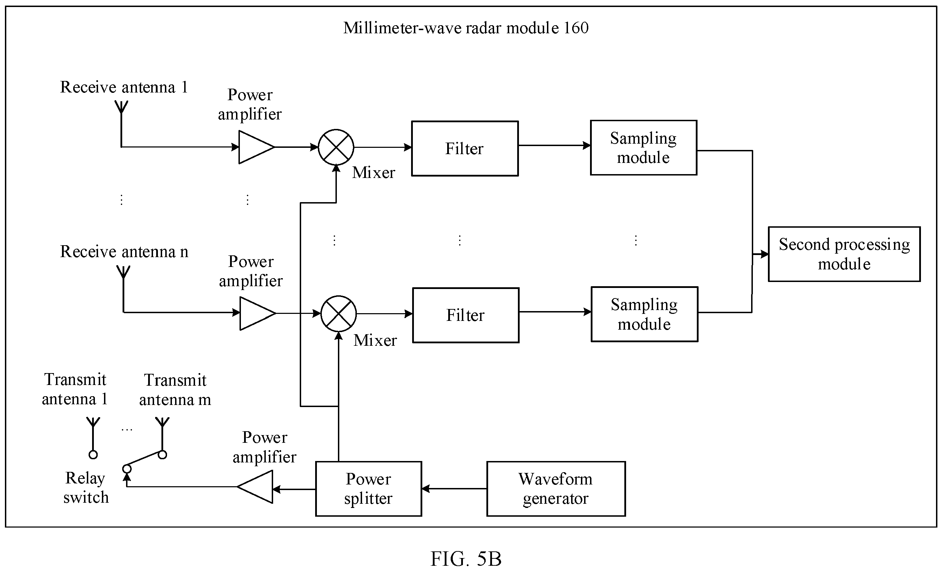

For example, FIG. 5B shows a structure of the millimeter-wave radar module in the first electronic device according to an embodiment of this application. As shown in FIG. 5B, the millimeter-wave radar module 160 includes a transmit antenna, a relay switch, a receive antenna, a waveform generator, a mixer, a filter, a second processing module, and the like. The waveform generator is configured to generate a to-be-transmitted signal. For example, the to-be-transmitted signal is a linear frequency-modulated continuous signal (linear frequency-modulated continuous wave, LFMCW). After the to-be-transmitted signal passes through a power splitter, one part of the to-be-transmitted signal undergoes power amplification performed by the power amplifier. Then, the relay switch selects a transmit antenna to transmit a millimeter-wave signal. The other part of the transmitted signal is used as a local oscillator signal, and is mixed, by the mixer, with a millimeter-wave signal received by the receive antenna. A signal output by the mixer is a difference frequency signal. The difference frequency signal is filtered and amplified by the filter, and undergoes analog-to-digital conversion (sampling) performed by the sampling module, to become a digital difference frequency signal. The second processing module detects a target by processing the digital difference frequency signal, and obtains information such as a distance and a signal coming direction of the target. In FIG. 5B, n and m are positive integers greater than or equal to 1.

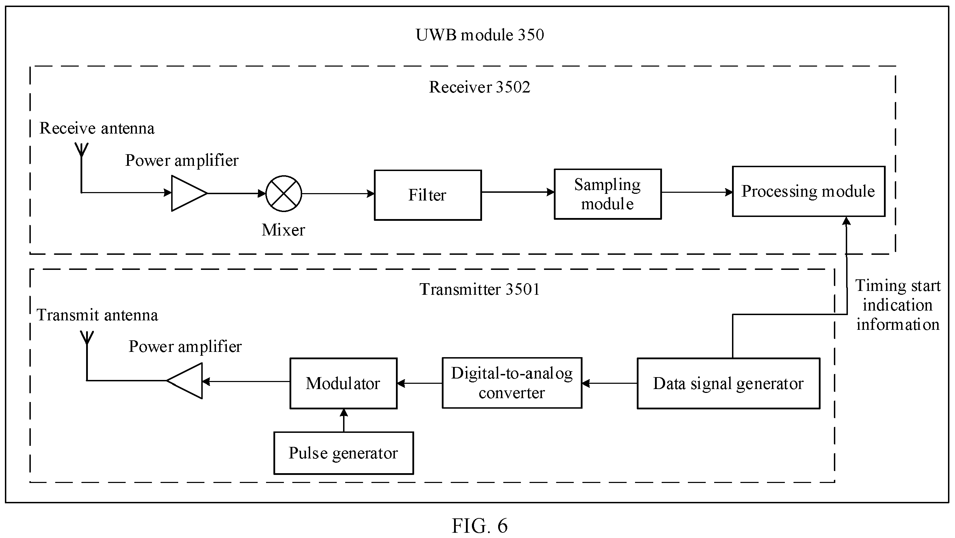

For example, FIG. 6 shows a structure of the UWB module in the second electronic device. As shown in FIG. 6, the UWB module 350 includes a transmitter 3501 and a receiver 3502. The transmitter 3501 and the receiver 3502 may run independently. The transmitter 3501 includes a data signal generator, a pulse generator, a modulator, a digital-to-analog converter, a power amplifier, a transmit antenna, and the like. The data signal generator is configured to generate a data signal. The pulse generator is configured to generate cyclical pulse signals. The digital-to-analog converter is configured to convert a digital signal into an analog signal. A data signal that needs to be sent is modulated by the modulator to the pulse signal generated by the pulse generator, and undergoes power amplification performed by the power amplifier. Then, a UWB signal is transmitted through the transmit antenna. The receiver 3502 includes a receive antenna, a mixer, a filter, a sampling module, a processing module, and the like. The receive antenna receives the UWB signal (for example, in a form of a pulse sequence). The received UWB signal is mixed by the mixer, filtered and amplified by the filter, and undergoes analog-to-digital conversion performed by the sampling module, to obtain a baseband digital signal. The processing module is configured to process the baseband digital signal, to detect the UWB signal. In an implementation, the transmit antennas in the transmitter 3501 and the receive antennas in the receiver 3502 may be integrated into a same antenna.

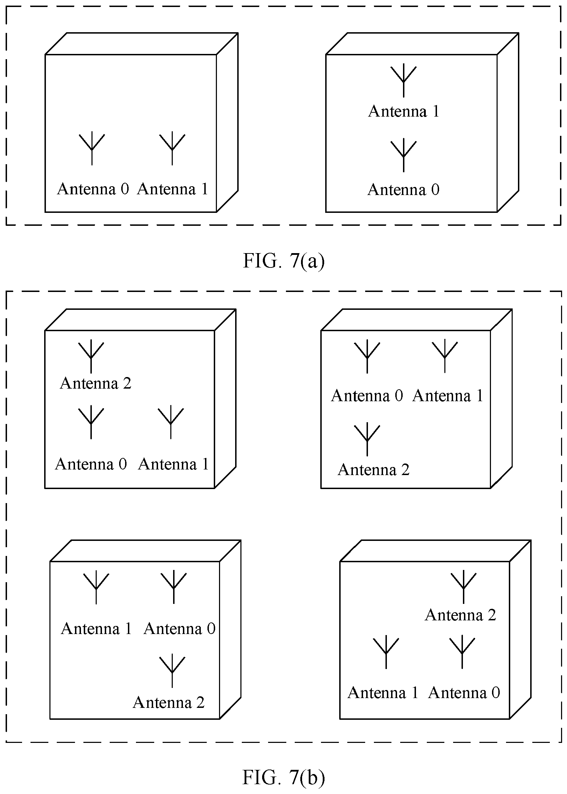

For example, FIG. 7(a) to FIG. 7(e) show several antenna distributions of the UWB module in the first electronic device according to an embodiment of this application. FIG. 7(a) shows examples of two two-antenna structures. One two-antenna structure is a transverse (for example, horizontal) antenna structure, and the other two-antenna structure is a longitudinal (for example, vertical) antenna structure. Preferably, a distance between an antenna 0 and an antenna 1 is λ/2, and λ is a wavelength of the UWB signal. The transverse antenna structure may be configured to measure a transverse coming direction (for example, a horizontal coming direction) of the UWB signal. The longitudinal antenna structure may be configured to measure a longitudinal coming direction (for example, a vertical coming direction) of the UWB signal. In an implementation, a first electronic device on the left shown in FIG. 7(a) may cooperate with a first electronic device on the right (for example, the two first electronic devices are arranged at a specific angle), to detect a signal coming direction of the second electronic device including the UWB module.

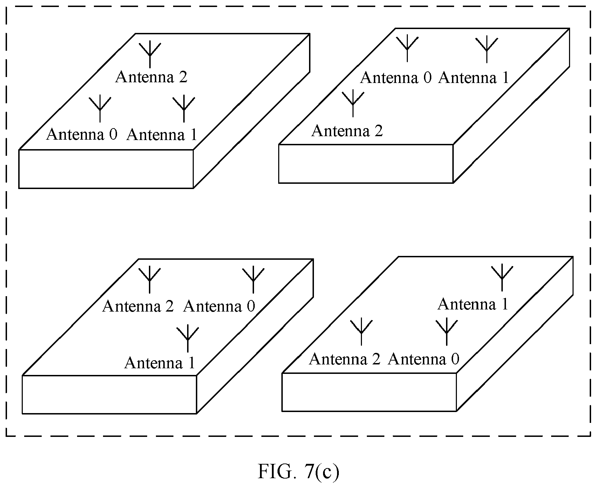

FIG. 7(b) and FIG. 7(c) show examples of a three-antenna structure. As shown in FIG. 7(b) and FIG. 7(c), three antennas have a structural relationship in an L shape (or referred to as a right triangle). As shown in FIG. 7(b), an antenna 0 and an antenna 1 are aligned in a transverse direction (for example, a horizontal direction). The antenna 0 and an antenna 2 are aligned in a longitudinal direction (for example, a vertical direction). To be specific, a plane on which the antenna 0, the antenna 1, and the antenna 2 are located is a longitudinal plane (for example, a longitudinal plane), and has a distribution relationship in the L shape on the longitudinal plane. As shown in FIG. 7(c), a plane on which an antenna 0, an antenna 1, and an antenna 2 are located is a transverse plane (for example, a horizontal plane), and a connection line between the antenna 0 and the antenna 1 (it is assumed that there is a connection line between the antenna 0 and the antenna 1) is perpendicular to a connection line between the antenna 0 and the antenna 2 (it is assumed that there is a connection line between the antenna 0 and the antenna 2). To be specific, the antenna 0, the antenna 1, and the antenna 2 have a distribution relationship in the L shape on the transverse plane. For example, when the antenna 0, the antenna 1, and the antenna 2 are distributed in the L shape, a distance between the antenna 0 and the antenna 1 and a distance between the antenna 0 and the antenna 2 may be less than or equal to λ/2, where λ is a wavelength of the UWB signal. The distance between the antenna 0 and the antenna 1 and the distance between the antenna 0 and the antenna 2 may be the same or different.

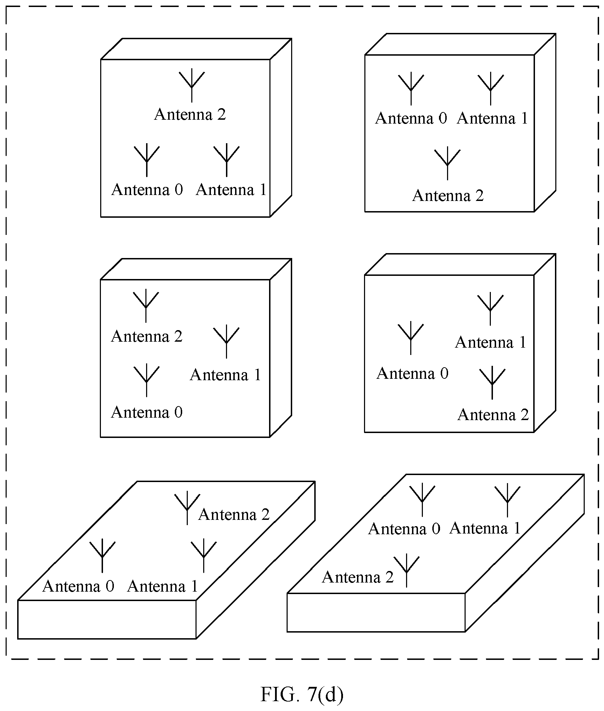

FIG. 7(d) shows examples of some other three-antenna structures. As shown in FIG. 7(d), three antennas have a structural relationship in shape of a triangle (for example, an equilateral triangle or an isosceles triangle). For example, a plane on which an antenna 0, an antenna 1, and an antenna 2 are located is a longitudinal plane (for example, a vertical plane), and are distributed in the shape of the triangle on the longitudinal plane. For another example, an antenna 0, an antenna 1, and an antenna 2 are distributed in the shape of the triangle on a transverse plane (for example, a horizontal plane). For example, when the antenna 0, the antenna 1, and the antenna 2 are distributed in the shape of the triangle, a distance between any two of the antenna 0, the antenna 1, and the antenna 2 may be less than or equal to λ/2, where λ is a wavelength of the UWB signal. In addition, distances between any two of the antenna 0, the antenna 1, and the antenna 2 may be the same or different. For example, a distance between the antenna 0 and the antenna 1 is λ/2, and a distance between the antenna 0 and the antenna 2 is $\sqrt{3}\lambda/2$ .

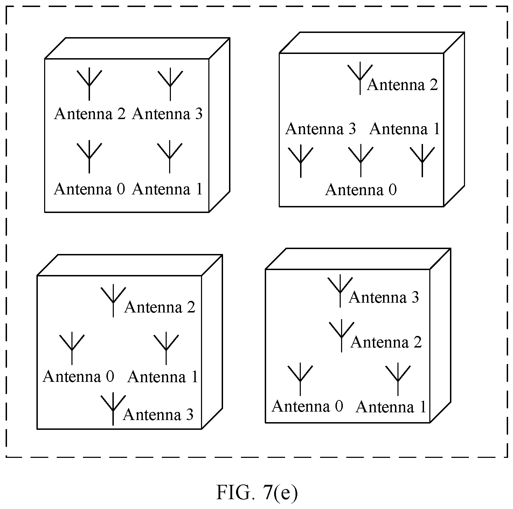

It may be understood that a case in which there are more than three antennas also falls within the scope of this application. For example, as shown in FIG. 7(e), four antennas, namely, an antenna 0, an antenna 1, an antenna 2, and an antenna 3, are distributed in a shape of a rectangle. Any three of the four antennas are distributed in the foregoing L shape or distributed in the forging shape of the triangle.

For example, the first electronic device 100 obtains a transverse coming direction of the UWB signal based on a phase difference obtained when the UWB signal from the second electronic device 300 reaches two transversely distributed antennas of the UWB module 150, and obtains a longitudinal coming direction of the UWB signal based on a phase difference obtained when the UWB signal from the second electronic device 300 reach two longitudinally distributed antennas of the UWB module 150. Further, the first electronic device 100 obtains a coming direction of the UWB signal based on the transverse coming direction and the longitudinal coming direction.

In some other examples, the UWB module 150 of the first electronic device 100 may include only one antenna. In this case, at least three first electronic devices 100 need to be used. The at least three first electronic devices 100 need to be distributed in an L shape or in a shape of a triangle, and cooperate with each other to obtain a coming direction of the UWB signal. A specific principle is similar to the foregoing descriptions. Details are not described herein again.

A quantity and a distribution of antennas in the UWB module of the first electronic device 100 are not limited in embodiments of this application provided that the coming direction of the UWB signal can be obtained.

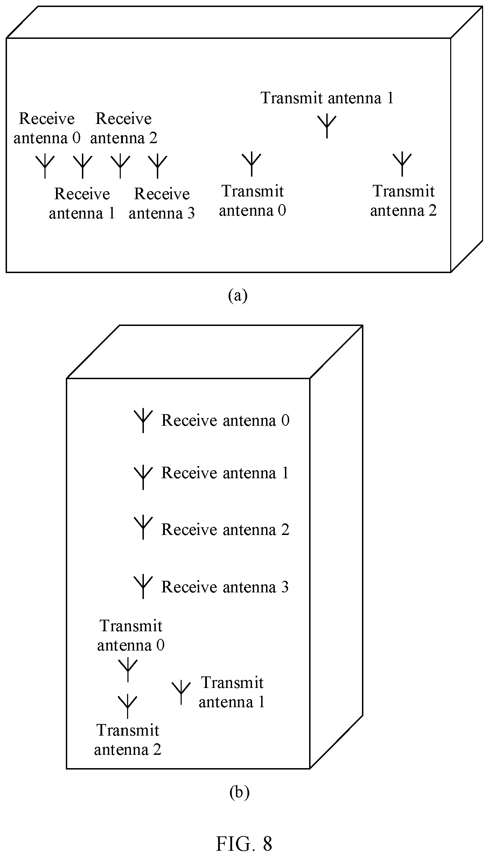

For example, FIG. 8 shows several antenna distributions of the millimeter-wave radar module in the first electronic device according to an embodiment of this application. For example, the transmit antenna includes a transmit antenna 0, a transmit antenna 1, and a transmit antenna 2. The receive antenna includes a receive antenna 0, a receive antenna 1, a receive antenna 2, and a receive antenna 3. A distribution of the transmit antenna 0, the transmit antenna 1, and the transmit antenna 2, and a distribution of the receive antenna 0, the receive antenna 1, the receive antenna 2, and the receive antenna 3 may be shown in (a) or (b) in FIG. 8. The transmit antenna is configured to transmit an electromagnetic signal (for example, an LFMCW) operating in a millimeter-wave band. The receive antenna is configured to receive a signal that is reflected by a reflector (an object or a human body) and that is of the electromagnetic signal operating in the millimeter-wave band. The millimeter-wave radar module 160 obtains a difference frequency signal based on the transmitted signal and the received signal, and determines a position of the object or the human body based on the difference frequency signal.

As shown in FIG. 8, three transmit antennas and four receive antennas are located on a same longitudinal plane (for example, a vertical plane). The three transmit antennas are distributed in a shape of a triangle on the longitudinal plane. In an example, as shown in (a) in FIG. 8, the transmit antenna 0 and the transmit antenna 2 are located on a same transverse plane (for example, a horizontal plane). The four receive antennas are located on a same transverse line (for example, a horizontal line). For example, distances between any two receive antennas are equal (for example, are $\lambda L/2$). A distance between the transmit antenna 0 and the transmit antenna 2 is equal (for example, is $2\lambda L$). A distance between the transmit antenna 1 and the transmit antenna 0 and a distance between the transmit antenna 1 and the transmit antenna 2 in a longitudinal direction are both equal (for example, are both $\lambda L/2$). Herein, $\lambda L$ is a wavelength of a highest frequency of the linear frequency-modulated continuous signal. In another example, as shown in (b) in FIG. 8, the transmit antenna 0 and the transmit antenna 2 are located on a same longitudinal line (for example, a vertical line). The four receive antennas are located on a same longitudinal line (for example, a vertical line). Distances between any two receive antennas are equal (for example, are $\lambda L/2$). A distance between the transmit antenna 0 and the transmit antenna 2 is equal (for example, is $2\lambda L$). A distance between the transmit antenna 1 and the transmit antenna 0 and a distance between the transmit antenna 1 and the transmit antenna 2 in a transverse direction are both equal (for example, are both $\lambda L/2$). It may be understood that a quantity and a distribution of transmit antennas and/or a quantity and a distribution of receive antennas may be in other forms. This is not limited in embodiments of this application.

A plurality of transmit antennas and a plurality of receive antennas are configured to: accurately measure a direction of a reflected signal, namely, a coming direction of the reflected signal, including a transverse coming direction (for example, a horizontal coming direction) and a longitudinal coming direction (for example, a vertical coming direction), and increase a receiving aperture of the millimeter-wave radar as much as possible. The millimeter-wave radar module 160 may calculate a transverse coming direction of the target based on a phase difference of the reflected signal on the plurality of receive antennas in the transverse direction (for example, the horizontal direction), and calculate a longitudinal coming direction of the target based on a phase difference of the reflected signal on the plurality of receive antennas in the longitudinal direction (for example, the vertical direction).

Optionally, there may be more than or fewer than three transmit antennas. Optionally, there may be more than or fewer than four receive antennas. This is not limited in this application. In an implementation, there is at least one transmit antenna, and there are at least three receive antennas.

In an implementation, there is one transmit antenna, and there are three receive antennas. The three receive antennas, namely, the receive antenna 0, the receive antenna 1, and the receive antenna 2, are distributed in the shape of the triangle. For ease of description, it is assumed that a connection line (actually, there is no connection line) between the receive antenna 0 and the receive antenna 1 is located in the transverse direction, and a connection line (actually, there is no connection line) between the receive antenna 0 and the receive antenna 2 is located in the longitudinal direction. In this way, after a transmitted signal of the transmit antenna is reflected by the reflector (the object or the human body), the three receive antennas separately receive reflected signals. The millimeter-wave radar module 160 may obtain a transverse coming direction (for example, a horizontal coming direction) of the reflected signal based on a phase difference between the reflected signals respectively received by the receive antenna 0 and the receive antenna 1, and obtain a longitudinal coming direction (for example, a vertical coming direction) of the reflected signal based on a phase difference between the reflected signals respectively received by the receive antenna 0 and the receive antenna 2. Further, a coming direction of the reflected signal may be determined based on the transverse coming direction and the longitudinal coming direction.

In another implementation, there are at least two transmit antennas, and there are at least two receive antennas. For example, there are two transmit antennas: the transmit antenna 0 and transmit antenna 1, and there are two receive antennas: the receive antenna 0 and the receive antenna 1. It is assumed that a connection line (actually, there is no connection line) between the transmit antenna 0 and the transmit antenna 1 is located in the transverse direction, and a connection line (actually, there is no connection line) between the receive antenna 0 and the receive antenna 1 is located in the longitudinal direction. After respective transmitted signals of the transmit antenna 0 and the transmit antenna 1 are separately reflected by the reflector (the object or the human body), at least one receive antenna receives reflected signals. The millimeter-wave radar module 160 may calculate a transverse coming direction (for example, a horizontal coming direction) of a signal (which may also be referred to as the reflected signal in this case), that is obtained after the transmitted signal is reflected, based on a phase difference obtained when the signals respectively transmitted by the transmit antenna 0 and the transmit antenna 1 reach a same receive antenna. After the transmitted signals of the transmit antennas are reflected by the reflector (the object or the human body), the two receive antennas respectively receive the reflected signals. A longitudinal coming direction (for example, a vertical coming direction) of the reflected signal is obtained based on a phase difference between the reflected signals respectively received by the receive antenna 0 and the receive antenna 1. Further, a coming direction of the reflected signal may be determined based on the transverse coming direction and the longitudinal coming direction.

In another implementation, there are at least two transmit antennas, and there are at least two receive antennas. For example, there are two transmit antennas: the transmit antenna 0 and transmit antenna 1, and there are two receive antennas: the receive antenna 0 and the receive antenna 1. It is assumed that a connection line (actually, there is no connection line) between the transmit antenna 0 and the transmit antenna 1 is located in the longitudinal direction, and a connection line (actually, there is no connection line) between the receive antenna 0 and the receive antenna 1 is located in the transverse direction. After transmitted signals of the two transmit antennas are separately reflected by the reflector (the object or the human body), at least one receive antenna receives reflected signals. The millimeter-wave radar module 160 may calculate a longitudinal coming direction (for example, a horizontal coming direction) of a signal (which may also be referred to as the reflected signal in this case), that is obtained after the transmitted signal is reflected, based on a phase difference obtained when the signals respectively transmitted by the transmit antenna 0 and the transmit antenna 1 reach a same receive antenna, and obtain a transverse coming direction (for example, a horizontal coming direction) of the reflected signal based on a phase difference between the reflected signals respectively received by the receive antenna 0 and the receive antenna 1. Further, a coming direction of the reflected signal may be determined based on the transverse coming direction and the longitudinal coming direction.

In another implementation, there are at least three transmit antennas, and there are at least one receive antenna. For example, there are three transmit antennas: the transmit antenna 0, the transmit antenna 1, and the transmit antenna 2, and there are one receive antenna: the receive antenna 0. The transmit antenna 0, the transmit antenna 1, and the transmit antenna 2 are distributed in the shape of the triangle. It is assumed that a connection line (actually, there is no connection line) between the transmit antenna 0 and the transmit antenna 1 is located in the transverse direction (for example, the horizontal direction), and a connection line (actually, there is no connection line) between the transmit antenna 0 and the transmit antenna 2 is located in the longitudinal direction. After transmitted signals of the transmit antenna 0, the transmit antenna 1, and the transmit antenna 2 are separately reflected by the reflector (the object or the human body), the receive antenna 0 receives reflected signals. The millimeter-wave radar module 160 may calculate a transverse coming direction (for example, a horizontal coming direction) of a signal (which may also be referred to as the reflected signal in this case), that is obtained after the transmitted signal is reflected, based on a phase difference obtained when the signals respectively transmitted by the transmit antenna 0 and the transmit antenna 1 reach a same receive antenna, and calculate a longitudinal coming direction (for example, a vertical coming direction) of a signal (which may also be referred to as the reflected signal in this case), that is obtained after the transmitted signal is reflected, based on a phase difference obtained when the signals respectively transmitted by the transmit antenna 0 and the transmit antenna 2 reach a same receive antenna.

### 3. Description of a locating principle

For ease of description, the following describes a specific locating principle in detail by using an example in which the first electronic device 100 includes the UWB module 150 and the millimeter-wave radar module 160.

It should be noted that locating means obtaining a position. In an embodiment of this application, coordinates in a coordinate system indicate a position. For example, coordinates of the first electronic device indicate a position of the first electronic device, coordinates of the second electronic device indicate the position of the second electronic device, and coordinates of the user indicates the position of the user. It may be understood that, in some other embodiments, a position may alternatively be indicated in another manner. This is not limited in embodiments of this application.

### (1) Establishment of the first coordinate system (first electronic device coordinate system)

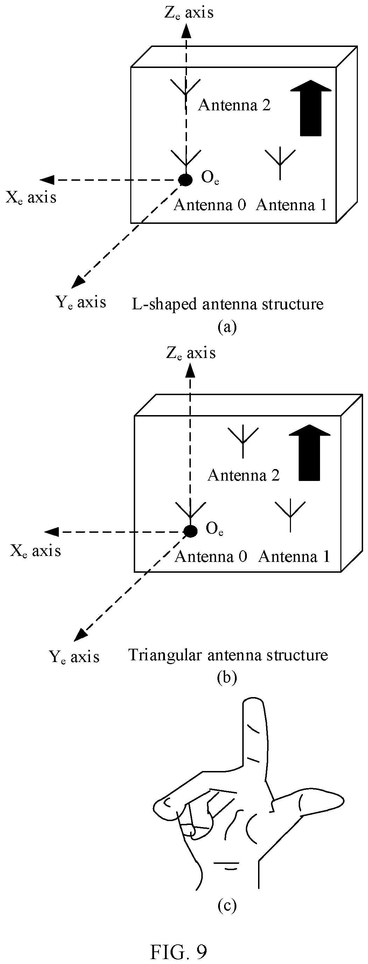

To accurately perform locating, the UWB module 150 needs to establish the first coordinate system, and specifically perform locating by using coordinates in the first coordinate system. With reference to FIG. 9, the following describes in detail a process of establishing the first coordinate system. For example, in (a) and (b) in FIG. 9, one UWB module includes three antennas. A distance between an antenna 0 and an antenna 1 and a distance between the antenna 0 and an antenna 2 are both preset distances. It is assumed that a point (for example, a center point or an end point at one end) on the antenna 0 is a coordinate origin Oe. A connection line between the antenna 0 and the antenna 1 is used as an Xe axis, and a direction in which the antenna 1 points to the antenna 0 is a positive direction of the Xe axis. In a plane on which the antenna 0, the antenna 1, and the antenna 2 are located, a straight line perpendicular to the Xe axis is used as a Ze axis, and a projection of the antenna 2 on the Ze axis is located in a positive direction of the Ze axis. In this way, the positive direction of the Ze axis can be determined. In (a) in FIG. 9, the antenna 2 is located in the positive direction of the Ze axis. In (b) in FIG. 9, the projection of the antenna 2 on the Ze axis is in the positive direction of the Ze axis. Finally, a direction of a Ye axis is determined according to a rule of a right-hand rectangular coordinate system based on directions of the Xe axis and the Ze axis. The right-hand rectangular coordinate system may be referred to as a right-hand system for short, and is one of methods for specifying a rectangular coordinate system in space. As shown in (c) in FIG. 9, positive directions of an Xe axis, a Ye axis, and a Ze axis in the right-hand rectangular coordinate system are defined as follows: The right hand is placed at a position of an origin, the thumb, the index finger, and the middle finger are at right angles to each other, when the thumb and index finger are on a same plane, the thumb points to the positive direction of the Xe axis, and the middle finger points to the positive direction of the Ze axis, a direction to which the index finger points is the positive direction of the Ye axis.

In some examples, to facilitate calculation and reduce a calculation error, the Ze axis may be set on a vertical plane, and the positive direction of the Ze axis is opposite to a gravity direction. Optionally, prompt information may be marked on an outer surface of the first electronic device 100, and indicates a correct installation manner or placement manner. In this way, the Ze axis of the first coordinate system is located on the vertical plane, and the positive direction of the Ze axis is opposite to the gravity direction. For example, as shown in (a) in FIG. 9 or (b) in FIG. 9, an arrow is marked on an outer surface of the UWB base station, and is for prompting to install or place the first electronic device 100 in a direction indicated by the arrow (a direction of the arrow faces upward). In this way, the Ze axis of the first coordinate system is located on the vertical plane, and the positive direction of the Ze axis is opposite to the gravity direction. For example, when installing the first electronic device, the user may make an arrow on an outer surface of the first electronic device parallel to a wall surface, and a direction of the arrow face upward. In this way, the Ze axis of the first coordinate system is located on the vertical plane, and the positive direction of the Ze axis is opposite to the gravity direction. For example, when installing the first electronic device, the user may use an instrument, for example, a plumb bob, to make an arrow on an outer surface of the first electronic device parallel to a vertical line determined by the plumb bob, and a direction of the arrow face upward. In this way, the Ze axis of the first coordinate system is located on the vertical plane, and the positive direction of the Ze axis is opposite to the gravity direction.

Alternatively, the first electronic device 100 may include only one UWB module, and the UWB module 150 may include only one antenna. As a result, three first electronic devices 100 need to cooperate with each other to establish the first coordinate system. In this case, for a detailed process of establishing the first coordinate system, refer to the Chinese Patent Application No. 202110872916.6. Details are not described herein again. It should be noted that the Chinese Patent Application No. 202110872916.6 is incorporated into this application by reference in its entirety, and falls within the scope of this application.

For example, a positive direction of a Ye axis is alternatively determined according to a rule of a left-hand rectangular coordinate system based on directions of the Xe axis and the Ze axis. The left-hand rectangular coordinate system may be referred to as a left-hand system for short, and is one of methods for specifying a rectangular coordinate system in space. Positive directions of an Xe axis, a Ye axis, and a Ze axis in the left-hand rectangular coordinate system are defined as follows: The left hand is placed at a position of an origin, the thumb, the index finger, and the middle finger are at right angles to each other, when the thumb and index finger are on a same plane, the thumb points to the positive direction of the Xe axis, and the middle finger points to the positive direction of the Ze axis, a direction to which the index finger points is the positive direction of the Ye axis.

For ease of description, in embodiments of this application, the Ye axis, a Yb axis, and a Yt axis, and positive directions of the Ye axis, the Yb axis, and the Yt axis are determined according to the rule of the right-hand rectangular coordinate system. A person skilled in the art should understand that determining the Ye axis, the Yb axis, the Yt axis, and the positive direction of the Ye axis, the Yb axis, and the Yt axis according to the rule of the left-hand rectangular coordinate system or in another manner also falls within the scope of this application. In addition, names of any two or more axes in any one of the first coordinate system, the second coordinate system, and a third coordinate system may be switched provided that the foregoing rule or manner is satisfied. Certainly, the positive directions of the three axes in the first coordinate system may alternatively be determined according to another rule. Details are not described herein again.

Optionally, the first coordinate system may be automatically established after a specific input is received, or may be pre-established.

For example, after the first electronic device 100 is installed according to a requirement of the prompt information, when the first electronic device 100 receives the specific input, the first electronic device 100 automatically establishes the first coordinate system. The specific input may be a user input, or may be a non-user input (for example, an instruction message from another device, for example, a remote control, is received).

For example, after the first electronic device 100 is installed according to a requirement of the prompt information, when the first electronic device 100 receives the specific input, the first electronic device 100 automatically invokes related information locally or from a server, to invoke the pre-established first coordinate system. Unless otherwise specified, the server in this application may be a hub device in a home, or may be a cloud server.

It should be noted that the point on the antenna 0 is used as the origin of the first coordinate system. This is merely an example. A point on another antenna (for example, the antenna 1) may alternatively be the origin of the first coordinate system.

**(2) Establishment of the second coordinate system**

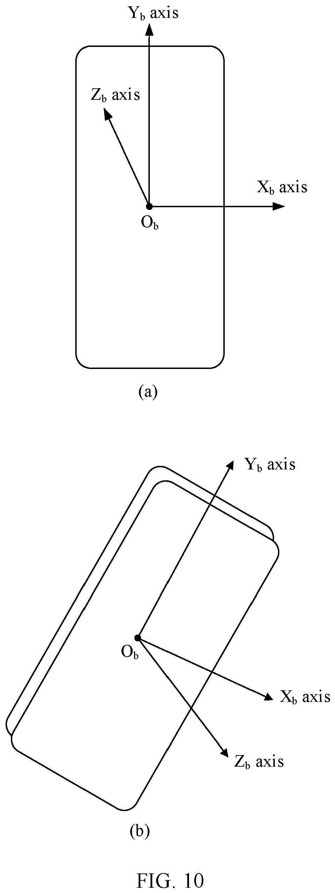

For example, FIG. 10 shows a process of establishing the second coordinate system according to an embodiment of this application. As shown in (a) in FIG. 10, an edge contour of the second electronic device includes four sides: two vertical sides and two horizontal sides. Ob is the gravity center or the center of the second electronic device. An axis that includes the point Ob and is parallel to the transverse edge of the second electronic device is an Xb axis. A positive direction of the Xb axis points to the right side of the second electronic device. An axis that includes the point Ob and is parallel to the vertical edge of the second electronic device is a Yb axis. A positive direction of the Yb axis points to the upper side of the second electronic device. A pointing direction of the second electronic device is the positive direction of the Yb axis. A Zb axis is perpendicular to a plane on which the Xb axis and the Yb axis are located. A positive direction of the Zb axis is determined according to a rule of a right-hand rectangular coordinate system. Optionally, Ob may be the center of the second electronic device, or Ob may be the center of an IMU module of the second electronic device (on a premise that the second electronic device includes the IMU module). (b) in FIG. 10 is a three-dimensional diagram of the second electronic device in (a) in FIG. 10.

It should be noted that FIG. 10 shows only an example of the second coordinate system. The second coordinate system may alternatively be defined according to another rule. For example, a coordinate origin Ob may alternatively be any point on the second electronic device, or any point outside the second electronic device. In addition, directions of the three axes of the second coordinate system are not limited to the positive directions of the Xb axis, the Yb axis, and the Zb axis shown in (a) or (b) in FIG. 10.

Optionally, the second coordinate system may be pre-established. For example, during delivery of the second electronic device, the second coordinate system has been established, and related information of the second coordinate system is stored locally or on a server. When the second electronic device is started or when the first electronic device receives a specific input, the second electronic device invokes the related information of the second coordinate system locally or from the server.

**(3) Establishment of the third coordinate system**

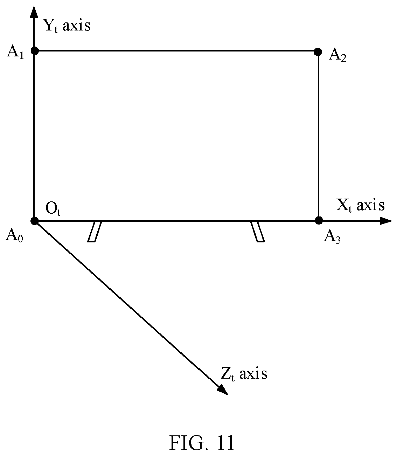

For example, FIG. 11 shows a process of establishing the third coordinate system according to an embodiment of this application. As shown in FIG. 11, an edge contour of the second electronic device includes four sides: a first side A0A1, a second side A1A2, a third side A2A3, and a fourth side A3A0. The first side A0A1 and the third side A2A3 are vertical sides, and the second side A1A2 and the fourth side A3A0 are horizontal sides. Optionally, an intersection (namely, the lower left corner of a display area of the second electronic device) at which the leftmost side of the display area of the second electronic device intersects with the bottommost side of the display area of the second electronic device is a coordinate origin Ot. An axis that includes the point Ot and is parallel to A3A0 is an Xt axis, and a positive direction of the Xt axis is a pointing direction from a point A0 to a point A3. An axis that includes the point Ot and is parallel to A0A1 is a Yt axis, and a positive direction of the Yt axis is a pointing direction from the point A0 to a point A1. A Zt axis is perpendicular to a plane on which the Xt axis and the Yt axis are located. A positive direction of the Zt axis is determined according to a rule of a right-hand rectangular coordinate system.

It should be noted that FIG. 11 shows only an example of the third coordinate system. The third coordinate system may alternatively be defined according to another rule. Optionally, Ot may be the center of the display area of the second electronic device, or any point of the display area of the second electronic device. In addition, the forward directions of the three axes of the third coordinate system are not limited to the forward direction indicated by the Xt axis, the Yt axis, and the Zt axis shown in FIG. 11.

It should be noted that, when the edge contour of the display area of the second electronic device is an edge contour of the second electronic device, the point A0 of the second electronic device coincides with the point Ot. When the edge contour of the display area of the second electronic device is not an edge contour of the second electronic device, for example, when there is a frame outside the display area of the second electronic device, the point A0 of the second electronic device does not coincide with the point Ot.

Optionally, the third coordinate system may be pre-established. For example, during delivery of the second electronic device, the third coordinate system has been established, and related information of the third coordinate system is stored locally or on a server. When the second electronic device is started, or when the second electronic device receives a trigger, the second electronic device invokes the third coordinate system locally or from the server.

**(4) Calculation of coordinates in the first coordinate system**

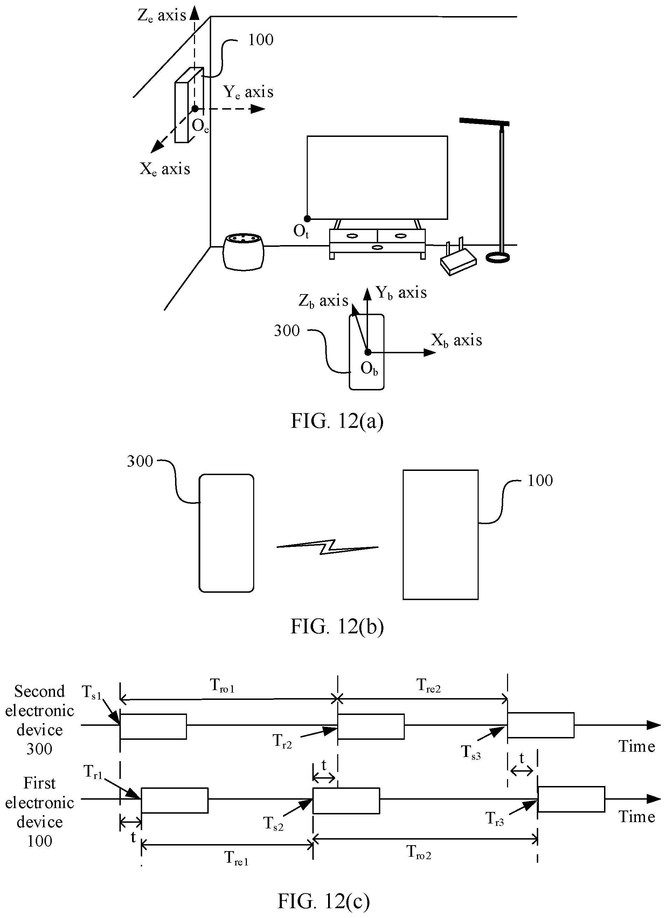

With reference to FIG. 12(a) to FIG. 12(f), the following specifically describes a calculation principle of locating coordinates of the second electronic device 300 by the first electronic device 100 in the first coordinate system. In FIG. 12(a) to FIG. 12(f), the second electronic device 300 includes a UWB module. For example, the second electronic device 300 is a mobile device (for example, a smartphone or a remote control). As shown in FIG. 12(a), the first electronic device 100 and the second electronic device 300 are located in a same room or area. The first electronic device 100 has established the first coordinate system, and the second electronic device 300 has established the second coordinate system. As shown in FIG. 12(b), the first electronic device 100 and the second electronic device 300 may perform UWB communication. Accordingly, a distance between the second electronic device 300 and the first electronic device 100 and a direction of the second electronic device 300 relative to the first electronic device 100 may be determined. As a result, the coordinates of the second electronic device 300 in the first coordinate system may be determined.

   1: Measure a distance L between the first electronic device and the second electronic device.

For example, as shown in FIG. 12(c), the distance L between the first electronic device and the second electronic device may be obtained in the following manner.
The first electronic device 100 may measure the distance L between the first electronic device 100 and the second electronic device 300 by using a two-way ranging method. The two-way ranging method includes a single-sided two-way ranging (Single-sided Two-way Ranging, SS-TWR) method and a double-sided two-way ranging (Double-sided Two-way Ranging, DS-TWR) method. The DS-TWR method is used as an example herein to briefly describe the ranging method.
In the DS-TWR method, a timestamp of a round trip between the first electronic device 100 and the second electronic device 300 is recorded, and finally time of flight is obtained. In the DS-TWR method, although response duration increases, a ranging error is reduced. The double-sided two-way ranging method includes two methods, namely, a four-message manner and a three-message manner, based on different quantities of sent messages.
The three-message manner is used as an example. The second electronic device 300 sends a ranging request packet (namely, a 1st packet), and records a sending time point Ts1. After receiving the request packet, the first electronic device 100 records a receiving time point Tr1. A time difference t between Tr1 and Ts1 is transmission duration of the packet between the two devices. It takes Tre1 for the first electronic device 100 to process the request packet. Then, the first electronic device 100 sends a response packet (that is, a 2nd packet), and records a sending time point Ts2. After receiving the response packet, the second electronic device 300 records a receiving time point Tr2. A time difference between Tr2 and Ts2 is t. A time difference between the time point at which the second electronic device 300 sends the 1st packet and the time point at which the second electronic device 300 receives the 2nd packet is a time difference Tro1. It takes Tre2 for the second electronic device 300 to process the response packet. The second electronic device 300 sends a last packet (namely, a 3rd packet), and records a sending time point Ts3. The first electronic device 100 receives the 3rd packet, and records a receiving time point Tr3. A time difference between Tr3 and Ts3 is t. In addition, a time difference from the time point at which the first electronic device 100 starts to send the 2nd packet to the time point at which the first electronic device 100 receives the 3rd packet is Tro2. Consequently, transmission duration t of the packet between the two devices may be calculated according to the following formula (1), and the distance L between the first electronic device 100 and the second electronic device 300 may be calculated according to formula (2).

$$t = \frac{T_{ro1} \times T_{ro2} - T_{re1} \times T_{re2}}{T_{ro1} \times T_{ro2} + T_{re1} \times T_{re2}} \qquad \qquad \text{formula (1)}$$

$$L = c \times t \qquad \qquad \text{formula (2)}$$

Herein, c is a transmission rate of a UWB signal in a medium, and c is generally a light speed through selection.
Optionally, a ranging request packet may alternatively be sent by the first electronic device 100. Correspondingly, a response packet may alternatively be sent by the second electronic device 300. Correspondingly, a last packet may alternatively be sent by the first electronic device 100. The first electronic device 100 or the second electronic device 300 may calculate L according to formula (1) and formula (2).
It should be noted that the distance L between the first electronic device 100 and the second electronic device 300 may alternatively be obtained through calculation in another manner, without being limited to the manner enumerated above.

2: Determine the signal coming direction of the second electronic device.

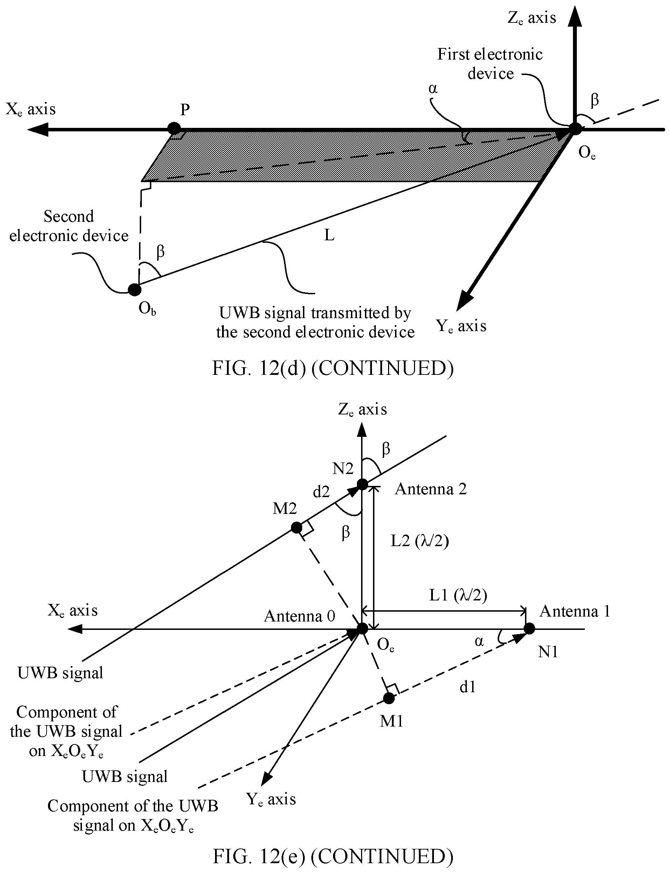

A direction of the second electronic device 200 may be indicated by using a coming direction of a signal that is transmitted by the second electronic device 200 and that is measured by the first electronic device 100. In this application, two angles $\alpha$ and $\beta$ may indicate the coming direction of the signal transmitted by the second electronic device 200. For example, as shown in FIG. 12(d), $\alpha$ is an included angle between a component that is of the UWB signal transmitted by the second electronic device and that is on a plane XeOeYe of the first coordinate system and a negative axis of the Xe axis. Usually, $\alpha \in [0, \pi]$. Herein, $\alpha$ may alternatively be understood, when it is assumed that a vector is constituted from the UWB module of the second electronic device to the UWB module of the first electronic device, as an included angle between a component of the vector on a plane XeOeYe and a negative axis of the Xe axis. Herein, $\beta$ is an included angle between the UWB signal transmitted by the second electronic device and a positive axis of the Ze axis of the first coordinate system. Usually, $\beta \in [0, \pi]$. $\beta$ may alternatively be understood, when it is assumed that the vector is constituted from the UWB module of the second electronic device to the UWB module of the first electronic device, as an included angle between the vector and a positive axis of the Ze axis.

With reference to two antenna distribution types, namely, the L shape and the triangle, in the UWB module of the first electronic device, the following uses three antennas as an example to describe in detail a process of solving $\alpha$ and $\beta$.

In an example, in FIG. 12(e), the UWB module of the first electronic device uses a three-antenna structure in the L shape. The first electronic device may determine the included angle $\alpha$ based on a UWB signal received by an antenna 1, and may determine the included angle $\beta$ based on a UWB signal received by an antenna 2. Usually, L1 and L2 are far less than the distance L between the second electronic device and the first electronic device. As a result, when reaching the UWB module of the first electronic device, the UWB signals may be considered as being parallel. Similarly, when the UWB signals reach the UWB module of the first electronic device, components of the UWB signals on the plane XeOeYe may also be considered as being parallel. As shown in FIG. 12(e), the UWB signals are represented by using two parallel solid lines, and the components of the UWB signals on the plane XeOeYe are represented by using two parallel dotted lines. A distance between an antenna 0 and the antenna 1 is L1, and a distance between the antenna 0 and the antenna 2 is L2. A straight line on which M1N1 is located and that is perpendicular to OeM1 is drawn passing through the point Oe. A straight line on which M2N2 is located and that is perpendicular to OeM2 is drawn passing through the point Oe. N1 is an intersection of a straight line on which the component of the UWB signal on the plane XeOeYe is located and the Xe axis. N2 is an intersection of a straight line on which the UWB signal is located and the Ze axis. An included angle between the UWB signal and the positive axis of the Ze axis is $\beta$. An included angle between the component of the UWB signal on the plane XeOeYe and the positive axis of the Xe axis is $\alpha$. Preferably, both L1 and L2 are $\lambda/2$. Herein, $\lambda$ is a wavelength of the UWB signal. Phases that are of a same UWB signal and that are measured by the antenna 0, the antenna 1, and the antenna 2 are respectively $\varphi_0$, $\varphi_1$, and $\varphi_2$. A phase difference between the antenna 1 and the antenna 0 is that $\Delta\varphi_1 = \varphi_1 - \varphi_0$. A phase difference between the antenna 2 and the antenna 0 is that $\Delta\varphi_2 = \varphi_2 - \varphi_0$. Because $\varphi_0$, $\varphi_1$, and $\varphi_2$ are obtained through measurement, $\Delta\varphi_1$ and $\Delta\varphi_2$ can be obtained through calculation. Because $\lambda/2$ corresponds to a phase difference $\pi$, $\alpha$ and $\beta$ may be obtained through calculation according to formula (3) and formula (4) with reference to a cosine formula, d1, d2, L1, and L2.

$$\alpha = \arccos\left(\frac{d1}{L1}\right) = \arccos\left(\frac{\Delta\varphi_1 \times (\lambda/2\pi)}{L1}\right) \qquad \text{formula (3)}$$

$$\beta = \arccos\left(\frac{d2}{L2}\right) = \arccos\left(\frac{\Delta\varphi_2 \times (\lambda/2\pi)}{L2}\right) \qquad \text{formula (4)}$$

When L1 is $\lambda/2$, $\alpha = \arccos\left(\frac{\Delta\varphi_1 \times (\lambda/2\pi)}{\lambda/2}\right) = \arccos(\Delta\varphi_1/\pi)$.

When L2 is $\lambda/2$, $\beta = \arccos\left(\frac{\Delta\varphi_2 \times (\lambda/2\pi)}{\lambda/2}\right) = \arccos(\Delta\varphi_2/\pi)$.

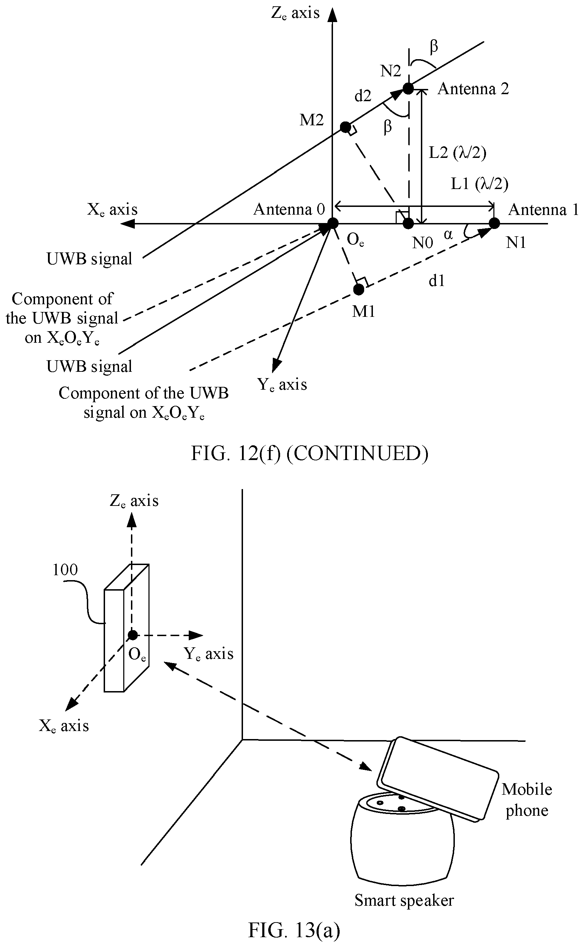

In an example, as shown in FIG. 12(f), the UWB module of the first electronic device uses a three-antenna structure in a shape of the triangle. The same as that in the descriptions in FIG. 12(e), when reaching the UWB module of the first electronic device, UWB signals may be considered as being parallel. Similarly, when the UWB signals reach the UWB module of the first electronic device, components of the UWB signals on the plane XeOeYe may also be considered as being parallel. As shown in FIG. 12(f), the UWB signals are represented by using two parallel solid lines, and the components of the UWB signals on the plane XeOeYe are represented by using two parallel dotted lines. A distance between an antenna 0 and an antenna 1 is L1. A distance between the projections of the antenna 0 and an antenna 2 on the Ze axis is L2. A point N0 is the center point of the antenna 1 and the antenna 0 on the Xe axis. A straight line on which M1N1 is located and that is perpendicular to OeM1 is drawn passing through the point Oe. A straight line on which M2N2 is located and that is perpendicular to N0M2 is drawn passing through the point N0. N1 is an intersection of a straight line on which a component of the UWB signal on the plane XeOeYe is located and the Xe axis. N2 is an intersection of a straight line on which the UWB signal is located and the Ze axis.

An included angle between the UWB signal and the positive axis of the Ze axis is β. An included angle between the component of the UWB signal on the plane XeOeYe and the negative axis of the Xe axis is α. Preferably, both L1 and L2 are λ/2. Herein, λ is a wavelength of the UWB signal.

A calculation formula for α is the same as formula (3). Details are not described herein again. When β is calculated, a difference between a phase $\varphi_0 + \Delta\varphi_1/2$ obtained when the UWB signal transmitted by the second electronic device reaches N0 and a phase $\varphi_2$ obtained when the UWB signal transmitted by the second electronic device reaches the antenna 2 is first calculated, that is,

$$\varphi_2 - (\varphi_0 + \Delta\varphi_1/2) = \varphi_2 - \varphi_0 - \Delta\varphi_1/2 = \Delta\varphi_2 - \Delta\varphi_1/2$$

Then, a wave path difference d2 obtained when the UWB signal transmitted by the second electronic device reaches N0 and the UWB signal transmitted by the second electronic device reaches the antenna 2 is calculated according to formula (5). Next, β is obtained through calculation according to formula (6).

$$d2 = (\Delta\varphi_2 - \Delta\varphi_1/2) \times (\lambda/2\pi) \qquad \text{formula (5)}$$

$$\beta = \arccos(d2/L2) = \arccos(\frac{(\Delta\varphi_2 - \Delta\varphi_1/2) \times (\lambda/2\pi)}{L2}) \qquad \text{formula (6)}$$

3: Calculate the coordinates of the second electronic device in the first coordinate system based on the distance L between the second electronic device and the first electronic device and the signal coming direction of the second electronic device.

As shown in FIG. 12(d), the first electronic device may calculate, according to formula (7) based on the distance L between the second electronic device and the first electronic device and the signal coming direction (the angle α and the angle β) of the second electronic device, coordinates ($x^e$, $y^e$, $z^e$) of the second electronic device in the first coordinate system established by the first electronic device.

$$\begin{cases} x^e = L\sin\beta\cos\alpha \\ y^e = L\sin\beta\sin\alpha \\ z^e = -R\cos\beta \end{cases} \qquad \text{formula (7)}$$

The first electronic device 100 and the second electronic device 300 may obtain the distance L between the first electronic device 100 and the second electronic device 300 through, for example, communication interaction shown in FIG. 12(c). The first electronic device 100 may determine the coming direction of the received UWB signal based on the UWB signal. In this way, the first electronic device 100 can obtain the direction and the distance of the second electronic device 300 relative to the first electronic device 100, to obtain the coordinates of the second electronic device 300 in the first coordinate system.

When some second electronic devices each include a UWB module, the coordinates of the second electronic device in the first coordinate system may be obtained in real time or cyclically through UWB signal communication between the second electronic device and the first electronic device.

When some second electronic devices each include no UWB module, for example, a smartphone includes a UWB module, a smart speaker includes no UWB module, or a smart air conditioner includes no UWB module, there may be two marking manners.

Marking manner 1: As shown in FIG. 13(a), the smartphone is moved to the smart speaker, coordinates of the smartphone in the first coordinate system are obtained through UWB signal communication between the smartphone and the first electronic device. The coordinates are marked as coordinates of the smart speaker in the first coordinate system.

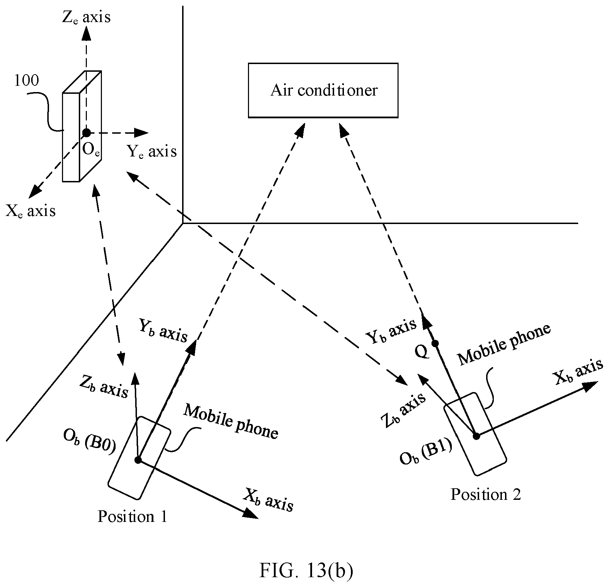

Marking manner 2: As shown in FIG. 13(b), the smartphone is first used to point to the smart air conditioner at a position 1, so that the Yb axis of the second coordinate system faces a first point (for example, a switch button) on the smart air conditioner. Coordinates 1 of the position 1 in the first coordinate system are obtained through UWB signal communication between the smartphone and the first electronic device. The smartphone further includes an IMU module. An attitude angle 1 of the smartphone at the position 1 is determined by using the IMU module. A straight line 1 established when the smartphone points to the first point on the smart air conditioner at the position 1 may be determined based on the coordinates 1 and the attitude angle 1. Then, the smartphone is used to point to the smart air conditioner at a position 2, so that the Yb axis of the second coordinate system faces the first point on the smart air conditioner. Coordinates 2 of the position 2 in the first coordinate system are obtained through UWB signal communication between the smartphone and the first electronic device. An attitude angle 2 of the smartphone at the position 2 is determined by using the IMU module. A straight line 2 established when the smartphone points to a second point on the smart air conditioner at the position 2 may be determined based on the coordinates 2 and the attitude angle 2. Coordinates, namely, coordinates of the smart air conditioner in the first coordinate system, of an intersection of the straight line 1 and the straight line 2 are calculated.

It should be noted that the smart speaker or the smart air conditioner are merely examples. The smart speaker is for representing a second electronic device easily touched by a mobile device, for example, the smartphone, held by the user. The smart air conditioner is for representing a second electronic device uneasily touched by a mobile device, for example, the smartphone, held by the user.

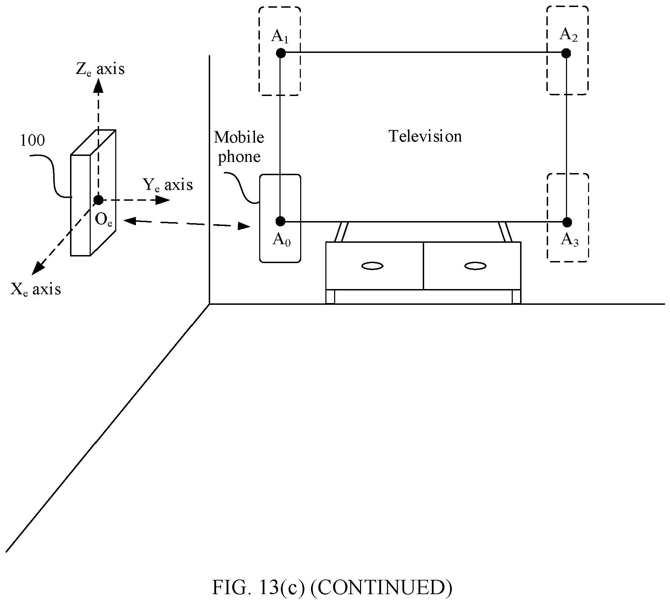

When some second electronic devices each include no UWB module, for example, a smartphone includes a UWB module, and a smart television includes no UWB module, the smart television may be marked a plurality of times by using the smartphone including the UWB module. As shown in FIG. 13(c), the smartphone is moved to a point A0 of the smart television to mark a position. The first electronic device obtains coordinates of a lower left corner contour point of the smart television. Correspondingly, in the foregoing manner, the first electronic device may obtain coordinates of a plurality of contour points (for example, the lower left corner contour point, an upper left corner contour point, and a lower right corner contour point) of the smart television. If A0, A1, A2, and A3 are marked, the first electronic device may obtain coordinates of four corner contour points. If A0, A1, and A2 are marked, the first electronic device may obtain coordinates of three corner contour points. If A0 and A2 are marked, the first electronic device may obtain coordinates of two corner contour points. In this application, which corner contour points are selected from the four corner contour points is not limited provided that a contour range of the smart television can be finally obtained. Optionally, the contour range may be a contour range of a display area of the smart television. Optionally, the display area may include a frame of a display of the smart television, or may include no frame of a display of the smart television.

Preferably, the smartphone may be moved to more than three different positions of the display area of the smart television. When being moved to one position of the display area of the smart television, coordinates of the smartphone are marked as coordinates of the position of the display area of the smart television based on input of the user. In this way, the operation is repeated, and coordinates of the more than three different positions of the display area of the smart television may be marked. Optionally, coordinates of three positions of the display area of the smart television are marked. Similarly, coordinates of a position in a front area of the smart television may be marked. In a process of marking the more than three different positions of the display area of the smart television, pointing directions, orientations, and the like of the smartphone at the more than three different positions are not required to be consistent. In other words, an orientation, a pointing direction, and the like of the smart television during marking are not limited.

Optionally, the more than three positions of the display area of the smart television may be more than three positions (for example, a 1/2 place and a 1/3 place) on an edge contour of the display area (for example, a horizontal side contour or a vertical contour of the display area) of the smart television, or may be at least three positions in a central part of the display area of the smart television.

Optionally, the second electronic device including the UWB module may not only mark the second electronic device including no UWB module, but also mark a spatial area, for example, mark a range of a three-dimensional spatial area. The following uses, as an example, the smartphone including the UWB module.

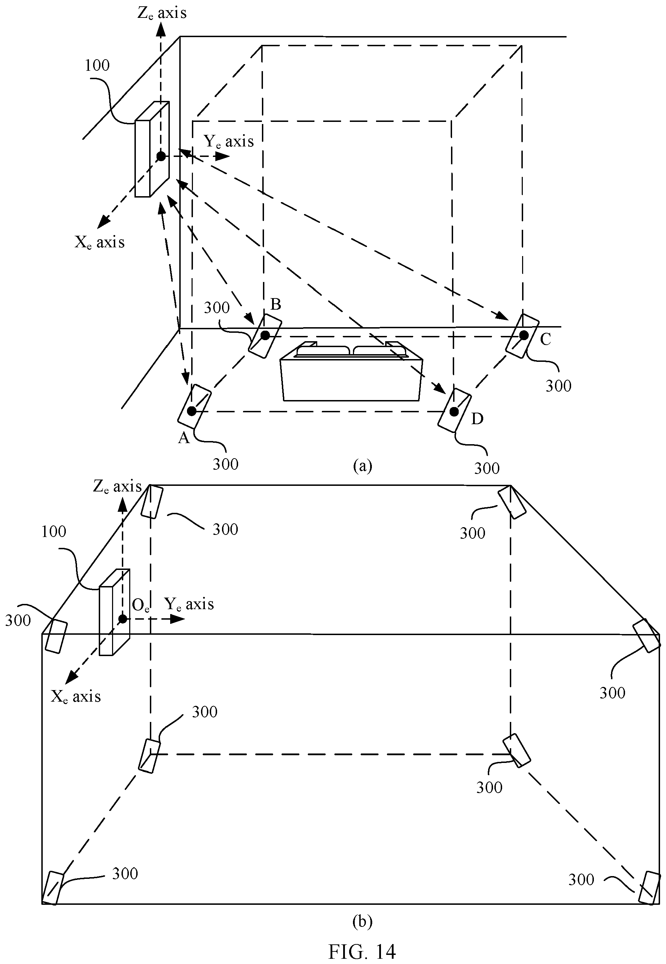

For example, as shown in (a) in FIG. 14, the smartphone is separately placed at four positions A, B, C, and D. The first electronic device 100 separately obtains coordinates $(x_A^e, y_A^e, z_A^e), (x_B^e, y_B^e, z_B^e), (x_C^e, y_C^e, z_C^e)$, and $(x_D^e, y_D^e, z_D^e)$ of the four positions A, B, C, and D in the first coordinate system based on the foregoing principle. A vertical line $(x_A^e, y_A^e, ze)$ passing through the position A, a vertical line $(x_B^e, y_B^e, ze)$ passing through the position B, a vertical line $(x_C^e, y_C^e, ze)$ passing through the position C, and a vertical line $(x_D^e, y_D^e, ze)$ passing through the

position D constitute a three-dimensional area through enclosure. Herein, ze may be a preset value, or may be a height of the room or the area.

In another example, as shown in (b) in FIG. 14, the smartphone is separately placed at eight vertex positions of a three-dimensional spatial area. The first electronic device 100 separately obtains coordinates of the eight vertex positions in the first coordinate system based on the foregoing principle. In this way, a coordinate range of the three-dimensional spatial area can be obtained. For example, the three-dimensional spatial area is a room. In other words, a coordinate range of the room in the first coordinate system is obtained. The foregoing uses only the vertex position as an example. An actual area may be determined based on a position in which the smartphone is placed. For example, the smartphone may not be placed at the vertex position. In this way, a determined area is an area less than an entire area of the room.

**(5) UWB-based conversion between different coordinate systems**

In this application, coordinate conversion in the different coordinate systems may be performed in a vector manner. Specifically, distances between two points are the same in the different coordinate systems, but direction representations of vectors constituted by the two points may be different in the different coordinate systems. For example, coordinates of the point Oe in the first coordinate system may be converted into coordinates of the point Oe in the second coordinate system in the vector manner. For example, conversion is performed in a manner of $\overrightarrow{O_eO_b}$. Distances (which are both L) of the vector $\overrightarrow{O_eO_b}$ in the first coordinate system and the second coordinate system are the same, but a direction that is of the vector $\overrightarrow{O_eO_b}$ and that is represented by using the first coordinate system is different from a direction that is of the vector $\overrightarrow{O_eO_b}$ and that is represented by using the second coordinate system. By obtaining a relative direction change between the first coordinate system and the second coordinate system, the direction that is of the vector $\overrightarrow{O_eO_b}$ and that is represented by using the second coordinate system may be learned when the direction that is of the vector $\overrightarrow{O_eO_b}$ and that is represented by using the first coordinate system is known. The coordinates of the point Oe in the second coordinate system may be obtained with reference to coordinates of the point Oe and the point Ob in the first coordinate system and coordinates of the point Ob in the second coordinate system.

The foregoing coordinate conversion manner of a same point in the different coordinate systems is merely an example. A coordinate conversion manner is not limited in this application.

A relative direction change of the different coordinate systems may be expressed by using a pitch (pitch) φ, a yaw (yaw) ψ, and a roll (roll) θ that are between the coordinate systems, for example, a pitch, a yaw, and a roll of the second coordinate system relative to the first coordinate system, and a pitch, a yaw, and a roll of the third coordinate system relative to the first coordinate system. The yaw may also be referred to as a drift angle or a heading angle. In order to obtain a pitch, a yaw, and a roll between the three coordinate systems, it is necessary to first assume that the coordinate origins of the three coordinate systems are converged to one point. For example, the coordinate origin Oe of the UWB base station is moved in parallel to the coordinate origin Ob of the second coordinate system. Correspondingly, the first coordinate system is also moved accordingly. Definitions of the pitch, the yaw, and the roll are well known to a person skilled in the art. Details are not described herein again.

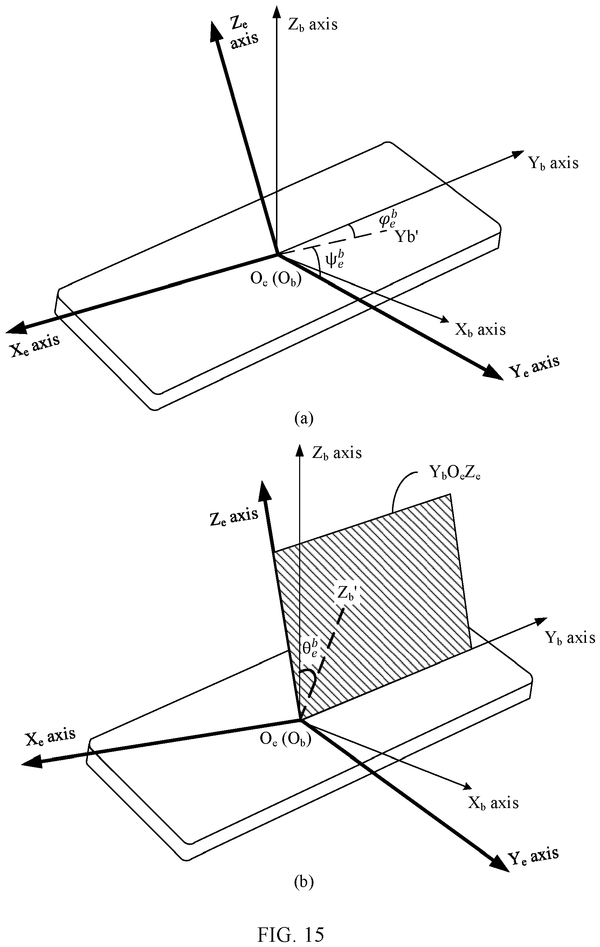

For example, FIG. 15 shows a pitch $\varphi_e^b$, a yaw $\psi_e^b$, and a roll $\theta_e^b$ of the second coordinate system relative to the first coordinate system.

The coordinate origin Ob of the second coordinate system coincides with the coordinate origin Oe of the first coordinate system after parallel movement. The three axes of the second coordinate system are the Xb axis, the Yb axis, and the Zb axis. The three axes of the first coordinate system are the Xe axis, the Ye axis, and the Ze axis. As shown in (a) in FIG. 15, OeYb' (namely, ObYb') is a projection of the Yb axis on the plane XeOeYe of the first coordinate system. As shown in (b) in FIG. 15, OeZb' (namely, ObZb') is a projection of the Zb axis on a plane YbObZe.

The pitch $\varphi_e^b$ of the second coordinate system relative to the first coordinate system is an included angle between the Yb axis of the second coordinate system and the plane XeOeYe of the first coordinate system. In other words, $\varphi_e^b$ is an included angle between ObYb' and the Yb axis. When a component of ObYb on the Ze axis is located on the positive axis of the Ze axis, $\varphi_e^b$ is positive. When a component of ObYb on the Ze axis is located on a negative axis of the Ze axis, $\varphi_e^b$ is negative.

The yaw $\psi_e^b$ of the second coordinate system relative to the first coordinate system is an included angle between the projection of the Yb axis of the second coordinate system on the plane XeOeYe of the first coordinate system and the Ye axis of the first coordinate system. In other words, $\psi_e^b$ is an included angle between ObYb' and the Ye axis. When a component of ObYb' on the Xe axis is located on the positive axis of the Xe axis, $\psi_e^b$ is positive. When a component of ObYb' on the Xe axis is located on the negative axis of the Xe axis, $\psi_e^b$ is negative.

The roll $\theta_e^b$ of the second coordinate system relative to the first coordinate system is an included angle between the Zb axis of the second coordinate system and a plane YbOeZe. In other words, $\theta_e^b$ is an included angle between ObZb' and the Zb axis. When a component that is of a projection of a positive axis of the Zb axis on the plane YbOeZe and that is on the Xb axis is located on a positive axis of the Xb axis, $\theta_e^b$ is positive. When a component that is of a projection of a positive axis of the Zb axis on the plane YbOeZe and that is on the Xb axis is located on a negative axis of the Xb axis, $\theta_e^b$ is negative.

Alternatively, when a component that is of a projection of ObZb' on a plane XbObYb and that is on the Xb axis is located on a positive axis of the Xb axis, $\theta_e^b$ is positive. When a component that is of a projection of ObZb' on a plane XbObYb and that is on the Xb axis is located on a negative axis of the Xb axis, $\theta_e^b$ is negative.

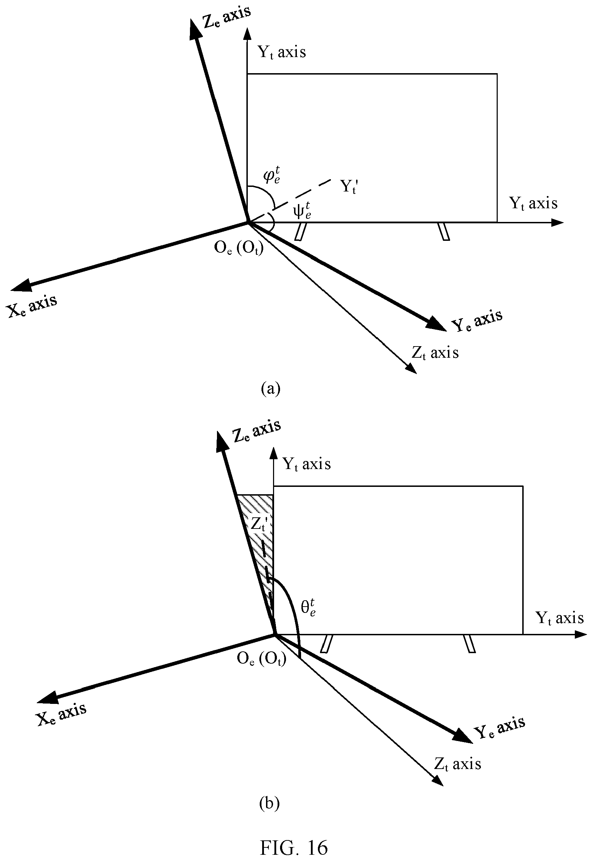

For example, FIG. 16 shows a pitch $\varphi_e^t$, a yaw $\psi_e^t$, and a roll $\theta_e^t$ of the third coordinate system relative to the first coordinate system.

As shown in FIG. 16, the coordinate origin Ot of the third coordinate system coincides with the coordinate origin Oe of the first coordinate system after parallel movement. The three axes of the third coordinate system are the Xt axis, the Yt axis, and the Zt axis. The three axes of the first coordinate system are the Xe axis, the Ye axis, and the Ze axis. As shown in (a) in FIG. 16, OeYt' (namely, OtYt') is a projection of the Yt axis on the plane XeOeYe of the first coordinate system. As shown in (b) in FIG. 16, OeZt' (namely, OtZt') is a projection of the Zt axis on a plane YtOeZe.

The pitch $\varphi_e^t$ of the third coordinate system relative to the first coordinate system is an included angle between the Yt axis of the third coordinate system and the plane XeOeYe of the first coordinate system. In other words, $\varphi_e^t$ is an included angle between OeYt' (namely, OtYt') and the Yt axis. When a component of OeYt on the Ze axis is located on the positive axis of the Ze axis, $\varphi_e^t$ is positive. When a component of OeYt on the Ze axis is located on the negative axis of the Ze axis, $\varphi_e^t$ is negative.

The yaw $\psi_e^t$ of the third coordinate system relative to the first coordinate system is an included angle between the projection of the Yt axis of the third coordinate system on the plane XeOeYe of the first coordinate system and the Ye axis of the first coordinate system. In other words, $\psi_e^t$ is an included angle between OeYt' (namely, OtYt') and the Ye axis. When a component of OeYt' on the Xe axis is located on the positive axis of the Xe axis, $\psi_e^t$ is positive. When a component of OeYt' on the Xe axis is located on the negative axis of the Xe axis, $\psi_e^t$ is negative.

The roll $\theta_e^t$ of the third coordinate system relative to the first coordinate system is an included angle between the Zt axis of the third coordinate system and the plane YtOeZe. In other words, $\theta_e^t$ is an included angle between OtZt' and the Zt axis. When a component that is of a projection of a positive axis of the Zt axis on the plane YtOeZe and that is on the Xt axis is located on a positive axis of the Xt axis, $\theta_e^t$ is positive. When a component that is of a projection of a positive axis of the Zt axis on the plane YtOeZe and that is on the Xt axis is located on a negative axis of the Xt axis, $\theta_e^t$ is negative.

Alternatively, when a component that is of a projection of OtZt' on a plane XtOtYt and that is on the Xt axis is located on a positive axis of the Xt axis, $\theta_e^t$ is positive. When a component that is of a projection of OtZt' on a plane XtOtYt and that is on the Xt axis is located on a negative axis of the Xt axis, $\theta_e^t$ is negative.

A direction change of the third coordinate system relative to the first coordinate system may be expressed by using an attitude matrix $C_e^t$ .

$$C_e^t = \begin{bmatrix} cos\theta_e^t cos\psi_e^t + sin\varphi_e^t sin\theta_e^t sin\psi_e^t & -cos\theta_e^t sin\psi_e^t + sin\varphi_e^t sin\theta_e^t cos\psi_e^t & -cos\varphi_e^t sin\theta_e^t \\ cos\varphi_e^t sin\psi_e^t & cos\varphi_e^t cos\psi_e^t & sin\varphi_e^t \\ sin\theta_e^t cos\psi_e^t - sin\varphi_e^t cos\theta_e^t sin\psi_e^t & -sin\theta_e^t sin\psi_e^t - sin\varphi_e^t cos\theta_e^t cos\psi_e^t & cos\varphi_e^t cos\theta_e^t \end{bmatrix}$$

formula (8)

The foregoing formula (8) for the attitude matrix $C_e^t$ is the conventional technology, and may be obtained by a person skilled in the art from the conventional technology, for example, the attitude matrix in Chapter 1.2.1 in the book Inertial Navigation (Beijing: Science Press, ISBN 7-03-016428-8, Qin Yongyuan, first edition in May 2006, first printing in May 2006).

The foregoing only shows examples of conversion of the second coordinate system and the third coordinate system relative to the first coordinate system. A person skilled in the art should understand that conversion of another coordinate system is also performed according to the same formula based on the foregoing principle, and only a corresponding parameter is modified.

Optionally, the second electronic device may include the IMU module. Optionally, the IMU module of the second electronic device is first calibrated. To be specific, a coordinate system on which a pitch, a yaw, and a roll that are output by the IMU module of the second electronic device are based is calibrated to the first coordinate system, or $C_b^e$ output by the IMU module of the second electronic device is calibrated to $\begin{bmatrix} 1 & 0 & 0 \\ 0 & 1 & 0 \\ 0 & 0 & 1 \end{bmatrix}$ . In this way, as the second electronic device is subsequently moved, the pitch, the yaw, and the roll that are output by the IMU module of the second electronic device are the pitch, the yaw, and the roll of the second coordinate system relative to the first coordinate system.

Alternatively, $C_b^e$ output by the IMU module of the second electronic device is transposed, to reflect a direction change of the second coordinate system relative to the first coordinate system.

For example, the second coordinate system of the second electronic device may be enabled to be parallel to the first coordinate system (for example, the Xb axis is parallel to the Xe axis, the Yb axis is parallel to the Ye axis, and the Zb axis is parallel to the Ze axis), and positive directions of corresponding coordinate axes of the two coordinate systems are enabled to be the same (for example, the positive direction of the Xb axis is the same as the positive direction of the Xe axis, the positive direction of the Yb axis is the same as the positive direction of the Ye axis, and the positive direction of the Zb axis is the same as the positive direction of the Ze axis). In this case, a pitch, a yaw, and a roll that are output by the IMU module of the second electronic device are set to 0.

For example, the second coordinate system of the second electronic device may be enabled to be parallel to the first coordinate system, and positive directions of all axes of the two coordinate systems are enabled to be the same.

$$C_b^e = \begin{bmatrix} 1 & 0 & 0 \\ 0 & 1 & 0 \\ 0 & 0 & 1 \end{bmatrix}$$

In this case, the IMU module of the second electronic device outputs through adjustment.

**(6) Establishment of a fourth coordinate system (millimeter-wave radar coordinate system)**

The millimeter-wave radar module 160 of the first electronic device 100 is configured to implement a function of the millimeter-wave radar. A plurality of antennas in the millimeter-wave radar have a distance difference in a transverse direction (for example, a horizontal direction) and/or a longitudinal direction (for example, a vertical direction). A coordinate system (the fourth coordinate system) of the millimeter-wave radar may be established by using the distance difference between the antennas.

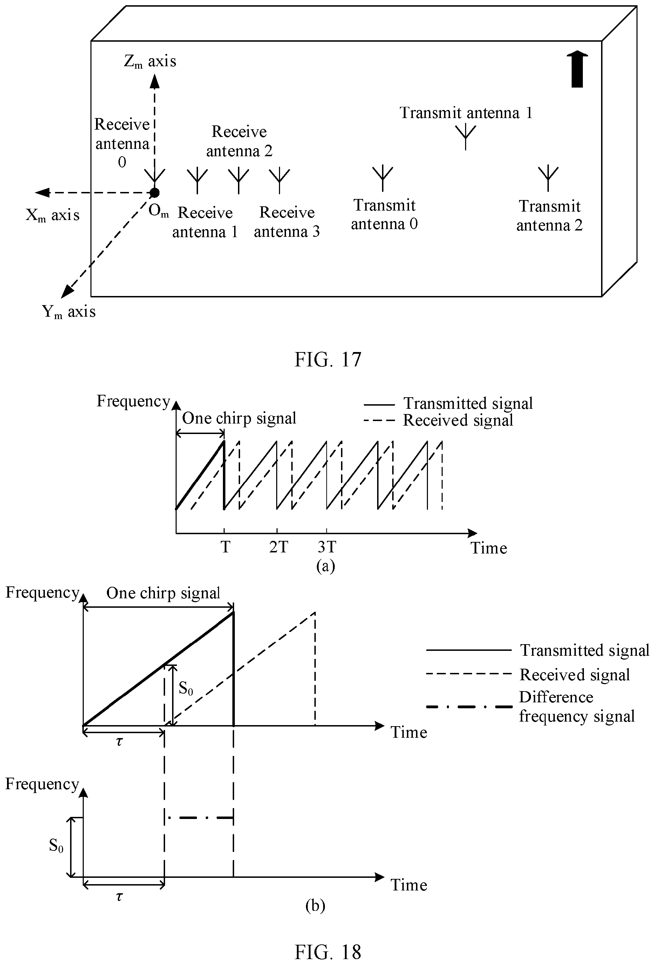

In an example, the millimeter-wave radar module 160 includes three transmit antennas and four receive antennas. For example, as shown in FIG. 17, the three transmit antennas and the four receive antennas are located on a same longitudinal plane (for example, a vertical plane). The three transmit antennas are distributed in a shape of a triangle on the longitudinal plane. A transmit antenna 0 and a transmit antenna 2 are located on a same transverse plane. The four receive antennas are located on a same transverse line (for example, a horizontal line). In an implementation, a point (for example, an end point on one side) on the receive antenna 0 is used as an origin Om of the fourth coordinate system. A connection line between the receive antenna 0 and a receive antenna 1 is used as an Xm axis of the fourth coordinate system. In addition, a direction in which the receive antenna 1 points to the receive antenna 0 is a positive direction of the Xm axis. A straight line that passes through the origin Om and is perpendicular to the Xm axis is a Zm axis of the fourth coordinate system. In addition, a direction pointing to a zenith is a positive direction of the Zm axis. Then, a Ym axis of the fourth coordinate system and a positive direction of the Ym axis are determined with reference to a rule of a right-hand rectangular coordinate system. Optionally, prompt information may be marked on the outer surface of the first electronic device 100, and is for prompting a correct installation manner or a correct placement manner. In this way, the three transmit antennas and the four receive antennas of the millimeter-wave radar module 160 in the first electronic device 100 are located on the same longitudinal plane.

Names of the three axes in the fourth coordinate system and positive directions of the three axes may alternatively be defined in another manner. Details are not described herein again. The Xm axis, the Ym axis, and the Zm axis in the fourth coordinate system shown in FIG. 17 are used as an example for description in embodiments of this application.

It should be noted that the point on the receive antenna 0 is used as the origin of the fourth coordinate system, which is merely an example. A point on another antenna (for example, the receive antenna 1) may alternatively be the origin of the fourth coordinate system.

Optionally, the fourth coordinate system may be pre-established. An installation worker only needs to install the first electronic device 100 as required. For example, before delivery of the first electronic device 100, the fourth coordinate system has been established, and related information of the fourth coordinate system is stored locally or on a server. When the first electronic device 100 is started, or when the first electronic device 100 receives a specific trigger, the first electronic device 100 invokes the related information of the fourth coordinate system locally or from the server. Unless otherwise specified, the server in this application may be a hub device 200 in a home, or may be a cloud server.

Preferably, the outer surface of the first electronic device 100 may have only one piece of marked prompt information. The marked prompt information indicates installation of the first electronic device. In this way, the transmit antenna and the receive antenna of the millimeter-wave radar module, and the antenna of the UWB module all meet a preset requirement.

**(7) Calculation of coordinates in the fourth coordinate system**

1: Determine a distance between a reflection point and the millimeter-wave radar module, and a radial speed of the reflection point.

(1) Determine the distance between the reflection point and the millimeter-wave radar module.

The transmit antenna of the millimeter-wave radar module 160 transmits a signal. The signal is reflected by the reflection point, and then is received by the receive antenna of the millimeter-wave radar module 160. A frequency at which an LFMCW millimeter-wave radar transmits a signal increases linearly as time varies. This type of signal is referred to as a chirp (Chirp) signal. With reference to FIG. 5B, the millimeter-wave radar module 160 receives the chirp signal through the receive antenna. The received signal and a local oscillator signal are mixed by the mixer, and a difference frequency signal is output. The difference frequency signal is filtered and amplified by the filter and the sampling module, and then undergoes analog-to-digital conversion to become a digital difference frequency signal.

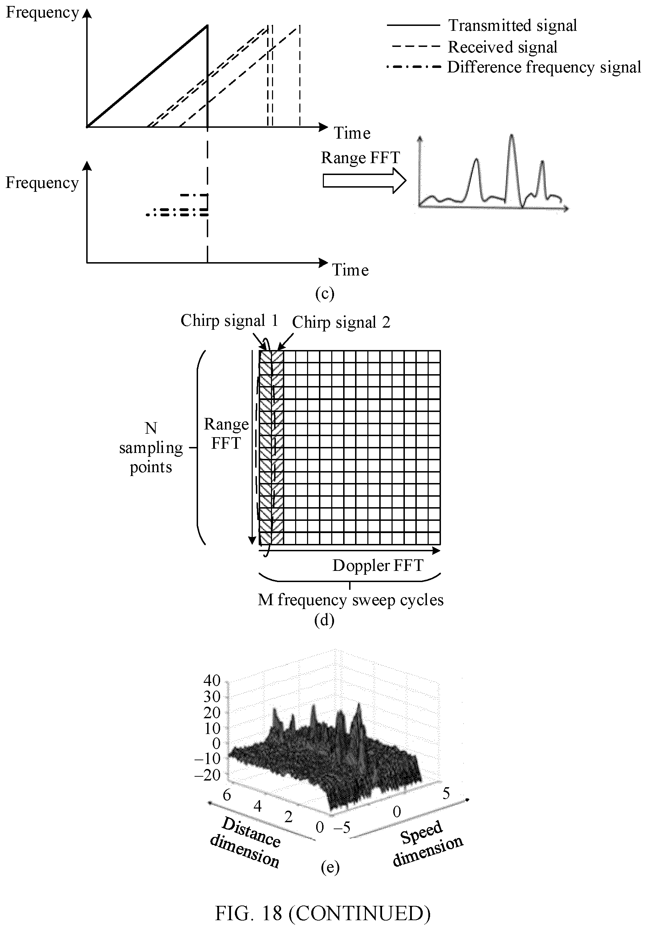

For example, FIG. 18 is a schematic diagram of a principle of determining the distance and the radial speed of the reflection point by the millimeter-wave radar according to an embodiment of this application. As shown in (a) in FIG. 18, a solid line is the transmitted signal of the millimeter-wave radar module 160, and a dotted line is the received signal of the millimeter-wave radar module 160. For a chirp signal, one frequency sweep cycle Tc is usually at a microsecond ($\mu$s) level, and a frequency modulation rate S0 (namely, a frequency change rate) reaches an order of magnitude of 1012 (unit: Hz/s). In this embodiment of this application, a chirp signal in one frequency sweep cycle Tc is referred to as one chirp signal. Usually, it is considered that a spatial position of the target within one frequency sweep cycle Tc remains unchanged.

As shown in (b) in FIG. 18, in one frequency sweep cycle Tc, the transmit antenna transmits the chirp signal. After duration $\tau$, the receive antenna receives a signal reflected back from the reflection point. A frequency difference between the received signal and the transmitted signal is $\tau * S0$. The frequency difference between the received signal and the transmitted signal is a frequency f0 of the difference frequency signal. In other words, $f0=\tau *S0$. Herein, $\tau=2d/c$, d is the distance between the reflection point and the millimeter-wave radar module (which may also be considered as the first electronic device), and c is a rate at which the chirp signal is transmitted in the air, and is generally a light speed through selection. Therefore, a relationship between the distance d of the reflection point and the frequency f0 of the difference frequency signal is shown in formula (9).

$$d=f_0\times c/(2\times S_0) \hspace{4cm} \text{formula (9)}$$

A time-domain signal may be converted into a frequency-domain signal through a Fourier transform. A sine wave in time domain correspondingly generates a peak value in frequency domain. The peak value corresponds to the frequency f0 of the difference frequency signal. For example, as shown in (c) in FIG. 18, the transmitted signal of the millimeter-wave radar module is reflected back by three reflection points, to obtain three signals. The millimeter-wave radar module receives the three received signals, and separately obtains three corresponding difference frequency signals. A fast Fourier transform (fast Fourier transform, FFT) is performed on the three difference frequency signals to obtain a range (range) curve (referred to as a range FFT (range FFT)), and a spectrum having three different separated peaks may be generated. Each peak value indicates that there is one reflection point in a corresponding place. A frequency of the difference frequency signal may be obtained by calculating a frequency corresponding to the peak value. A distance of the reflection point may be obtained by detecting the frequency of the difference frequency signal.

Similarly, a Doppler FFT (Doppler FFT) is performed on a plurality of difference frequency signals from a same reflection point within different frequency sweep cycles Tc, to obtain a phase difference between the plurality of difference frequency signals. A radial speed of the reflection point may be obtained by detecting the phase difference between the plurality of difference frequency signals. For a detailed principle, refer to the conventional technology. Details are not described herein again.

The millimeter-wave radar module receives the chirp signal, performs mixing, power amplification, and filtering on the transmitted signal and the received signal to obtain the difference frequency signal. The analog-to-digital conversion is performed on the difference frequency signal, to obtain the digital difference frequency signal. The distance and the radial speed of the reflection point may be obtained by detecting the digital difference frequency signal.

For example, as shown in (d) in FIG. 18, a frame of data of the millimeter-wave radar module is data within one radar scanning cycle. One radar scanning cycle includes M frequency sweep cycles Tc. There are N sampling points of a difference frequency signal in each frequency sweep cycle Tc.

A one-dimensional range FFT may be performed on a digital difference frequency signal within one frequency sweep cycle Tc, to obtain a frequency of the difference frequency signal. In this way, the distance of the reflection point can be calculated based on the frequency of the difference signal. A quantity of points in the range FFT is a quantity N of sampling points of the difference frequency signal corresponding to the chirp signal.

A one-dimensional Doppler FFT may be performed on digital difference frequency signals from a same reflection point within a plurality of adjacent frequency sweep cycles Tc, to obtain a phase difference between the plurality of difference frequency signals. In this way, the radial speed of the reflection point can be calculated based on the phase difference between the plurality of difference frequency signals. A quantity of points in the Doppler FFT is a quantity of frequency sweep cycles included in the frame of data.

A combined operation of the range FFT and the Doppler FFT may be considered as a two-dimensional FFT performed on the frame of data. In embodiments of this application, a frame of data obtained after two-dimensional FFT processing is referred to as a frame of two-dimensional FFT data. For example, (e) in FIG. 18 is a schematic diagram of the frame of two-dimensional FFT data obtained by the millimeter-wave radar module. As shown in (e) in FIG. 18, there are a plurality of peak values in the frame of two-dimensional FFT data. Each peak value indicates that there is one reflection point in a corresponding place. A value of the reflection point in a distance dimension or a speed dimension is a distance of the reflection point or a radial speed of the reflection point.

(2) Determine a signal coming direction of the reflected signal from the reflection point.

The signal coming direction of the reflected signal includes a transverse coming direction (for example, a horizontal coming direction) and a longitudinal coming direction (for example, a vertical coming direction). A yaw may be used to represent a transverse coming direction of a signal, and a pitch may be used to represent a longitudinal coming direction of the signal. In an implementation, the yaw and the pitch may be calculated by using a phase difference between received signals of the plurality of receive antennas of the millimeter-wave radar module.

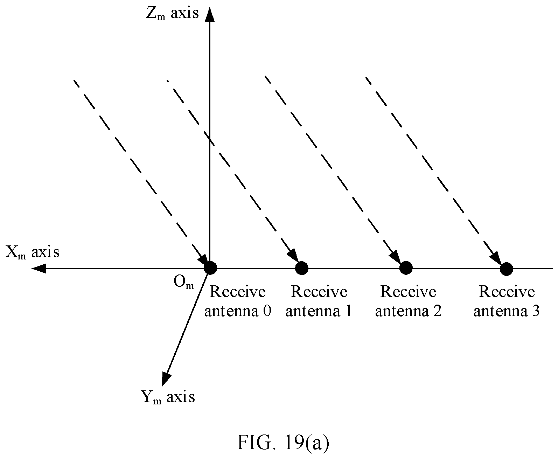

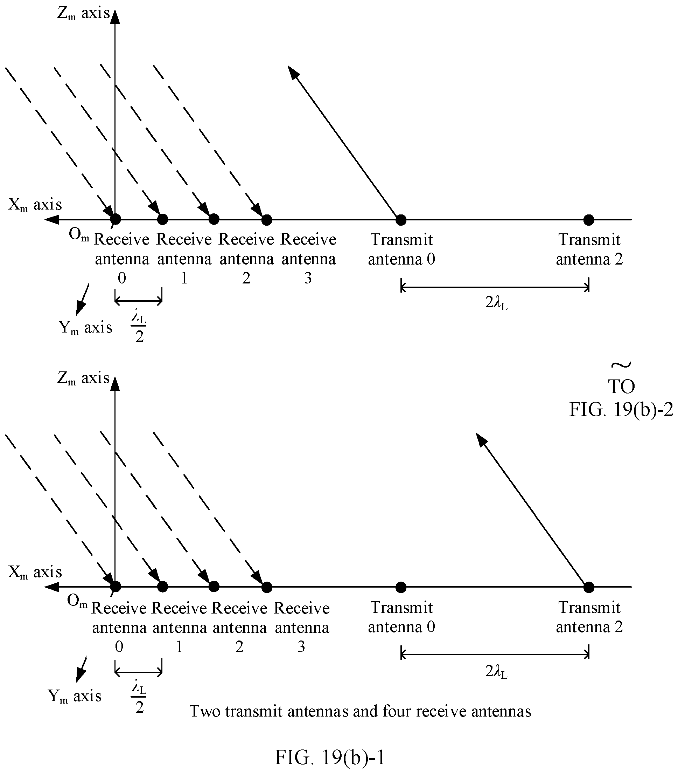

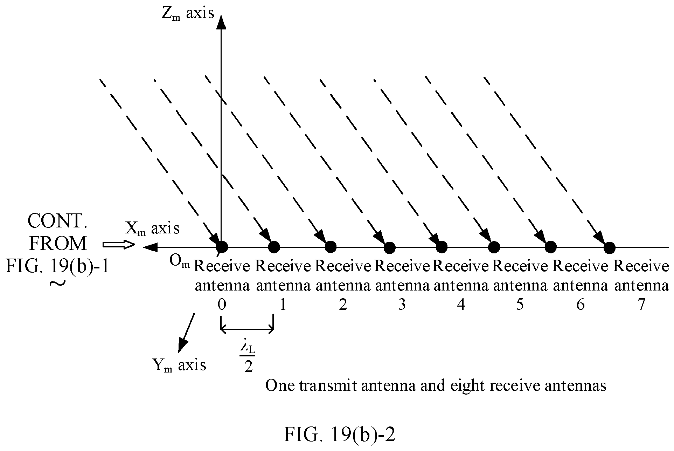

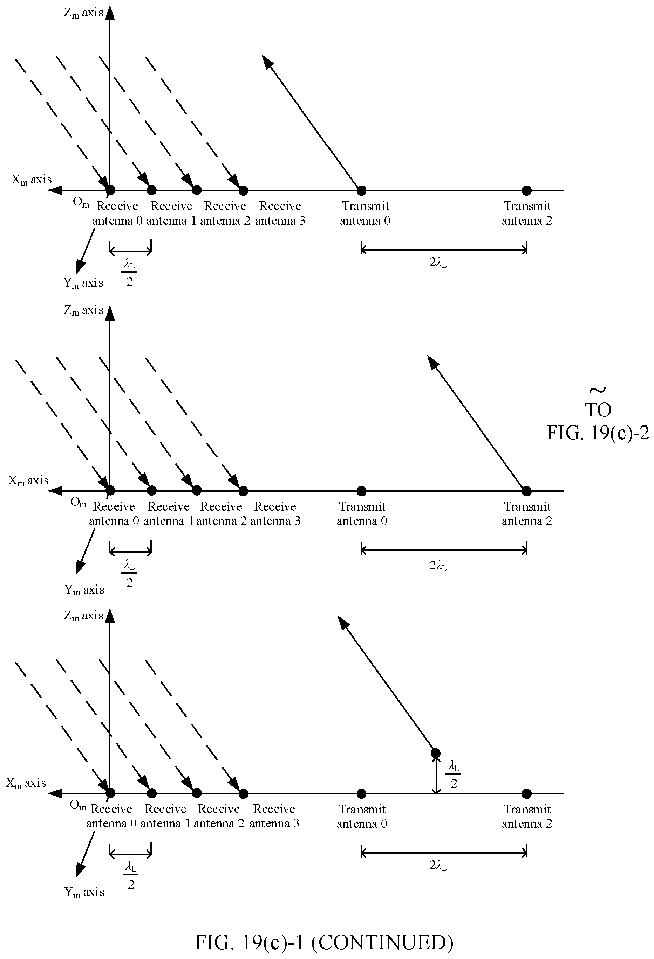

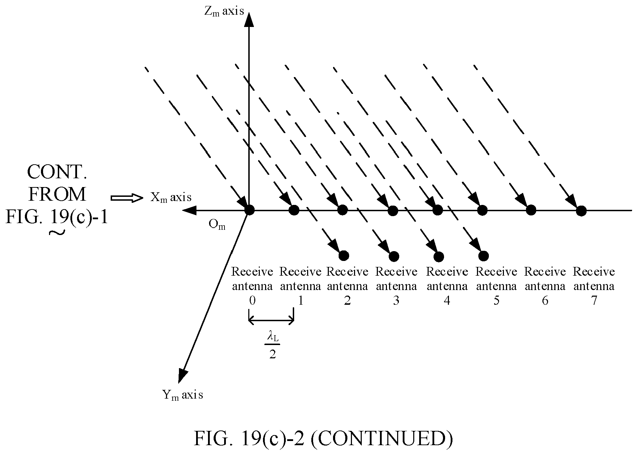

For example, FIG. 19(a) to FIG. 19(c)-2 are a schematic diagram of a principle of determining the signal coming direction of the reflected signal from the reflection point by the millimeter-wave radar according to an embodiment of this application. As shown in FIG. 19(a), the millimeter-wave radar module includes four receive antennas. A phase difference between reflected signals that reach any two different receive antennas after signals transmitted by a same transmit antenna are reflected by the reflection point may be used by the millimeter-wave radar module to measure a yaw of the reflected signal. For a specific manner in which the millimeter-wave radar module determines a transverse coming direction of the reflected signal based on a phase difference obtained when signals reach two adjacent receive antennas, refer to the method for calculating the angle $\alpha$ in FIG. 12(e). Details are not described herein again.

In an implementation, precision of measuring the signal coming direction may be improved by increasing a quantity of antennas. In an example, the antennas of the millimeter-wave radar module are of the distribution structure shown in (a) in FIG. 8. When the millimeter-wave radar module transmits signals, the transmit antenna may be switched by changing the relay switch, to separate signals of different transmit antennas by the receive antennas. For example, as shown in FIG. 19(b)-1 and FIG. 19(b)-2, when a transmit antenna 0 and a transmit antenna 2 alternately transmit signals, an effect that two transmit antennas and four receive antennas are equivalent to one transmit antenna and eight receive antennas may be implemented based on a principle that a phase difference is generated by a position difference between the antennas. For example, in FIG. 19(b)-1 and FIG. 19(b)-2, a distance between the receive antennas is $\lambda L/2$, and a distance between the transmit antenna 0 and the transmit antenna 2 is $2\lambda L$, where $\lambda L$ is a wavelength of a millimeter wave. A signal that is of the transmitted signal of the transmit antenna 2 and that reaches a receive antenna 0 may be equivalent to a received signal of a receive antenna 4. A signal that is of the transmitted signal of the transmit antenna 2 and that reaches a receive antenna 1 may be equivalent to a received signal of a receive antenna 5. A signal that is of the transmitted signal of the transmit antenna 2 and that reaches a receive antenna 2 may be equivalent to a received signal of a receive antenna 6. A signal that is of the transmitted signal of the transmit antenna 2 and that reaches a receive antenna 3 may be equivalent to a received signal of a receive antenna 7. For example, the receive antenna 4, the receive antenna 5, the receive antenna 6, and the receive antenna 7 in the schematic diagram of the transmit antenna and the eight receive antennas in FIG. 19(b)-1 and FIG. 19(b)-2 are equivalently obtained virtual receive antennas.

A phase difference obtained when signals transmitted by transmit antennas between which there is a distance in a longitudinal dimension reach the receive antenna after being reflected by the reflection point may be used by the millimeter-wave radar module to measure a longitudinal coming direction (for example, represented by a pitch) of the reflected signal. In an example, the antennas of the millimeter-wave radar module are of the structure shown in (a) in FIG. 8. There is a distance between a transmit antenna 1 and the transmit antenna 0 in the longitudinal dimension. There is a distance between the transmit antenna 1 and the transmit antenna 2 in the longitudinal dimension. A pitch of a reflected signal may be determined by comparing signals separately received by a same receive antenna from the transmit antenna 1, the transmit antenna 0 and the transmit antenna 2. For example, as shown in FIG. 19(c)-1 and FIG. 19(c)-2, signals between which there is a phase difference in the longitudinal dimension may be compared with reference to a signal that is transmitted by the transmit antenna 0 and received by the receive antenna 2 and the receive antenna 3, a signal that is transmitted by the transmit antenna 2 and received by the receive antenna 0 and the receive antenna 1, and a signal that is transmitted by the transmit antenna 1 and received by the receive antenna 0, the receive antenna 1, the receive antenna 2, and the receive antenna 3, and a longitudinal coming direction (for example, represented by a pitch) of a reflected signal may be obtained through calculation. For example, a signal that is transmitted by the transmit antenna 0 and received by the receive antenna 2 may be compared with a signal that is transmitted by the transmit antenna 1 and received by the receive antenna 0, to obtain a phase difference between the two signals, and a pitch is calculated based on the phase difference. For a specific step of calculating the pitch based on the phase difference between the received signals, refer to the method for calculating the angle $\beta$ in FIG. 12(f). Details are not described herein again.

(3) Determine coordinates of the reflection point in the fourth coordinate system.

The first electronic device may calculate, according to formula (7) based on the distance between the reflection point and the millimeter-wave radar and the signal coming direction (the yaw and the pitch) of the reflected signal, the coordinates of the reflection point in the fourth coordinate system established by the first electronic device.

(4) Determine coordinates of the user in the fourth coordinate system.

In some cases, because different parts of a human body in a large size may be provided with different clothes through wearing and have different bone structures, there may be different detection results. As a result, for detection of the human body, there may be a plurality of reflection points that may not be even distributed quite uniformly. For an object in a large size, different parts may be made from different materials, in different shapes, and the like. As a result, for detection of such an object, there may also be a plurality of reflection points that may not be even distributed quite uniformly. For a human body, because parts such as the head, a hand and a foot of the human body all reflect the transmitted signal of the millimeter-wave radar, the millimeter-wave radar detects the human body as a plurality of reflection points within the detectable range. In this case, clustering processing may be performed on point cloud data of the reflection points. To be specific, the plurality of detected reflection points are aggregated into one category, and the cluster is determined as an object or a human body.

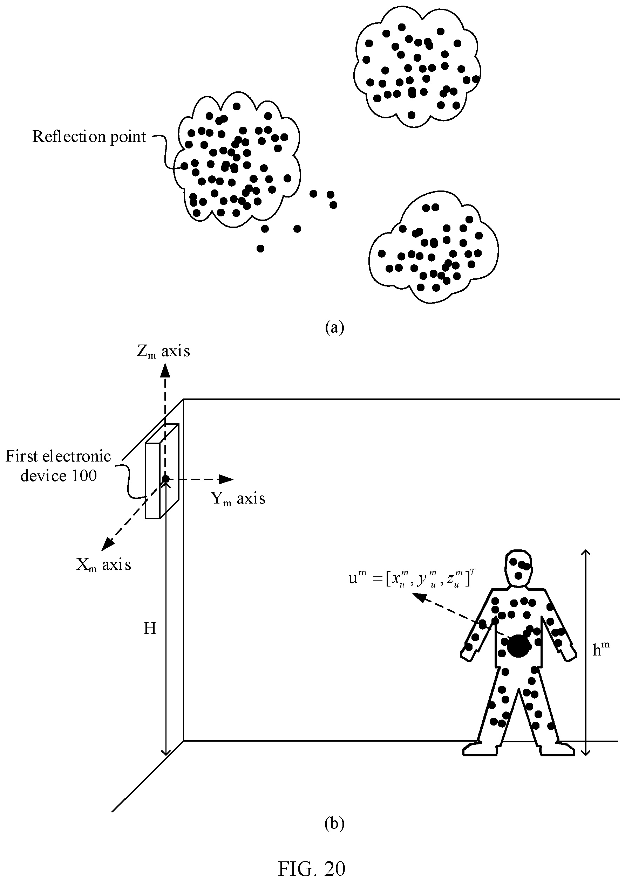

For example, (a) in FIG. 20 is a schematic diagram of an effect of performing clustering processing on the point cloud data. In (a) in FIG. 20, each point represents one reflection point detected by the millimeter-wave radar module, three closed curves respectively represent categories obtained through aggregation, and a point outside the three closed curves represents a reflection point that is not aggregated into any category. In an example, the millimeter-wave radar module clusters the plurality of reflection points into one object or human body (user) according to a clustering algorithm, and may obtain, through calculation, coordinates of the object or the human body (the user) in the fourth coordinate system based on coordinates of a plurality of reflection points obtained after clustering. For example, the coordinates of the object or the human body (the user) in the fourth coordinate system may be coordinates of a gravity center of the object or the human body in the fourth coordinate system. For example, as shown in (b) in FIG. 20, a smaller point in (b) in FIG. 20 represents the reflection point detected by the millimeter-wave radar, and a largest point is a coordinate point of the human body (the user) in the fourth coordinate system. The coordinates of the human body (the user) in the fourth coordinate system are denoted as $u^m = [x_u^m, y_u^m, z_u^m]\mathrm{T}$.

Further, a height of the object or a height of the human body (the user) may be further obtained through calculation based on a height H of the first electronic device from the ground and the coordinates of the object or the human body (the user) in the fourth coordinate system. For example, a height hm of the human body (the user) may be calculated according to formula (10).

$$\mathrm{hm}=(\mathrm{H}+z_u^m)\times2 \qquad\qquad \text{formula (10)}$$

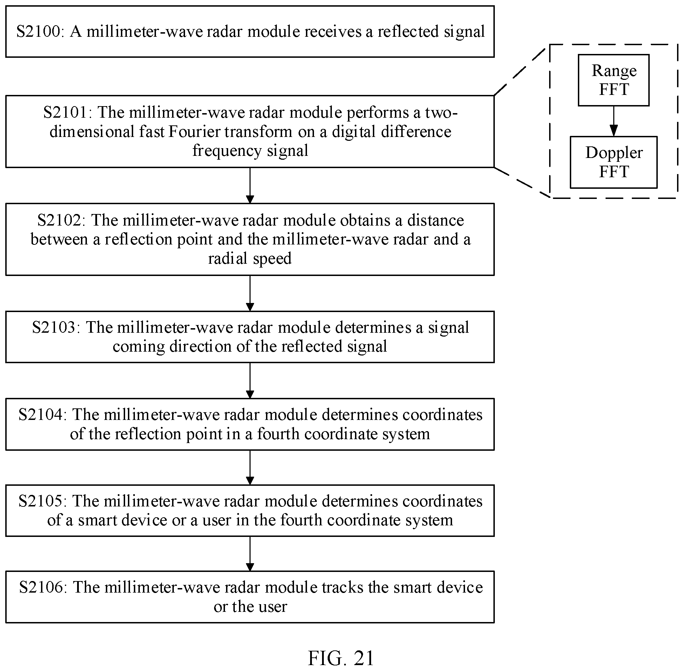

For example, FIG. 21 is a flowchart of a method for determining the coordinates of the user by the first electronic device in the fourth coordinate system according to an embodiment of this application. As shown in FIG. 21, the method may include the following steps.

S2100: The millimeter-wave radar module receives a reflected signal.

S2101: The millimeter-wave radar module performs a two-dimensional fast Fourier transform on a digital difference frequency signal.

The receive antenna of the millimeter-wave radar module receives the reflected signal. The digital difference frequency signal is obtained based on the reflected signal. The two-dimensional fast Fourier transform is performed on the digital difference frequency signal, to obtain two-dimensional FFT data.

S2102: The millimeter-wave radar module obtains a distance between a reflection point and the millimeter-wave radar and a radial speed according to a target detection algorithm.

The millimeter-wave radar may perform target detection on a frame of two-dimensional FFT data according to a target detection algorithm, to obtain a distance and a radial speed of a target.

It should be noted that, in an indoor environment, a multipath effect and clutter interference are generated, and a signal received by the millimeter-wave radar includes a target reflected signal, background noise, clutter interference, and the like. For example, in a test environment for the frame of two-dimensional FFT data shown in (e) in FIG. 18, there are moving human bodies respectively located in places that are 1 m, 2 m and 4 m away from the millimeter-wave radar. However, it can be learned from (e) in FIG. 18 that, in addition to three peak values in the places that are 1 m, 2 m and 4 m away from the millimeter-wave radar, there is a larger peak value caused by other reflected signals (the background noise, the clutter interference, and the like). If a reflected signal caused by the background noise, the clutter interference, and the like is detected as a reflection point, a false alarm is generated. In an implementation method, the distance and the radial speed of the reflection point may be obtained according to a constant false alarm rate (constant false alarm rate, CFAR) target detection algorithm, to maintain a constant false alarm rate, and improve target detection precision.

It should be noted that the millimeter-wave radar module may obtain, based on two-dimensional FFT data, the distance and the radial speed of the reflection point according to a target detection algorithm in the conventional technology as required. A used target detection algorithm is not limited in embodiments of this application. A specific implementation method of the target detection algorithm may be obtained from the conventional technology. Details are not described herein again.

S2103: The millimeter-wave radar module determines a signal coming direction of the reflected signal.

For example, a yaw and a pitch may be estimated according to an algorithm, for example, a phase difference method, a sum difference beam method, or a music method. The algorithm, for example, the phase difference method, the sum difference beam method, or the music method, may be obtained from the conventional technology. Details are not described herein again.

S2104: The millimeter-wave radar module determines coordinates of the reflection point in the fourth coordinate system.

The millimeter-wave radar determines the coordinates of the reflection point in the fourth coordinate system based on the distance between the reflection point and the millimeter-wave radar and a signal coming direction of the reflection point.

S2105: The millimeter-wave radar module determines coordinates of a smart device or the user in the fourth coordinate system.

In an implementation, detected reflection points are clustered according to the clustering algorithm, and a plurality of reflection points are clustered into the smart device or the user. The clustering algorithm includes a partition-based clustering method, a density-based partitioning method, a model-based partitioning method, a network-based partitioning method, and the like. For example, common clustering algorithms include density-based spatial clustering of applications with noise (density-based spatial clustering of applications with noise, DBSCAN), a K-means algorithm, a BIRCH algorithm, and the like. Clustering processing may be performed according to any clustering algorithm. This is not limited in embodiments of this application.

The coordinates of the smart device or the user in the fourth coordinate system may be obtained through calculation based on a coordinate average value of a plurality of reflection points of the smart device or the user obtained after clustering.

S2106: The millimeter-wave radar module tracks the smart device or the user.

The millimeter-wave radar module performs target detection on each received frame of data. Further, after an object (a smart device) or a human body in each frame of data is detected according to the target detection algorithm and the clustering algorithm, a detection result in a current frame may be further matched with a detection result in a previous frame one by one according to an association algorithm, to track the object or the human body (that is, obtain a change in a coordinate value of the object or the human body as time varies). For example, a tracking algorithm (a previous frame and subsequent frame association algorithm) is as follows: Calculate a Euclidean distance (a straight line distance between two spatial points) between two targets in two frames, determine two targets with a shortest Euclidean distance as a same target, and then link and track the target according to a Hungarian algorithm.

Further, in an implementation, the millimeter-wave radar module may determine, based on a target tracking result, that the target is stationary or moving. The millimeter-wave radar module may be further configured to detect a physiological feature (for example, a breath frequency or a heartbeat frequency) of a target in a stationary state. If it is determined that the physiological feature of the target satisfies a set condition (for example, the breath frequency falls within a preset range, or the heartbeat frequency falls within a preset range), it is determined that the target or a target obtained after clustering is the human body (the user). The user is tracked.

**(8) Detection of the physiological feature, an identity category, a human body attitude, and the like of the user by the millimeter-wave radar module**

With reference to accompanying drawings, the following describes in detail a specific method for detecting information such as the physiological feature, the identity category, and the human body attitude by the millimeter-wave radar module.

    (1) The millimeter-wave radar module detects the physiological feature of the user.

The physiological feature of the user includes the breath frequency, the heartbeat frequency, and the like of the user. When the user is still (the position of the user does not change), slight displacement of the body of the user caused by a breath and a heartbeat may cause a phase change of the reflected signal of the millimeter-wave radar module. The breath frequency and the heartbeat frequency of the user may be obtained by detecting the phase change of the reflected signal of the millimeter-wave radar module when the user is still.

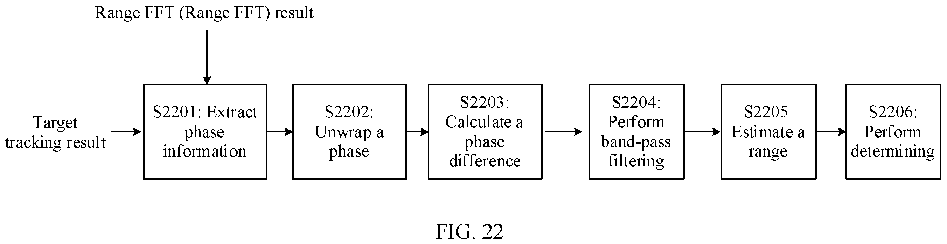

In an implementation, as shown in FIG. 22, a method for obtaining the breath frequency and the heartbeat frequency of the user by the millimeter-wave radar module may include the following steps.

S2201: Extract phase information.

In S2201, a range FFT is performed on each frame of data of the millimeter-wave radar, and a frequency of the difference frequency signal may be obtained based on a range FFT result. To be specific, a phase of the difference frequency signal may be obtained. In S2206, the millimeter-wave radar performs target tracking on the user, and may obtain a change of the position of the user as time changes, that is, may obtain a position of the user at a moment.

If the millimeter-wave radar determines that the user is still (for example a coordinate change amount of the user is less than a specified value within a period of time) based on the target tracking result, the millimeter-wave radar performs phase extraction on a range FFT result obtained at the current position of the user, that is, extracts the phase information of the difference frequency signal. For example, the radar scanning cycle is 100 ms, that is, a cycle of a frame of data is 100 ms. The phase information of the difference frequency signal is extracted once from each frame of data. Phase information of a plurality of frames of data is continuously extracted, to obtain a relationship that the phase changes with a quantity of the frames, that is, a relationship that the phase changes with time. The phase is denoted as a vibration signal v(j), where j is the quantity of the frames.

S2202: Unwrap the phase.

The phase is unwrapped, to obtain an actual displacement curve. It is specified that a phase value falls within $[-\pi, \pi]$. If the phase value obtained through calculation in S2201 is greater than $\pi$, phase unwrapping is performed by subtracting $2\pi$ from the phase value. If the phase value obtained through calculation in S2201 is less than $-\pi$, phase unwrapping is performed by adding $2\pi$ to the phase value.

S2203: Calculate a phase difference.

A subtraction is performed on consecutive phase values, and a phase difference operation is performed on the unwrapped phase, to obtain a phase difference $\Delta v$. This can enhance a heartbeat signal and eliminates any phase drift. Herein, $\Delta v(k)=v(k)-v(k-1)$.

S2204: Perform band-pass filtering.

The phase value is filtered by a band-pass filter for distinguishing separately based on the heartbeat frequency and the breath frequency. For example, a passband range of the band-pass filter is set to 0.8 Hz to 4 Hz, and the phase value is filtered, to detect heartbeat. The passband range of the band-pass filter is set to 0.1 Hz to 0.6 Hz, and the phase value is filtered, to detect a breath.

S2205: Estimate a range.

An FFT is performed on a phase difference signal, and a breath frequency and a heartbeat frequency within N frames of time are obtained based on a magnitude of a peak value and a harmonic feature.

S2206: Perform determining.

A breath frequency and a heartbeat frequency within a period of time are recorded. Obtained breath frequency and heartbeat frequency values are screened based on a preset confidence indicator (for example, an accuracy rate of 95% or a false alarm rate of 5%), and a relationship that the breath frequency and the heartbeat frequency changes with time is output.

(2) The millimeter-wave radar module detects the identity category of the user.

The millimeter-wave radar module may determine the identity category of the user based on the height hm (of the user) obtained through calculation. The identity category of the user includes adult, child, and the like.

In an implementation, when the user is within a detection range of the millimeter-wave radar module, if it is detected that the user is a moving state, the millimeter-wave radar module calculates a height of a user detected in each frame of data. The height is denoted as hm(t), indicating a height value at a moment t. An average value Hm of the height of the user may be further calculated based on hm(t), and the identity category of the user may be determined based on Hm. For example, a correspondence between the height of the user and the identity category of the user is shown in Table 1.

**Table 1**

| Identity category | Height |
|---|---|
| Adult | $H^m$>120 centimeters |
| Child | 120 centimeters$\geq H^m$>50 centimeters |
| Pet | $H^m\leq$50 centimeters |

(3) The millimeter-wave radar module detects the human body attitude of the user.

The millimeter-wave radar module may determine the human body attitude of the user based on a change of the height hm (of the user) obtained through calculation. The human body attitude includes standing, sitting, lying, and the like.

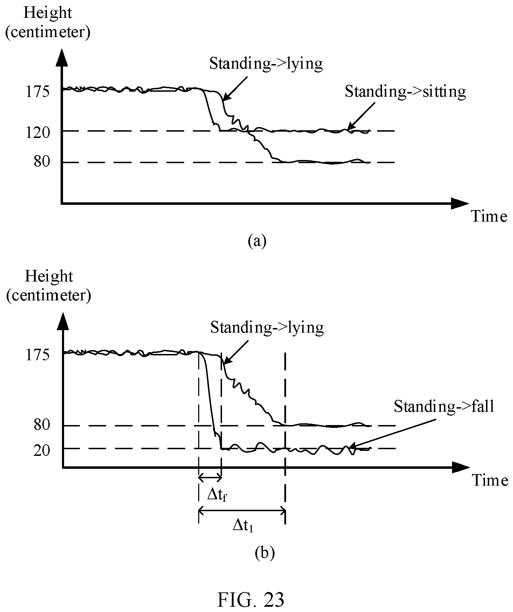

In an implementation, the millimeter-wave radar module performs target tracking on the user, and if it is determined that the height of the user changes, a value of a height change is greater than a preset height difference threshold, and maintenance duration after the height changes is greater than preset duration, determines that the human body attitude of the user changes. For example, as shown in (a) in FIG. 23, if the millimeter-wave radar module detects that the height of the user changes from 175 centimeters to 80 centimeters and remains at 80 centimeters for a period of time, the millimeter-wave radar module determines that the user changes from standing to lying. If the millimeter-wave radar module detects that the height of the user changes from 175 centimeters to 120 centimeters and remains at 120 centimeters for a period of time, the millimeter-wave radar module determines that the user changes from standing to sitting.

In an example, the millimeter-wave radar module determines the human body attitude of the user based on a height difference $\Delta h$ between a current height of the user and a height obtained when the user stands. For example, Δh(t) may be obtained through calculation according to formula (11), and Δh(t) indicates a height difference between a height of the user at the moment t and the height obtained when the user stands.

$$\Delta h(t) = H^m - h^m(t) \qquad\qquad \text{formula (11)}$$

For example, if the millimeter-wave radar module determines that Δh at a plurality of consecutive moments (that are greater than the preset duration) satisfies the preset height difference threshold, the millimeter-wave radar module determines that the human body attitude of the user changes. For example, a correspondence between the height difference Δh and the human body attitude is shown in Table 2.

**Table 2**

| Identity category | Human body attitude | Height difference Δh |
|---|---|---|
| Adult | Sitting | 30 centimeters≤Δh<60 centimeters |
| | Lying | 60 centimeters≤Δh<120 centimeters |
| Child | Sitting | 20 centimeters≤Δh<40 centimeters |
| | Lying | 40 entimeters≤Δh<80 centimeters |

Further, the millimeter-wave radar may further identify a fall behavior of the user by monitoring the height change of the user. For example, (b) in FIG. 23 shows a height change between a fall and normal lying of the user. As shown in (b) in FIG. 23, compared with that obtained during normal lying, the height of the user changes faster during the fall (that is, a height difference generated within same duration is large), and the height obtained after the fall is lower.

In an implementation, if the millimeter-wave radar module determines that the height difference Δh between the current height of the user and the height obtained when the user stands satisfies a preset fall height threshold, and duration Δt taken by the user to change from the height obtained when the user stands to the current height satisfies a preset fall duration threshold, the millimeter-wave radar module determines a user fall. For example, a correspondence between Δh, Δt, and the user fall is shown in Table 3.

**Table 3**

| Identity category | Status | Height difference Δh | Δt |
|---|---|---|---|
| Adult | Fall | 120 centimeters≤Δh | Δt≤0.3 second |
| Child | Fall | 80 centimeters≤Δh | Δt≤0.3 second |

**(9) Conversion between the first coordinate system and the fourth coordinate system**

After the first electronic device 100 establishes the first coordinate system and the fourth coordinate system, a coordinate value in the first coordinate system and a coordinate value in the fourth coordinate system need to be converted, to facilitate collaboration. For example, the coordinates of the second electronic device 300 in the first coordinate system are converted into coordinates of the second electronic device 300 in the fourth coordinate system, or coordinates of the user in the fourth coordinate system are converted into coordinates of the user in the first coordinate system. Consequently, conversion between the first coordinate system and the fourth coordinate system is mentioned.

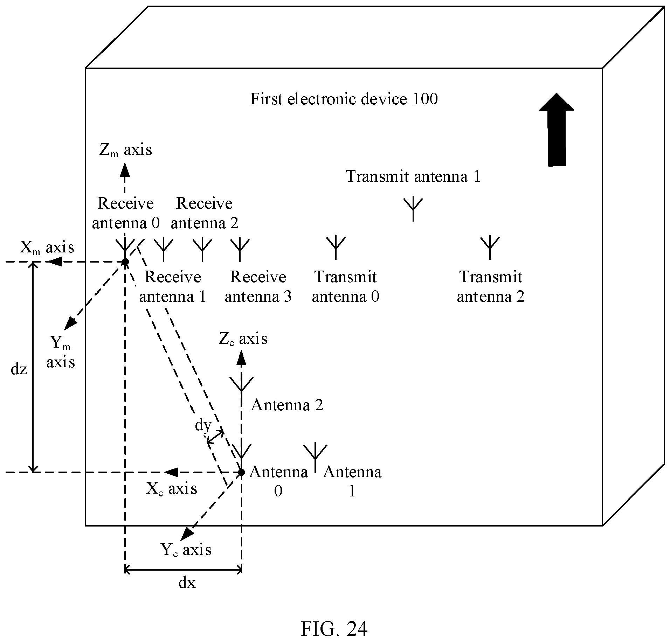

For example, the antenna distributions of both the UWB module 150 and the millimeter-wave radar module 160 of the first electronic device 100 may be set as shown in FIG. 24. The antenna 0, the antenna 1, and the antenna 2 are distributed in an L shape on a longitudinal plane (for example, a vertical plane). The transmit antenna 0, the transmit antenna 1, and the transmit antenna 2 are distributed in a shape of a triangle on a longitudinal plane (for example, a vertical plane). The receive antenna 0, the receive antenna 1, the receive antenna 2, and the receive antenna 3 are on a same horizontal line on a longitudinal plane (for example, a vertical plane). In addition, the three transmit antennas and the four receive antennas are disposed on a same longitudinal plane.

For example, as shown in FIG. 24, an end point (which may alternatively be replaced with the center point or the like) of a tail end the antenna 0 is used as the origin Oe of the first coordinate system, the connection line between the antenna 0 and the antenna 1 is used as the Xe axis of the first coordinate system. The direction in which the antenna 1 points to the antenna 0 is the positive direction of the Xe axis. In the plane in which the antenna 0, the antenna 1, and the antenna 2 are located, the straight line perpendicular to the Xe axis is used as the Ze axis of the first coordinate system, and the antenna 2 is located in the positive direction of the Ze axis. Then, with reference to the rule of the right-hand rectangular coordinate system, the Ye axis of the first coordinate system and the positive direction of the Ye axis are determined. An end point (which may alternatively be replaced with the center point or the like) of a tail end of the receive antenna 0 is used as the origin Om of the fourth coordinate system. The connection line between the receive antenna 0 and the receive antenna 1 is used as the Xm axis of the fourth coordinate system. The direction in which the receive antenna 1 points to the receive antenna 0 is the positive direction of the Xm axis. A longitudinal line (for example, a vertical line) passing through the origin Om is used as the Zm axis of the fourth coordinate system. The direction pointing to the zenith is the positive direction of the Zm axis. Then, with reference to the rule of the right-hand rectangular coordinate system, the Ym axis of the fourth coordinate system and the positive direction of the Ym axis are determined. It can be learned that the Xe axis is parallel to the Xm axis, the Ye axis is parallel to the Ym axis, the Ze axis is parallel to the Zm axis, and the fourth coordinate system and the first coordinate system can be converted to each other only through translation.

For example, as shown in FIG. 24, when the first coordinate system is moved a distance dx in a direction parallel to the Xe axis, then moved a distance dy in a direction parallel to the Ye axis, and next, moved a distance dz in a direction parallel to the Ze axis, the first coordinate system coincides with the fourth coordinate system. For example, if a case in which coordinates of a point in the first coordinate system are (xe, ye, ze) and coordinates of the point in the fourth coordinate system are (xm, ym, zm) is defined, [xm, ym, zm]T=[xe, ye, ze]T-[dx, dy, dz]T.

It may be understood that a relative position between the first coordinate system and the fourth coordinate system of the first electronic device 100 may be set in another manner. A similar method may be used to perform conversion between the fourth coordinate system and the first coordinate system. Details are not described herein again.

**(10) Establishment of a fifth coordinate system (whole-house coordinate system) and conversion between the fifth coordinate system and the first coordinate system**

In an example, one first electronic device is disposed in each room or each area. The first electronic device obtains position information of each device and each specified area in the room or the area by using a second electronic device including a UWB module to mark a second electronic device including no UWB module and communicating and interacting with the second electronic device including the UWB module. By using a millimeter-wave radar module, the first electronic device obtains position information of a user in the room or the area, and may further obtain information such as a physiological feature, an identity category, and a human body attitude of the user. The first electronic device controls or notifies, based on the received information, a second electronic device to perform a preset operation. In this example, a separate room or area is used as an example.

In another example, for example, in a whole-house scenario, a hub device may be disposed. Devices such as the hub device, a first electronic device, and a second electronic device constitutes a whole-house system in a wired or wireless manner. The first electronic device obtains position information of each device and each specified area in the room or the area by using a second electronic device including a UWB module to mark a second electronic device including no UWB module and communicating and interacting with the second electronic device including the UWB module. By using a millimeter-wave radar module, the first electronic device obtains position information of a user in the room or the area, and obtain information such as a physiological feature, an identity category, and a human body attitude of the user. The first electronic device sends, to the hub device in a wired or wireless manner, the position information of each device and each specified area, and at least one of information such as a position, the physiological feature, the identity category, and the human body attitude of the user. The hub device controls or notifies, based on the received information, a second electronic device to perform a preset operation.

Optionally, the hub device and a specific first electronic device (for example, the first electronic device in the living room) may be integrated into one device.

In the whole-house scenario, the foregoing information of each room and/or each area needs to be collected in a unified manner. Conversion between different coordinate systems in all rooms is mentioned. For example, position information of the primary bedroom in the first coordinate system and position information of the secondary bedroom in the first coordinate system should be both unified to one coordinate system. In this way, unified control or notification can be performed from a whole-house level. Therefore, the fifth coordinate system (also referred to as the whole-house coordinate system) needs to be established.

For example, the user may input, to the hub device, a floor plan of the whole-house, an installation position of the hub device, a position of the installation position of the hub device in the floor plan of the whole house, height information of a whole house, and the like. The floor plan of the whole house is a plane spatial layout of the house, is a drawing for describing a usage function, a relative position, a size, and the like of each piece of independent space of the whole house. The hub device establishes the fifth coordinate system based on the floor plan of the whole house.

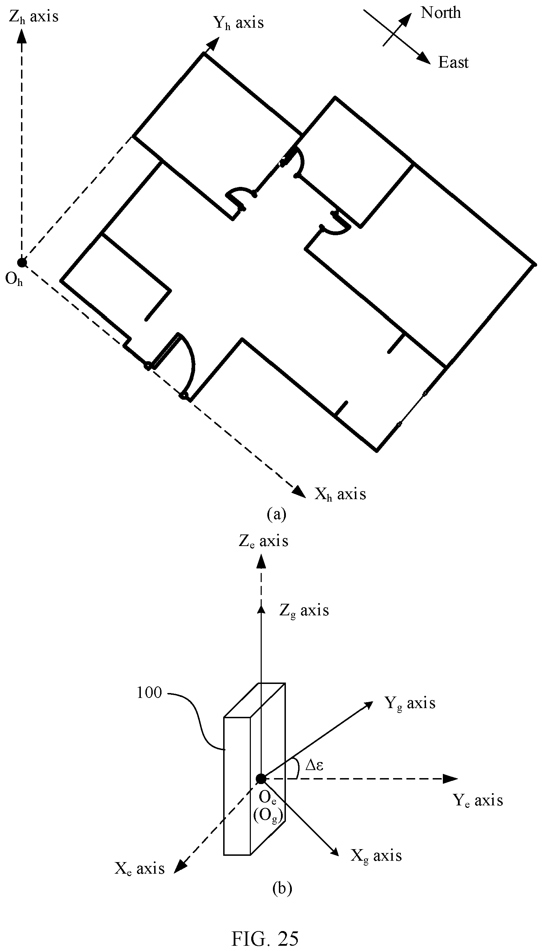

In an example, as shown in (a) in FIG. 25, a projected point that is of the southernmost point of the floor plan of the whole house and that is projected onto a horizontal plane is a first projection point. A first straight line parallel to the east-west direction is drawn passing through the first projected point. A projected point that is of the westernmost point of the floor plan of the whole house and that is projected onto the horizontal plane is a second projected point. A second straight line parallel to the north-south direction is drawn passing through the second projected point. An intersection of the first straight line and the second straight line is used as an origin Oh of the fifth coordinate system. The first straight line is used as an Xh axis, and the due east direction is a positive direction of the Xh axis. The second straight line is used as a Yh axis, and the due north direction is a positive direction of the Yh axis. A Zh axis is perpendicular to the horizontal plane, and a direction pointing to the sky is a positive direction of the Zh axis. Optionally, names of the three axes in the fifth coordinate system and the positive directions of the three axes may alternatively be determined in another manner. Details are not described herein again.

Optionally, the first electronic device includes an IMU module. For example, one hub device is installed in the living room. Both the hub device and the first electronic device in the living room are installed in parallel in a position, for example, a wall or a ceiling. There may be an included angle between the first coordinate system established by the first electronic device and a geographical coordinate system (a sixth coordinate system) on all or some of three axes. The included angle may be output by using the IMU module of the first electronic device, or may be obtained by parsing a result output by the IMU module of the first electronic device, or may be obtained through calculation by using a measurement result of an instrument, for example, a level instrument and/or a plumb bob. For example, (b) in FIG. 25 shows an included angle $\Delta\varepsilon$ between a positive direction of a Yg axis and a positive direction of the Ye axis. Conversion between the sixth coordinate system and the fifth coordinate system is well known to a person skilled in the art. Details are not described herein again. In an implementation, three axes of the sixth coordinate system are respectively parallel to the three axes of the fifth coordinate system. In this way, conversion between the first coordinate system and the fifth coordinate system can be implemented.

In another example, when establishing the fifth coordinate system, the hub device makes three axes of the fifth coordinate system respectively parallel to three axes of the sixth coordinate system, and may further determine an origin Oh of the fifth coordinate system according to the method shown in (a) in FIG. 25. In addition, when the first electronic device is installed, an instrument, for example, a level instrument and/or a plumb bob or a device including an IMU module is used for assistance, so that the three axes of the first coordinate system established by the first electronic device are respectively parallel to the three axes of the sixth coordinate system. In this way, the three axes of the first coordinate system are respectively parallel to the three axes of the fifth coordinate system. Conversion does not need to be performed between the first coordinate system and the fifth coordinate system.

A distance difference between the coordinate origins of the first coordinate system and the fifth coordinate system may be obtained by using two coordinate values of a same hub device in the first coordinate system and the fifth coordinate system. Specifically, the hub device may obtain coordinate information of the hub device in the fifth coordinate system. The coordinate information of the hub device in the first coordinate system may be obtained in two manners. (i) If the hub device includes a UWB module, the coordinate information of the hub device in the first coordinate system may be obtained through UWB communication between the hub device and the first electronic device. (ii) If the hub device includes no UWB module, the second electronic device including the UWB module may mark the hub device, to obtain the coordinate information of the hub device in the first coordinate system. The distance difference between the coordinate origins of the first coordinate system and the fifth coordinate system can be obtained by using the two coordinate values of the same hub device in the first coordinate system and the fifth coordinate system.

**4. Human sensing-based automatic control method**

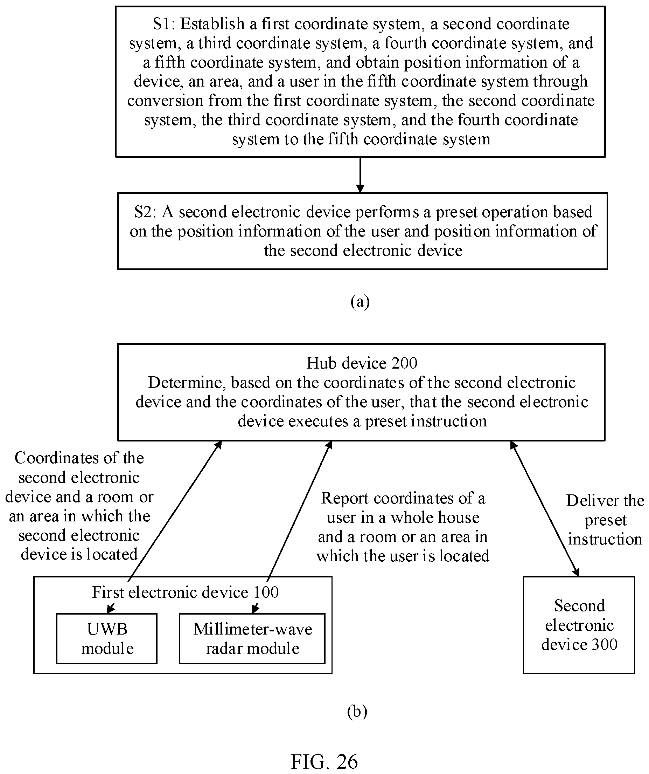

As described above, in a whole-house scenario, one first electronic device is disposed in each room in all or some rooms, one first electronic device is disposed in all or some areas, and one or more second electronic devices are disposed in a single room. For example, (a) in FIG. 26 shows general steps of the human sensing-based automatic control method. As shown in (a) in FIG. 26, the method may include the following steps.

**S1: The first electronic device establishes a first coordinate system and a fourth coordinate system. The second electronic device establishes a second coordinate system. A hub device establishes a fifth coordinate system. Position information of each device, area, and user, and the like in the fifth coordinate system is obtained through conversion from the first coordinate system, the second coordinate system, a third coordinate system, and the fourth coordinate system to the fifth coordinate system.**

S1 is specifically described in the following steps.

**(1) The first electronic device establishes the first coordinate system and the fourth coordinate system. The second electronic device establishes the second coordinate system. The hub device establishes the fifth coordinate system.**

Optionally, a second electronic device including a UWB module establishes the second coordinate system. A second electronic device including no UWB module establishes the third coordinate system.

For establishment of the first coordinate system and the fourth coordinate system by the first electronic device, establishment of the second coordinate system by the second electronic device, and establishment of the fifth coordinate system by the hub device, refer to the foregoing principles. Details are not described herein again.

**(2) Correct an installation error of the first electronic device, and obtain the position information of each device, area, and user, and the like in the fifth coordinate system through conversion from the first coordinate system, the second coordinate system, the third coordinate system, and the fourth coordinate system to the fifth coordinate system.**

There may be an error during installation of the first electronic device. As a result, locating for the second electronic device or the user by the first electronic device is deviated. In an implementation, the first electronic device may be corrected during first use, to reduce or even avoid the error caused by installation.

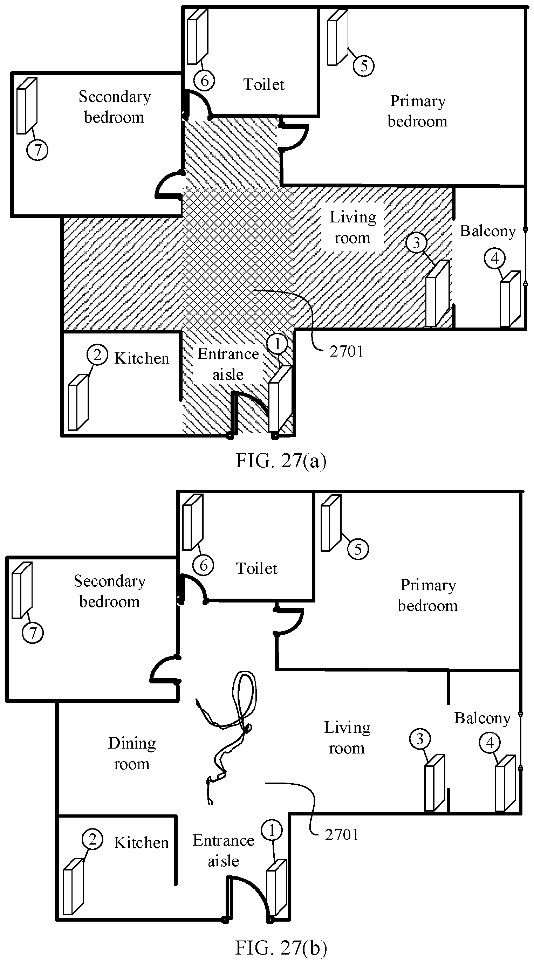

The installation error of the first electronic device may reduce measurement precision of a UWB system. For example, as shown in FIG. 27(a), at least one of a first electronic device ① located in the entrance aisle and a first electronic device ③ located in the living room may have an installation error. For a same second electronic device, the first electronic device ① determines that the second electronic device is located in a position 1, and the first electronic device ③ determines that the second electronic device is located in a position 2. The first electronic device ① and the first electronic device ③ determine, based on an identifier of the second electronic device, that electronic devices respectively located in the position 1 and the position 2 are actually the same second electronic device. Consequently, it is indicated that there is an installation error, which reduces measurement precision.

The first electronic device includes a UWB module and a millimeter-wave radar module. Installation error correction may be separately performed on the UWB module and the millimeter-wave radar module. In an implementation, antennas in the UWB module 150 and the millimeter-wave radar module 160 of the first electronic device 100 are distributed as shown in FIG. 24. Hardware is disposed to ensure that a relative position of the UWB module and the millimeter-wave radar in the first electronic device is fixed. Therefore, installation error correction may be performed on only the UWB module or the millimeter-wave radar module. In another implementation, installation error correction may be performed on both the UWB module and the millimeter-wave radar module of the first electronic device. Correction precision is improved through a plurality of times of corrections.

In an embodiment of this application, an example in which the UWB module of the first electronic device is corrected is used for description. It may be understood that a procedure of correcting the millimeter-wave radar module of the first electronic device is similar to that of correcting the UWB module of the first electronic device. Details are not described herein again. The following method is merely an example, and is not intended to limit a correction method. Another correction method also falls within the scope of this application.

**Step 11: Correct an installation error of a reference first electronic device by using the hub device, to obtain a first correction parameter.**

The reference first electronic device is one of a plurality of first electronic devices. For example, the hub device is installed in the living room, and the first electronic device in the living room may be used as the reference first electronic device. A first coordinate system of the reference first electronic device is denoted as a system e1.

Optionally, the hub device may display a whole-house map. The hub device may indicate the user to hold a second electronic device including a UWB module, and move from a known and easily identified position 1 to another known and easily identified position 2 based on a first track. The reference first electronic device detects a movement track of the second electronic device (by using the UWB module) or a movement track of the user (by using the millimeter-wave radar module). The movement track of the second electronic device is used as an example. There is a deviation between the detected movement track of the second electronic device and the first track.

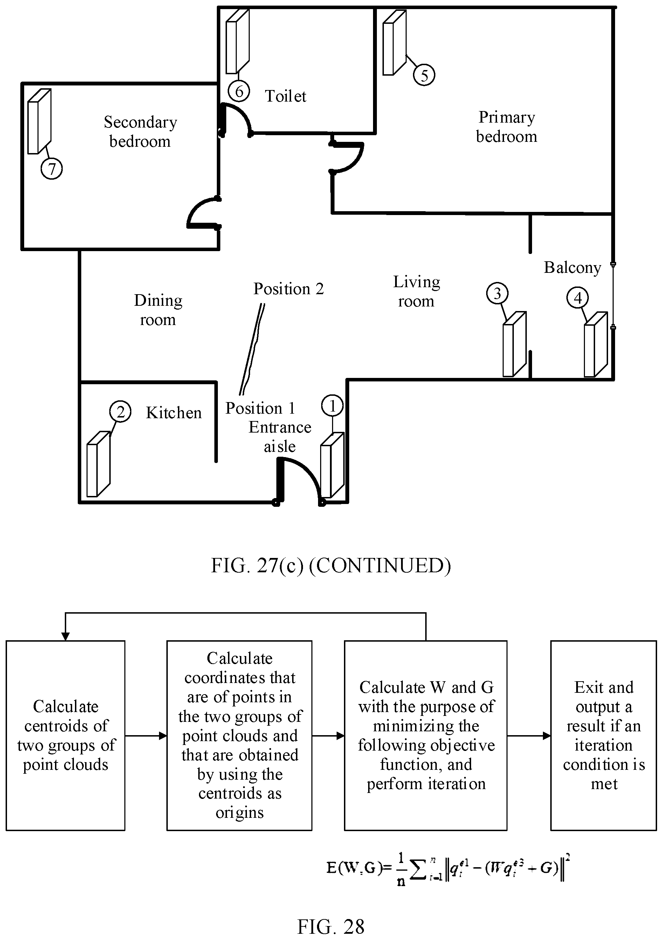

For example, as shown in FIG. 27(c), the hub device may indicate the user to hold the second electronic device including the UWB module, and move from a position 1 with known coordinates to a position 2 with known coordinates along a straight line. The first electronic device ① may obtain an actual movement track of the second electronic device based on detection performed by the UWB module. As shown in FIG. 27(c), there is a specific deviation between a movement track of the user detected by the first electronic device ① and an actual movement track of the user.

Then, an attitude error rotation matrix W and a position error vector G may be obtained through calculation according to an algorithm. For example, the algorithm may be an ICP algorithm shown in FIG. 28. An optimal matching attitude error rotation matrix W and position error vector G may be obtained through calculation according to the ICP algorithm. In this way, an error function is minimized. For specific content of the ICP algorithm, refer to the conventional technology. Details are not described herein again. The first correction parameter includes the current optimal matching attitude error rotation matrix W and position error vector G.

After the reference first electronic device is corrected, the installation error of the first electronic device is corrected based on the reference first electronic device.

**Step 12: Correct the installation error of the first electronic device by using the reference first electronic device, to obtain a second correction parameter.**

In an example, the user holds the second electronic device including the UWB module, and moves in each room of a whole house. Each first electronic device in the whole house locates the second electronic device. For example, as shown in FIG. 27(a), there is an overlapping area 2701 between a signal coverage area of the first electronic device ① and a signal coverage area of the first electronic device (3). When the user moves in the overlapping area 2701, as shown in FIG. 27(b), there is a specific deviation between movement tracks of the second electronic device that are obtained by the first electronic device ① and the first electronic device (3). The first electronic device ① is the reference first electronic device.

In an implementation, installation errors of two first electronic devices are corrected based on movement tracks of the second electronic device that are respectively detected by the two first electronic devices. For example, the correction parameter includes an attitude error rotation matrix W and a position error vector G. For example, the first electronic device ① obtains a movement track $\mathrm{qe}1 = [q_{t1}^{e1}, q_{t2}^{e1}, q_{t3}^{e1}, \dots, q_{tn}^{e1}]$ of the second electronic device in the first coordinate system (the system e1) established by the first electronic device ①. The first electronic device (3) obtains a movement track $\mathrm{qe}3 = [q_{t1}^{e3}, q_{t2}^{e3}, q_{t3}^{e3}, \dots, q_{tn}^{e3}]$ of the second electronic device in a first coordinate system (a system e3) established by the first electronic device ③. Herein, $q_{tn}^{e1}$ represents coordinates of the second electronic device in the system e1 that are detected by the first electronic device ① at a moment tn, and $q_{tn}^{e3}$ represents coordinates of the second electronic device in the system e3 that are detected by the first electronic device ③ at the moment tn. The movement tracks qe1 and qe3 of the user may be converted to the fifth coordinate system according to a subsequent formula (13), and are respectively denoted as qe1->h and qe3->h.

Herein, qe1->h and qe3->h are point clouds that record a movement track of the user. An attitude error rotation matrix We3->e1 and a position error vector Ge3->e1 between the two point clouds qe1->h and qe3->h may be calculated according to an iterative closest point (iterative closest point, ICP) algorithm, to minimize a three-dimensional spatial error between a corrected point cloud qe1->h and the point cloud qe3->h. A basic principle of the ICP algorithm is shown in FIG. 28. A closest point ( $q_i^{e1}$, $q_i^{e3}$ ) is found in a to-be-matched target point cloud qe3->h and a reference point cloud qe1->h based on a constraint condition $\mathrm{E}(\mathrm{W}, \mathrm{G}) = \frac{1}{n} \sum_{i=1}^{n} \| q_i^{e1} - (W q_i^{e3} + G) \|^2$. Then an optimal matching attitude error rotation matrix W and position error vector G are obtained through calculation, to minimize an error function. For a specific step of the ICP algorithm, refer to the conventional technology. Details are not described again in embodiments of this application. The second correction parameter includes the current optimal matching attitude error rotation matrix W and position error vector G.

Optionally, an order between step 11 and step 12 may be changed. In addition, the foregoing step 11 and step 12 are merely examples. In another example, correction of all first electronic devices is implemented by using the hub device, instead of using the reference first electronic device.

When the user moves without holding the second electronic device including the UWB module, each first electronic device may detect a movement track of the user. A related manner is similar to a manner of processing the movement track of the second electronic device. Details are not described herein again.

**Step 13: Convert, to coordinates in the fifth coordinate system, coordinates of the second electronic device in the first coordinate system or coordinates of the user in the fourth coordinate system that are obtained by the first electronic device through calculation.**

In an example, an attitude error rotation matrix and a position error vector that are of the reference first electronic device relative to the hub device are obtained by performing the foregoing step 11. For example, the first electronic device ① is used as the reference first electronic device. An attitude error rotation matrix and a position error vector of the first electronic device ① relative to the hub device are denoted as [We1->h, Ge1->h].

An attitude error rotation matrix and a position error vector of another first electronic device relative to the reference first electronic device are corrected by performing the foregoing step 12. For example, the first electronic device ① is used as the reference first electronic device. An attitude error rotation matrix and a position error vector of the another first electronic device relative to the first electronic device ① are denoted as [Wek->e1, Gek->e1], where k ∈ 2, ..., n.

In an example, the first electronic device ① is used as the reference first electronic device. Coordinates that are of an origin of a first coordinate system of a kth other first electronic device other than the reference first electronic device and that are in the fifth coordinate system are expressed as $[x_o^{kh}, y_o^{kh}, z_o^{kh}]$. Any point in space is selected as a point q. Coordinates of the point q in the first coordinate system established by the kth first electronic device are expressed as $qek = [x_q^{ek}, y_q^{ek}, z_q^{ek}]$. Coordinates of the point q in the fifth coordinate system that are obtained after installation error correction are expressed as $q'^h = [x_q'^h, y_q'^h, z_q'^h]$. The coordinates of the point q in the first coordinate system established by the kth first electronic device may be converted to the fifth coordinate system according to formula (12) after undergoing installation error correction.

$$[x_q'^h, y_q'^h, z_q'^h]^T = W^{e1 \to h}(W^{ek \to e1}(C_e^h \cdot [x_q^{ek}, y_q^{ek}, z_q^{ek}]^T + [x_o^{kh}, y_o^{kh}, z_o^{kh}]^T) + G^{ek \to e1}) + G^{e1 \to h}$$

$$\text{formula (12)}$$

Coordinates, of an origin of the first coordinate system established by the reference first electronic device, in the fifth coordinate system are expressed as $[x_o^{1h}, y_o^{1h}, z_o^{1h}]$. Coordinates of the point q in the first coordinate system established by the reference first electronic device are expressed as $q^{e1} = [x_q^{e1}, y_q^{e1}, z_q^{e1}]$. Coordinates of the point q in the fifth coordinate system that are obtained after installation error correction are expressed as $q'^h = [x_q'^h, y_q'^h, z_q'^h]$. The coordinates of the point q in the first coordinate system established by reference the first electronic device may be converted to the fifth coordinate system according to formula (13) after undergoing installation error correction.

$$[x_q'^h, y_q'^h, z_q'^h]^T = W^{e1 \to h}(C_e^h \cdot [x_q^{e1}, y_q^{e1}, z_q^{e1}]^T + [x_o^{1h}, y_o^{1h}, z_o^{1h}]^T) + G^{e1 \to h} \qquad \text{formula (13)}$$

In another example, coordinates detected by the first electronic device are directly converted to the fifth coordinate system after undergoing installation error correction, without using the reference first electronic device. For example, the attitude error rotation matrix and the position error vector between two point clouds of the movement track of the user detected by the first electronic device and the actual movement track of the user, are respectively denoted as $W^{e \to h}$ and $G^{e \to h}$. Coordinates, of an origin of the first coordinate system established by the first electronic device, in the fifth coordinate system are expressed as $[x_o^h, y_o^h, z_o^h]$. Coordinates of the point q in the first coordinate system established by the first electronic device are expressed as $q^e = [x_q^e, y_q^e, z_q^e]$.

Coordinates of the point q in the fifth coordinate system that are obtained after installation error correction are expressed as $q'^h = [x_q'^h, y_q'^h, z_q'^h]$. The coordinates of the point q in the first coordinate system established by the first electronic device may be converted to the fifth coordinate system according to formula (14) after undergoing installation error correction.

$$[x_q'^h, y_q'^h, z_q'^h]^T = W^{e \to h}(C_e^h \cdot [x_q^e, y_q^e, z_q^e]^T + [x_o^h, y_o^h, z_o^h]^T) + G^{e \to h} \qquad \text{formula (14)}$$

It may be understood that a method for converting, to the fifth coordinate system, the coordinates of the user in the fourth coordinate system that are detected by the first electronic device and that undergo installation error correction is similar to a method for converting, to the fifth coordinate system, the coordinates of the user in the first coordinate system that are detected by the first electronic device and that undergo installation error correction. Details are not described herein again.

During first use, the second electronic device in the whole house may be located by using the UWB module of the first electronic device. A first electronic device in each room or area may determine coordinates of a second electronic device in the room or the area in a first coordinate system of the first electronic device. Optionally, for a plurality of first electronic devices whose signal coverage areas have an overlapping area, one of the plurality of first electronic devices may locate a second electronic device in the overlapping area.

Further, in an implementation, each first electronic device in the whole house converts obtained coordinates of one or more second electronic devices in a first coordinate system to the fifth coordinate system, and sends coordinates of the one or more second electronic devices in the fifth coordinate system to the hub device. In another implementation, each first electronic device in the whole house sends obtained coordinates of one or more second electronic devices in first coordinate system to the hub device, and the hub device converts, to the fifth coordinate system, the coordinates of the second electronic device in the first coordinate system that are received from each first electronic device. Optionally, converting the coordinates of the second electronic device in the first coordinate system to the fifth coordinate system includes: converting, to the fifth coordinate system, the coordinates of the second electronic device in the first coordinate system that undergo installation error correction. The hub device may store the obtained coordinates of the second electronic device in the fifth coordinate system.

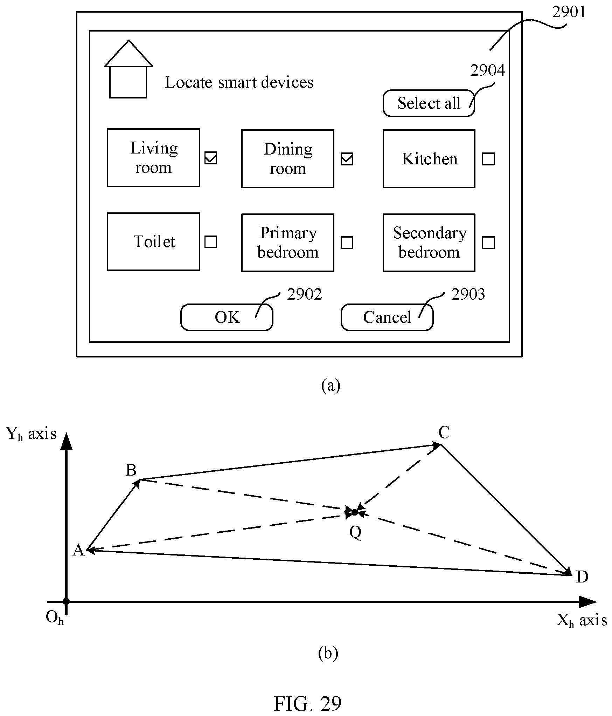

Optionally, after first use, a second electronic device may be added to or removed from the whole house, or a position of the second electronic device may change. In an implementation, the hub device cyclically locates the second electronic device by using the first electronic device in each room or each area, and updates the coordinates of the second electronic device that are stored in the hub device. In another implementation, when detecting the second electronic device newly added to the whole house, the hub device triggers locating the second electronic device by using the first electronic device, and updates the coordinates of the second electronic device that are stored in the hub device. For example, the hub device stores configuration information of all devices such as the first electronic device and the second electronic device in the whole house. The second electronic device accesses the hub device, and corresponding configuration information is newly added. If the hub device determines, based on the configuration information, that the second electronic device is newly added, the hub device triggers the first electronic device to locate the second electronic device. In another implementation, after the second electronic device is added to or removed from the whole house, or the position of the second electronic device changes, the user may manually trigger the first electronic device to locate the second electronic device, and update the coordinates of the second electronic device that are stored in the hub device. For example, the user starts, through a human-machine interaction interface displayed on a control panel, the first electronic device to locate the second electronic device. For example, as shown in (a) in FIG. 29, a "Locate a hub device" interface 2901 is displayed on the control panel. The "Locate a hub device" interface 2901 includes room options such as the living room, a dining room, and a kitchen. The user may select one or more of the room options, and click an "OK" button 2902, to enable a first electronic device in a corresponding room to locate a hub device in the room. The "Locate a hub device" interface 2901 further includes a "Cancel" button 2903, used to cancel performing of locating an IoT device. Optionally, the "Locate an IoT device" interface 2901 further includes a "Select all" button 2904. The user may tap the "Select all" button 2904 to select all rooms in the house, and tap the "OK" button 2902, to enable the first electronic device in the whole house to separately locate the second electronic devices.

Optionally, after first use, the first electronic device in each room or each area may be used to cyclically locate the user in the whole house and track a movement track of each user. For example, a cycle is 1 second. The first electronic device performs detection at a frequency of 10 Hz (hertz) (10 times per second), and sends a detection result to the hub device at a frequency of 1 Hz (once per second). Each first electronic device in the whole house may locate (obtain the coordinates of the user in the fourth coordinate system) and track (obtain a movement track of the user in the fourth coordinate system) the user in a signal coverage area of the first electronic device. Optionally, for the plurality of first electronic devices whose signal coverage areas have the overlapping area, one of the plurality of first electronic devices may locate and track a user in the overlapping area. Further, in an implementation, each first electronic device in the whole house converts obtained coordinates or movement tracks of one or more users in the fourth coordinate system to coordinates or movement tracks in the fifth coordinate system, and sends the coordinates or the movement tracks of the one or more users in the fifth coordinate system to the hub device. In another implementation, each first electronic device in the whole house sends obtained coordinates or movement tracks of one or more users in the fourth coordinate system to the hub device. The hub device converts, to coordinates or movement tracks in the fifth coordinate system, the coordinates or the movement track that is of the user in the fourth coordinate system and that is received from each first electronic device. Optionally, converting the coordinates or the movement track of the user in the fourth coordinate system to the fifth coordinate system includes: converting, to the coordinates or the movement track in the fifth coordinate system after, the coordinates or the movement track that is of the user in the fourth coordinate system and that undergoes installation error correction. The hub device may store and cyclically update an obtained position of the user (for example, the coordinates of the user in the fifth coordinate system) or movement track (a coordinate track in the fifth coordinate system).

It should be noted that step (2) is not mandatory and is optional. For example, at the beginning of installation, correction is performed once. Later, correction is generally not required, or correction is performed once again after a long period of use. When step (2) is performed, step (3) does not need to be performed. When step (2) is not performed, step (3) is performed. That is, either step (2) or step (3) is performed.

**(3) Obtain the position information of each device, area, and user, and the like in the fifth coordinate system through conversion from the first coordinate system, the second coordinate system, the third coordinate system, and the fourth coordinate system to the fifth coordinate system.**

Conversion from the first coordinate system, the second coordinate system, the third coordinate system, and the fourth coordinate system to the fifth coordinate system may be specifically implemented as follows: Conversion between the second coordinate system, the third coordinate system, and the first coordinate system may be implemented by obtaining $C_e^b$ and $C_e^t$ based on the foregoing principle. The conversion between the fourth coordinate system and the first coordinate system is described in the foregoing principle part. After conversion from all the second coordinate system, the third coordinate system, and the fourth coordinate system to the first coordinate system, $C_e^h$ may be obtained based on the foregoing principle. Then, conversion from the first coordinate system to the fifth coordinate system is implemented.

$$C_e^h =$$

$$\begin{bmatrix} cos\theta_e^h cos\psi_e^h + sin\varphi_e^h sin\theta_e^h sin\psi_e^h & cos\varphi_e^h sin\psi_e^h & sin\theta_e^h cos\psi_e^h - sin\varphi_e^h cos\theta_e^h sin\psi_e^h \\ -cos\theta_e^h sin\psi_e^h + sin\varphi_e^h sin\theta_e^h cos\psi_e^h & cos\varphi_e^h cos\psi_e^h & -sin\theta_e^h sin\psi_e^h - sin\varphi_e^h cos\theta_e^h cos\psi_e^h \\ -cos\varphi_e^h sin\theta_e^h & sin\varphi_e^h & cos\varphi_e^h cos\theta_e^h \end{bmatrix}$$

$$\text{formula (15)}$$

Herein, $\psi_e^h$, $\varphi_e^h$, and $\theta_e^h$ are respectively a heading angle, a pitch, and a roll of the fifth coordinate system relative to the first coordinate system. For the any point q in the space of the whole house, coordinates $q^h = [x_q^h, y_q^h, z_q^h]$ of the any point q in the fifth coordinate system are obtained, the coordinates $q^e = [x_q^e, y_q^e, z_q^e]$ of the any point q in the first coordinate system are obtained, and coordinates of an origin $O_e$ of the first coordinate system in the fifth coordinate system are $[x_o^h, y_o^h, z_o^h]$. The coordinates of the point q in the first coordinate system may be converted to the fifth coordinate system according to formula (16).

$$[x_q^h, y_q^h, z_q^h]^T = C_e^h \cdot [x_q^e, y_q^e, z_q^e]^T + [x_o^h, y_o^h, z_o^h]^T \qquad \text{formula (16)}$$

Optionally, the first electronic device may convert coordinates of the first electronic device in the first coordinate system or the fourth coordinate system to the fifth coordinate system. To be specific, the coordinates in the fourth coordinate system do not need to be first converted into coordinates in the first coordinate system, and then the coordinates in the first coordinate system are converted into coordinates in the fifth coordinate system. Instead, the coordinates in the fourth coordinate system may be directly converted into the coordinates in the fifth coordinate system. Then, the converted coordinates in the fifth coordinate system are sent to the hub device. $C_m^h$ may be obtained based on the foregoing principle. Then, conversion from the fourth coordinate system to the fifth coordinate system is implemented.

$$C_m^h =$$

$$\begin{bmatrix} cos\theta_m^h cos\psi_m^h + sin\varphi_m^h sin\theta_m^h sin\psi_m^h & cos\varphi_m^h sin\psi_m^h & sin\theta_m^h cos\psi_m^h - sin\varphi_m^h cos\theta_m^h sin\psi_m^h \\ -cos\theta_m^h sin\psi_m^h + sin\varphi_m^h sin\theta_m^h cos\psi_m^h & cos\varphi_m^h cos\psi_m^h & -sin\theta_m^h sin\psi_m^h - sin\varphi_m^h cos\theta_m^h cos\psi_m^h \\ -cos\varphi_m^h sin\theta_m^h & sin\varphi_m^h & cos\varphi_m^h cos\theta_m^h \end{bmatrix}$$

$$\text{formula (17)}$$

Herein, $\psi_m^h$, $\varphi_m^h$, and $\theta_m^h$ are respectively a heading angle, a pitch, and a roll of the fifth coordinate system relative to the fourth coordinate system. For the any point q in the space of the whole house, the coordinates $q^h=[x_q^h, y_q^h, z_q^h]$ of the any point q in the fifth coordinate system are obtained, coordinates $q^m=[x_q^m, y_q^m, z_q^m]$ of the any point q in the fourth coordinate system are obtained, and coordinates of an origin $O_m$ of the fourth coordinate system in the fifth coordinate system are $[x_o^h, y_o^h, z_o^h]$. Coordinates of the point q in the first coordinate system may be converted to the fifth coordinate system according to formula (18).

$$[x_q^h, y_q^h, z_q^h]^{\mathrm{T}}=C_m^h \cdot [x_q^m, y_q^m, z_q^m]^{\mathrm{T}}+[x_o^h, y_o^h, z_o^h]^{\mathrm{T}} \qquad \text{formula (18)}$$

Optionally, the foregoing conversion is performed by the hub device. The first electronic device separately sends coordinates of the first electronic device in the first coordinate system or the fourth coordinate system to the hub device. The hub device converts the coordinates, that are based on the first coordinate system or the fourth coordinate system of each first electronic device, into coordinates in the fifth coordinate system.

Optionally, one reference first electronic device is set in the plurality of first electronic devices. Another first electronic device other than the reference first electronic device sends coordinate information of the another first electronic device in a first coordinate system or a fourth coordinate system to the reference first electronic device. The reference first electronic device converts coordinates, that are based on the first coordinate system or the fourth coordinate system of each first electronic device, into coordinates in the fifth coordinate system, and sends, to the hub device, the coordinates in the fifth coordinate system that are obtained after conversion.

**S2: The second electronic device performs a preset operation based on the position information of the user and the position information of the second electronic device.**

In an embodiment of this application, the whole house is divided into one or more rooms and/or one or more areas, which do not overlap each other. The hub device may locate the room or the area by using the first electronic device, and obtain and store a coordinate range of each room or area. For example, the coordinate range of each room or area may be obtained according to the method in (b) in FIG. 14.

The hub device may determine a room or an area in which each first electronic device and each second electronic device are located. In an implementation, the user may query by using the hub device, for example, input a device name (a name of the first electronic device or a name of the second electronic device), a room or an area in which a device is located, or the like. In an implementation, at least one first electronic device is installed in each room or area. The hub device determines, based on an input of the user, a room or an area in which each first electronic device is located. In an implementation, the hub device determines, based on coordinates of the first electronic device or the second electronic device and the coordinate range of each room or area in the whole house, a room or an area in which each first electronic device or each second electronic device is located.

For example, as shown in (b) in FIG. 29, for a room whose horizontal direction is a quadrilateral, a smartphone may be separately placed at four positions in the room, namely, a point A, a point B, a point C, and a point D according to the method shown in (a) in FIG. 14. Coordinates $(x_A^h, y_A^h, 0), (x_B^h, y_B^h, 0), (x_C^h, y_C^h, 0)$, and $(x_D^h, y_D^h, 0)$ of the four positions, namely, the point A, the point B, the point C, and the point D, in the fifth coordinate system are separately obtained by using the first electronic device 100. A vertical line passing through the position A, a vertical line passing through the position B, a vertical line passing through the position C, and a vertical line passing through the position D may determine an area range of an area obtained, through division, in the room. Coordinates of the second electronic device in the fifth coordinate system are $(x_Q^h, y_Q^h, z_Q^h)$, and $(x_Q^h, y_Q^h, 0)$ are coordinates of a projected point Q of the second electronic device in a plane XhOhYh. A convex quadrilateral is obtained by connecting A, B, C, and D in sequence in the clockwise direction. Four sides of the convex quadrilateral are respectively $\overrightarrow{AB}$, $\overrightarrow{BC}$, $\overrightarrow{CD}$, and $\overrightarrow{DA}$. If it is determined that the point Q is on the right side of the four sides $\overrightarrow{AB}$, $\overrightarrow{BC}$, $\overrightarrow{CD}$, and $\overrightarrow{DA}$, it is determined that the point Q is located in the convex quadrilateral including the point A, the point B, the point C, and the point D. To be specific, it is determined that the second electronic device is located in the room. For example, if the coordinates $(x_Q^h, y_Q^h, 0)$ of the point Q and the coordinates of the point A, the point B, the point C, and the point D satisfies formula (19), it is determined that the point Q is located in an area range including the point A, the point B, the point C, and the point D.

$$\overrightarrow{AB} \times \overrightarrow{AQ} < 0$$

$$\overrightarrow{BC} \times \overrightarrow{BQ} < 0 \qquad\qquad \text{formula (19)}$$

$$\overrightarrow{CD} \times \overrightarrow{CQ} < 0$$

$$\overrightarrow{DA} \times \overrightarrow{DQ} < 0$$

Herein, $\times$ indicates a vector cross product. $\overrightarrow{AB} \times \overrightarrow{AQ}$ indicates a vector cross product of $\overrightarrow{AB}$ and $\overrightarrow{AQ}$. $\overrightarrow{BC} \times \overrightarrow{BQ}$ indicates a vector cross product of $\overrightarrow{BC}$ and $\overrightarrow{BQ}$. $\overrightarrow{CD} \times \overrightarrow{CQ}$ indicates a vector cross product of $\overrightarrow{CD}$ and $\overrightarrow{CQ}$. $\overrightarrow{DA} \times \overrightarrow{DQ}$ indicates a vector cross product of $\overrightarrow{DA}$ and $\overrightarrow{DQ}$. A vector cross product of two vectors is a scalar.

For example,

$$\overrightarrow{AB} = ((x_B^h - x_A^h), (y_B^h - y_A^h)), \ \overrightarrow{AQ} = ((x_Q^h - x_A^h), (y_Q^h - y_A^h)),$$

$$\overrightarrow{BC} = ((x_C^h - x_B^h), (y_C^h - y_B^h)), \ \overrightarrow{BQ} = ((x_Q^h - x_B^h), (y_Q^h - y_B^h)),$$

$$\overrightarrow{CD} = ((x_D^h - x_C^h), (y_D^h - y_C^h)), \ \overrightarrow{CQ} = ((x_Q^h - x_C^h), (y_Q^h - y_C^h)),$$

$$\overrightarrow{DA} = ((x_A^h - x_D^h), (y_A^h - y_D^h)), \ \overrightarrow{DQ} = ((x_Q^h - x_D^h), (y_Q^h - y_D^h)),$$

$$\overrightarrow{AB} \times \overrightarrow{AQ} = (x_B^h - x_A^h)(y_Q^h - y_A^h) - (x_Q^h - x_A^h)(y_B^h - y_A^h),$$

$$\overrightarrow{BC} \times \overrightarrow{BQ} = (x_C^h - x_B^h)(y_Q^h - y_B^h) - (x_Q^h - x_B^h)(y_C^h - y_B^h),$$

$$\overrightarrow{CD} \times \overrightarrow{CQ} = (x_D^h - x_C^h)(y_Q^h - y_C^h) - (x_Q^h - x_C^h)(y_D^h - y_C^h),$$

and

$$\overrightarrow{DA} \times \overrightarrow{DQ} = (x_A^h - x_D^h)(y_Q^h - y_D^h) - (x_Q^h - x_D^h)(y_A^h - y_D^h).$$

In an example, the hub device may store a device information table in the whole house. The device information table includes information about one or more devices (including but not limited to the first electronic device, the second electronic device, and the like) in the whole house. For example, the information about the device includes a device name and a room or an area (room) in which the device is located, and optionally, may further include coordinates (for example, coordinates in the fifth coordinate system) of the device. For example, the device information table is shown in Table 4.

**Table 4**

| Room | Device name | Coordinates |
|------|-------------|-------------|
| Living room | Home theater (left) | $(x_1^h, \ y_1^h, \ z_1^h)$ |
| Living room | Home theater (right) | $(x_2^h, \ y_2^h, \ z_2^h)$ |

(continued)

| Room | Device name | Coordinates |
|---|---|---|
| Living room | Router 1 | $(x_3^h,\ y_3^h,\ z_3^h)$ |
| Living room | Living-room television | $(x_4^h,\ y_4^h,\ z_4^h)$ |
| Primary bedroom | Bedside speaker | $(x_5^h,\ y_5^h,\ z_5^h)$ |
| Primary bedroom | Control panel 1 | $(x_6^h,\ y_6^h,\ z_6^h)$ |
| Secondary bedroom | Escritoire speaker | $(x_7^h,\ y_7^h,\ z_7^h)$ |
| Balcony | Balcony speaker | $(x_8^h,\ y_8^h,\ z_8^h)$ |
| ... | ... | ... |

The hub device may further determine a room or an area in which the user is located. In an implementation, the at least one first electronic device is installed in each room or area. The hub device determines the room or the area in which each first electronic device is located. The room or the area in which each first electronic device is located is the room or the area in which the user that can be detected by the first electronic device is located. In an implementation, the hub device determines the room or the area in which each user is located based on coordinates of the user and the coordinate range of each room or area in the whole house. For a specific method, refer to the method of determining, by the hub device based on the coordinates of the second electronic device and the coordinate range of each room or area in the whole house, the room or the area in which each second electronic device is located. Further, the hub device cyclically obtains the coordinates of the user, and determines, based on the coordinates of the user, the room or the area in which the user is located. In another implementation, the hub device obtains a coordinate range of the whole house and the coordinate range of each room or each area in the whole house based on a floor plan of the whole house, an installation position of the hub device, a position of the installation position of the hub device in the floor plan of the whole house, height information of the whole house, and the like. Then, the hub device may know, based on obtained coordinates of the user through comparison, the room or the area in which the user is located and that is in the whole house. The hub device may determine, based on a room or an area in which the user is currently located and a room or an area in which the user is located within a previous cycle, that the user enters another room or area from one room or area, or leaves the whole house, enters the whole house, or the like.

Optionally, the hub device may further obtain at least one of information such as a physiological feature, an identity category, and a human body attitude from the first electronic device in each room or each area, and then may subsequently notify or control, based on the at least one of the information such as the position information, the physiological feature, the identity category, and the human attitude, a corresponding second electronic device in a corresponding room or a corresponding area to perform the preset operation.

Specific content of S2 may be further described subsequently with reference to a specific scenario.

For example, (b) in FIG. 26 shows an implementation of the human sensing-based automatic control method according to an embodiment of this application. As shown in (b) in FIG. 26, the UWB module of the first electronic device locates the device, the room, the area, and the like in the whole house, obtains coordinates of the device in the whole house and the room or the area in which the device is located, and reports the coordinates and room or the area to the hub device. The millimeter-wave radar module of the first electronic device performs target tracking on the user in the whole house, and cyclically reports, to the hub device, the coordinates of the user in the whole house and the room or the area in which the user is located. The hub device sends a corresponding preset instruction to the second electronic device based on the coordinates of the second electronic device and the coordinates of the user. In an implementation, if it is determined that a relative position between a first user and the second electronic device satisfies a preset condition, the second electronic device is controlled to execute the preset instruction. Coordinates of the first user may be coordinates of one user or an average value of coordinates of a plurality of users. The second electronic device executes the preset instruction. In this way, for example, when the user approaches a smart lamp, the second electronic device can execute the preset instruction, for example, turn on the smart lamp, by performing a procedure shown in (b) in FIG. 26.

It should be noted that, in the human sensing-based automatic control method provided in embodiments of this application, the hub device may determine, based on the coordinates of the second electronic device and the coordinates of the user, that the second electronic device executes the preset instruction. Alternatively, another device other than the hub device may determine, based on the coordinates of the second electronic device and the coordinates of the user, that the second electronic device executes the preset instruction. Alternatively, the hub device sends the coordinates of the second electronic device and the coordinates of the user to the second electronic device, and the second electronic device determines, based on the coordinates, to execute the preset instruction. It may be understood that an execution body of the human sensing-based automatic control method is not limited in embodiments of this application.

**5. Description of specific embodiments**

After the general descriptions of the overall scenario, the hardware structure of the electronic device, the locating principle, and the human sensing-based automatic control method are provided, the following further describes, with reference to accompanying drawings and specific scenarios, the human sensing-based automatic control method by using a plurality of embodiments. In this way, how a user is enabled to perform automatic control on an IoT device more conveniently according to the technical solutions provided in embodiments of this application is more clearly described, to further improve user experience. In this process, the user does not need to carry any electronic device.

It should be noted that some embodiments include communication and interaction between a plurality of different second electronic devices and a hub device, and even further include communication interaction between the plurality of different second electronic devices. For ease of subsequent description, the following uses a first device, a second device, a third device, and the like to indicate different second electronic devices.

It should be noted that, in specific embodiments, the human sensing-based automatic control method provided in embodiments of this application may be separately refined into a human sensing-based method for automatically switching a device to perform an alarm clock, a human sensing-based device registration and control method, a human sensing-based device automatic reminding method, a human sensing-based method for calling a member in a house, and the like. The following specifically provides descriptions with reference to the specific embodiments.

**Embodiment 1: Distributed alarm clock**

An alarm clock is a practical gadget in daily life and office. The alarm clock can effectively remind a user of a schedule and a key affair in a current day, to help the user appropriately plan time and effectively arrange various affairs of the user. Currently, most devices such as mobile phones, smart speakers, large-screen devices (such as televisions), and chatbots each are provided with an alarm clock function.

In the conventional technology, for the alarm clock set on the mobile phone, an alarm clock task may be performed by a smartwatch worn on the user. When determining that an alarm start moment arrives, the mobile phone may notify, by using a Bluetooth connection, the smartwatch to perform the alarm clock task. If a distance between the mobile phone and the smartwatch is long, for example, is greater than a Bluetooth communication range (for example, 10 meters), the smartwatch cannot receive a notification from the mobile phone, and cannot perform the alarm clock task. In addition, in an indoor environment, due to interference from a wall, a door, a window, and furniture, a Bluetooth signal is seriously attenuated. This also affects communication quality of mobile phone and smartwatch. If a Bluetooth function of the mobile phone is not enabled in time at the alarm start moment, the mobile phone cannot send, to the smartwatch, the notification for performing the alarm clock task. In addition, if both the mobile phone and the smartwatch are located near the user, both the two devices perform the alarm clock task, resulting in poor alarm clock experience for the user.

Therefore, embodiments of this application provide a solution of the distributed alarm clock. A most suitable device is matched, based on information such as a current position or a movement track of a user obtained by a whole-house sensing system, for the user to perform an alarm clock function, to provide a short-distance alarm clock service for the user.

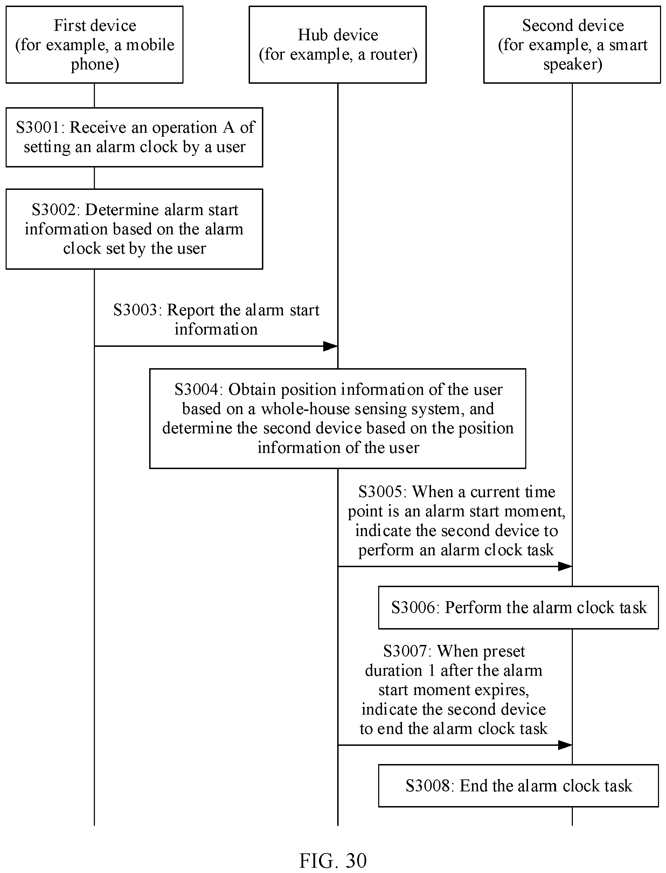

FIG. 30 is a schematic flowchart of a method for implementing a distributed alarm clock according to an embodiment of this application. The procedure includes the following steps.

S3001: A first device receives an operation A of setting an alarm clock by a user.

The first device may be, for example, a mobile phone, a tablet computer, a smartwatch, a PC, a smart speaker, or a large-screen device. The user may set the alarm clock in a manner of a graphical user interface (graphical user interface, GUI) or a voice user interface (voice user interface, VUI).

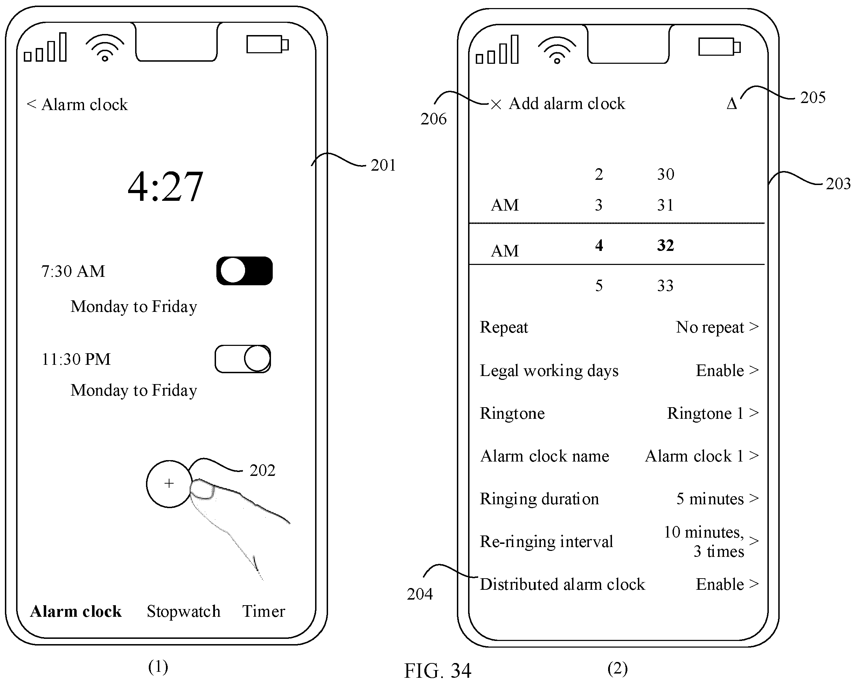

Herein, the mobile phone is used as an example to describe a process in which the user sets the alarm clock in the manner of the GUI. For example, in response to an operation of starting an alarm clock application on a desktop of the mobile phone by the user, the mobile phone displays an interface 201 shown in (1) in FIG. 34. The interface 201 is a main interface of the alarm clock application. The interface 201 includes an alarm clock that is set and a control 202 for adding an alarm clock. In response to operating, by the user, the control 202 for adding the alarm clock, the mobile phone displays an interface 203 shown in (2) in FIG. 34. The interface 203 is an interface for adding the alarm clock. The user may input, on the interface 203, basic information of the to-be-added alarm clock, for example, an alarm start moment, whether to ring repeatedly, whether to avoid legal holidays, a ring tone, an alarm clock name, ringing duration, whether to re-ring, and a re-ringing interval of the to-be-added alarm clock. Optionally, the interface 203 may further include a function option 204 of the distributed alarm clock. The function option 204 of the distributed alarm clock is used by the user to enable or disable a distributed alarm clock function of the added alarm clock. The distributed alarm clock function is a function of ringing on another device according to the technical solutions provided in embodiments of this application based on a user position. The following describes the distributed alarm clock function in detail, and is not described herein. After inputting alarm start information of the to-be-added alarm clock, the user may operate a determining control 205, and the mobile phone stores the alarm start information of the to-be-added alarm clock. Alternatively, the user may alternatively cancel the to-be-added alarm clock edited this time by operating a cancel control 206, and the mobile phone returns to the interface 201 shown in (1) in FIG. 34.

The operation A includes a series of operations of adding the alarm clock, for example, includes starting the alarm clock application, operating the control 202 for adding the alarm clock, inputting the alarm start information of the to-be-added alarm clock, and operating the determining control 205. Certainly, the operation A herein may further include modifying an existing alarm clock, for example, modifying an alarm clock, whose distributed alarm clock function is not enabled, to enable the distributed alarm clock function, or modifying alarm start information of an alarm clock whose distributed alarm clock function is enabled.

It should be noted that an example in which the user sets the clock application on the mobile phone to set the alarm clock is used for description herein. In some other examples, the user may alternatively set the alarm clock by using another application on the mobile phone, for example, setting a reminder alarm clock when setting a schedule by using a calendar application. Alternatively, the user may set the alarm clock in a manner of VUI by using a voice assistant of the mobile phone. A specific operation of setting the alarm clock by the user is not limited in embodiments of this application.

S3002: The first device determines the alarm start information based on the alarm clock set by the user.

The alarm start information includes one or more of the alarm start moment, the alarm clock name, and the ringtone. Optionally, the alarm start information may further include other content, for example, information such as whether to ring repeatedly, whether to avoid legal holidays, the ringing duration, whether to re-ring, and the re-ringing interval.

S3003: The first device reports the alarm start information to a hub device.

The hub device may be, for example, the hub device 200 in (a) in FIG. 1, and is configured to centrally manage all devices in a home network. In some examples, a device that is powered on for a long time and has a specific storage capability and computing capability may be selected as the hub device in the home network, for example, a router, a mobile phone, a smart speaker, or a set-top box. In some other embodiments, the hub device may alternatively be the first device or a second device. For example, the first device or the second device is equipped with the hub apparatus, and the hub apparatus is configured to centrally manage all devices in a home network. In this case, interaction between the first device and the hub device or interaction between the second device and the hub device may be considered as interaction between different modules in a same device.

In some embodiments of this application, the first device is responsible for alarm start triggering logic, that is, detecting whether an alarm start condition of the alarm clock is met. The alarm start condition of the alarm clock is, for example, that a current time point is the alarm start moment or is close to the alarm start moment. In this case, after the first device determines the alarm start information, the first device detects whether the alarm start condition of the alarm clock is met. When detecting that the alarm start condition of the alarm clock is met, the first device reports the alarm start information of the alarm clock to the hub device. Then, the hub device is responsible for logical arbitration of the alarm clock. Being responsible for logical arbitration of the alarm clock means determining, according to a preset arbitration rule, which one or more devices in a whole house to perform alarm start, that is, perform an alarm clock task of the alarm clock.

In some other embodiments of this application, the first device is not responsible for alarm start triggering logic, and the hub device is responsible for the alarm start triggering logic. In this case, after the first device determines the alarm start information, the first device sends the alarm start information of the alarm clock to the hub device. Then, the hub device detects whether the alarm start condition of the alarm clock is met. When detecting that the alarm start condition of the alarm clock is met, the hub device determines, according to a preset arbitration rule, which one or more devices in a whole house to perform alarm start, that is, perform an alarm clock task of the alarm clock.

S3004: The hub device obtains position information of the user based on a whole-house sensing system, and determines the second device based on the position information of the user.

The whole-house sensing system includes the first electronic device 100 in (a) in FIG. 1, and is configured to locate the user and/or obtain a position of each device in the whole house, and the like. As described above, some first electronic devices 100 each are equipped with a UWB module, and may detect, by using the UWB module, information such as a position of a device in each room in the whole house, an orientation and a distance between devices, a display area of a display device (for example, a large-screen device), and an audio playing area of an audio playing device (for example, a smart speaker). Some first electronic devices 100 each are equipped with a millimeter-wave radar, and may further detect, by using the millimeter-wave radar, a position of a human body in any room in the whole house, a movement track of the human body in the whole house, a distance and an orientation between the human body and the device, and the like.

In some embodiments of this application, the hub device may determine, based on a human body position obtained at the alarm start moment based on the whole-house sensing system, the second device to perform the alarm clock task. In an example, the hub device may select a device, as the second device, that is closest to the human body. In other words, after obtaining the human body position obtained at the alarm start moment based on the whole-house sensing system, the hub device may select and determine, as the second device, a device that is closest to the position.

Optionally, in some other examples, the hub device may select devices that are close to the user, and determine a device as the second device based on a priority sequence of the devices, to execute the alarm clock task. Specifically, after obtaining the human body position obtained at the alarm start moment, the hub device obtains devices that are within a preset distance range (for example, within a distance range of 3 meters) from the position, and selects and determines, based on a priority sequence of the devices, a device with a high priority as the second device. A priority sequence of the smart devices may be determined, for example, based on types of the smart devices or interaction manners of the smart devices, for example, priorities of the smart devices in descending order are as follows: the large-screen device (which may alarm and display a text prompt)>the smart speaker (which have a good ringtone effect)>the tablet computer. When determining that the large-screen device, the smart speaker, and the tablet computer are all located within the preset distance range from the human body position, the hub device preferably selects the large-screen device to perform the alarm clock task, that is, preferably determines the large-screen device as the second device. When both the smart speaker and the tablet computer are located within the preset distance range from the human body position, the smart device is preferably selected to perform the alarm clock task. To be specific, the smart device is preferably determined as the second device. For another example, if it is determined, based on the whole-house sensing system, that the human body position obtained at the alarm start moment is located in the display area of the large-screen device, the large-screen device is preferably selected to perform the alarm clock task. To be specific, the large-screen device is preferably determined as the second device.

S3005: When the current time point is the alarm start moment, the hub device indicates the second device to perform the alarm clock task.

As described in the foregoing step S3003, in some embodiments of this application, the first device is responsible for alarm start triggering logic, that is, detecting whether an alarm start condition of the alarm clock is met. The alarm start condition of the alarm clock is, for example, that the current time point is the alarm start moment or is close to the alarm start moment. In addition, when detecting that the alarm start condition of the alarm clock is met, the first device reports the alarm start information of the alarm clock to the hub device. In other words, when the hub device receives the alarm start information sent by the first device, the current time point is already the alarm start moment or is close to the alarm start moment. In this case, after determining the second device (that is, performing step S3004), the hub device directly indicates the second device to perform the alarm clock task.

In some other embodiments of this application, the first device is not responsible for the alarm start triggering logic, and the hub device is responsible for the alarm start triggering logic. In this case, when detecting that the alarm start condition of the alarm clock is met, for example, after the current time point is the alarm start moment or is close to the alarm start moment, the hub device indicates the second device to perform the alarm clock task.

S3006: The second device performs the alarm clock task.

For example, the second device specifically performs an alarm of the alarm clock. Optionally, a text prompt of the alarm clock may be further displayed. For example, the alarm clock name or a title of the schedule is displayed.

S3007: When preset duration 1 after the alarm start moment expires, the hub device indicates the second device to end the alarm clock task.

For example, after indicating the second device to perform the alarm clock task, the hub device starts a timer. When timing duration of the timer is the preset duration 1 (which is, for example, the ringing duration of the alarm clock), the hub device indicates the second device to end the alarm clock task.

S3008: The second device ends the alarm clock task.

Optionally, in some examples, after indicating the second device to end the alarm clock task, the hub device may also send execution information of the alarm clock to the first device, so that the first device updates the execution information of the alarm clock.

The following describes a specific application scenario.

For example, the user sets a wake-up alarm (for example, an alarm clock at 7:00 AM) on the mobile phone (namely, the first device). Usually, the user sleeps in a bedroom and places the mobile phone in a living room. In this case, at 7:00 AM the next day, the router (namely, the hub device) learns, based on the whole-house sensing system, that the user is in the bedroom, and indicates a smart speaker (namely, the second device) in the bedroom to perform the wake-up alarm. On some overtime days, the user sleeps in a study. At 7:00 AM the next day, the router (namely, the hub device) learns, based on the whole-house sensing system, that the user is in the study, and indicates a smart speaker (namely, the second device) in the study to perform the wake-up alarm.

It can be learned that, for a same alarm clock set by the user, if positions of the user are different every day, the hub device can indicate, based on a current position, a different device to perform the alarm clock task, to provide a short-distance reminder for the user. In addition, the user does not need to be required to operate/wear a specific device, to bring immersive alarm clock experience to the user.

In addition, the user may quickly and conveniently create a new alarm clock or modify an existing alarm clock by using a mobile phone that is carried with the user. The mobile phone may automatically synchronize, to the hub device (for example, a router), information about the alarm clock, that is created by the user or the alarm clock that is modified by the user. The hub device indicates, based on alarm start information of the alarm clock, another device (for example, the smart speaker or the large-screen device) to specifically perform an alarm clock task. It can be learned that the user does not need to repeatedly set/modify the alarm start information of the alarm clock on a plurality of devices, to simplify an operation of the user.

It should be further noted that, in this embodiment of this application, a device (for example, the second device) that is most suitable for performing the alarm clock task is dynamically selected in a whole-house scenario based on the position of the user, to provide a close-in reminder for the user. It may be understood that, if one or some devices are offline in the whole-house scenario, the hub device may alternatively select another online device to perform the alarm clock task, to avoid a case in which the alarm clock task cannot be performed because an individual device is offline.

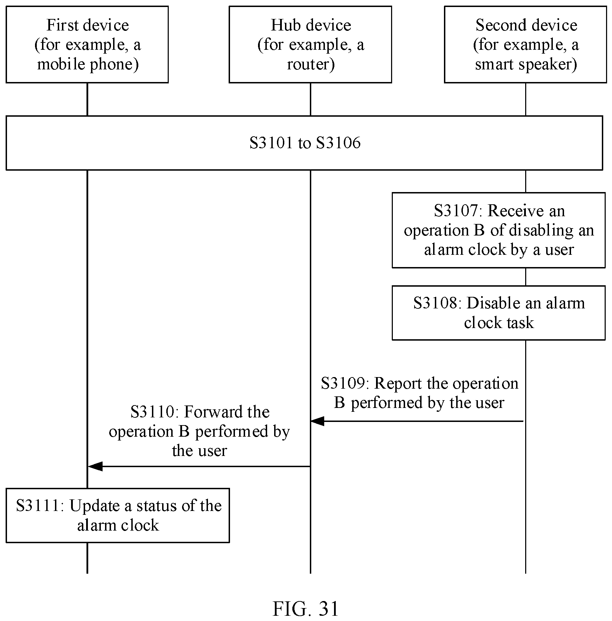

In some other embodiments of this application, when the second device performs the alarm clock task, the user may alternatively disable, by using the second device, the current alarm clock task or indicate to send a reminder later. To be specific, step S3007 and step S3008 that are shown in FIG. 30 are replaced with step S3107 to step S3111 that are shown in FIG. 31.

FIG. 31 is a schematic flowchart of another method for implementing a distributed alarm clock according to an embodiment of this application. The procedure includes the following steps.

S3101 to S3106: For these steps, correspondingly refer to the descriptions of the related content in steps S3001 to S3006 in FIG. 30. Details are not described herein again.

S3107: The second device receives an operation B of disabling the alarm clock by the user.

For example, when duration within which the second device performs the alarm clock task does not reach preset duration 1 (for example, the ringing duration of the alarm clock), the second device receives the operation B of disabling the alarm clock by the user. The operation B is, for example, inputting a voice command (for example, "disabling the alarm clock"), or selecting, on an interface displayed by the second device, a control for disabling the alarm clock.

S3108: The second device ends the alarm clock task.

Specifically, the second device stops ringing, disables text prompt information of the alarm clock, and the like.

S3109: The second device reports, to the hub device, the operation B performed by the user.

S3110: The hub device forwards, to the first device, the operation B performed by the user.

As described in the foregoing step S3003, in some embodiments of this application, the first device is responsible for the alarm start triggering logic, and the hub device is not responsible for the alarm start triggering logic. In this case, the hub device forwards, to the first device, the operation B performed by the user, so that the first device updates the status of the alarm clock.

In some other embodiments of this application, the first device is not responsible for the alarm start triggering logic, and the hub device is responsible for the alarm start triggering logic. In this case, the hub device updates a status of the alarm clock according to the operation B performed by the user. Optionally, the hub device may not forward, to the first device, the operation B performed by the user, that is, step S3111 is not performed.

S3111: The first device updates the status of the alarm clock.

In some other embodiments, after the second device receives the operation B of disabling the alarm clock by the user (that is, performs step S3107), the second device cannot immediately disable an alarm clock task. The second device reports, to the hub device, the operation B performed by the user (that is, performs step S3109). The hub device delivers, to the second device, an instruction for disabling the alarm clock task. The second device disables the alarm clock task after receiving the instruction for disabling the alarm clock task sent by the hub device.

It can be learned from the foregoing descriptions that the user can directly disable the alarm clock on the device (namely, the second device) that executes the alarm clock task. The device that performs the alarm clock task is located near the user, and therefore this helps the user quickly and conveniently disable the alarm clock, thereby not only reminding the user but also avoiding excessively disturbing the user. In other words, the distributed alarm clock can effectively balance a function of reminding the user and do-not-disturb for the user, to improve experience of using the alarm clock by the user. In addition, the operation of disabling the alarm clock by the user is also reported to the hub device, or reported to the first device by using the hub device, so that the hub device or the first device updates the status of the alarm clock in real time.

In still some embodiments of this application, the user may further directly indicate, on the device (namely, the second device) that executes the alarm clock task, to re-ring later for the alarm clock. In this case, the second device disables the current alarm clock task, and reports the operation of the user to the hub device, or reports the operation to the first device by using the hub device. The hub device or the first device updates the status of the alarm clock. When a moment for re-ringing for the alarm clock arrives, the hub device again determines, based on position information of the user provided by the whole-house sensing system, a new device suitable for performing an alarm clock re-ringing task, for example, a third device. The third device performs the re-ringing task.

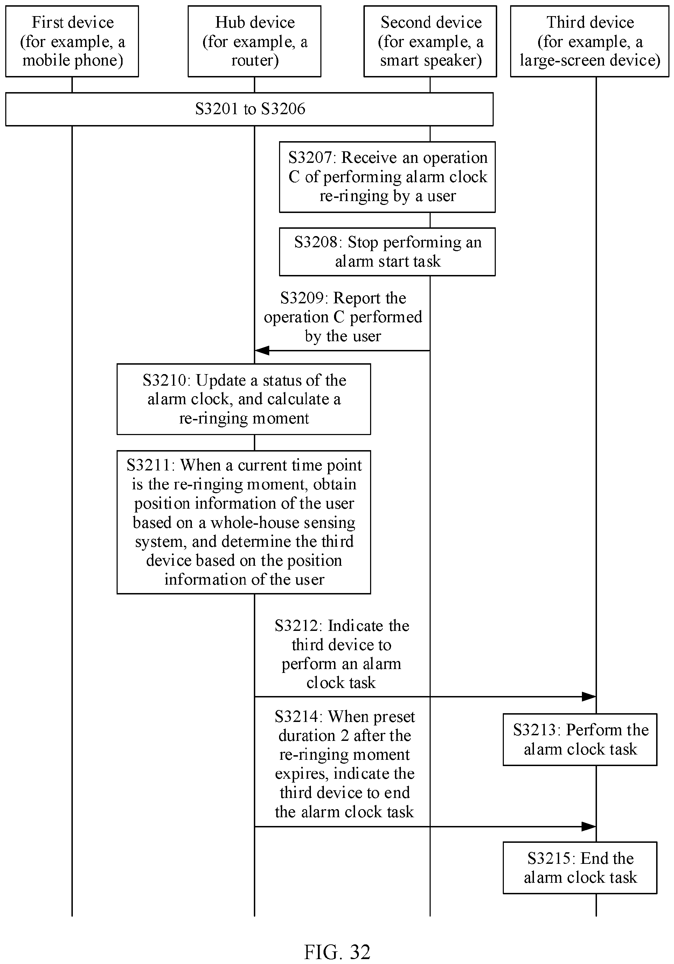

FIG. 32 is a schematic flowchart of another method for implementing a distributed alarm clock according to an embodiment of this application. The procedure includes the following steps.

S3201 to S3206: For these steps, correspondingly refer to the descriptions of the related content in steps S3001 to S3006 in FIG. 30. Details are not described herein again.

S3207: The second device receives an operation C of performing alarm clock re-ringing by the user.

For example, when duration within which the second device performs the alarm clock task does not reach preset duration 1 (for example, the ringing duration of the alarm clock), the second device receives the operation C of performing alarm clock re-ringing by the user. The operation C is, for example, inputting a voice command (for example, "re-ringing after 5 minutes"), or selecting, on an interface displayed by the second device, a control for alarm clock re-ringing.

S3208: The second device stops performing an alarm start task.

S3209: The second device reports, to the hub device, the operation C performed by the user.

S3210: The hub device updates a status of the alarm clock based on the operation C performed by the user, and calculates the re-ringing moment of the alarm clock.

The re-ringing moment is the current time point plus preset duration 2. If the user indicates re-ringing interval duration (for example, 5 minutes), the re-ringing interval duration indicated by the user is the preset duration 2. If the user indicates no re-ringing interval duration, the hub device may use a default value (for example, 10 minutes or 15 minutes) of the preset duration 2.

S3211: When a current time point is the re-ringing moment, the hub device obtains position information of the user based on the whole-house sensing system, and determines a third device based on the position information of the user.

The hub device obtains the current position information of the user based on the whole-house sensing system, and determines the third device (for example, the large-screen device) based on the current position of the user. A specific method for determining the third device is similar to the method for determining the second device in step S3004 in FIG. 30. Details are not described herein again.

S3212: The hub device indicates the third device to perform the alarm start task.

S3213: The third device performs the alarm start task.

S3214: When the preset duration 2 after the re-ringing moment expires, the hub device indicates the third device to end the alarm clock task.

S3215: The third device ends the alarm clock task.

For content of step S3212 to step S3215, refer to the related content of step S3005 to step S3008 in FIG. 30. Details are not described herein again.

In some other examples of this application, after receiving the operation C of performing alarm clock re-ringing by the user reported by the second device (that is, step S3207 is performed), the hub device may directly forward, to the first device, the operation C performed by the user. Then, the first device updates a status of the alarm clock based on the operation C performed by the user, and calculates the re-ringing moment. When the re-ringing moment arrives, the re-rigging moment is sent to the hub device. Then, the hub device obtains the current position information of the user based on the whole-house sensing system, determines the third device, and then indicates the third device to perform the alarm clock task.

The following describes a specific application scenario.

For example, the user sets a wake-up alarm (for example, an alarm clock at 7:00 AM) on the mobile phone (namely, the first device). At 7:00 AM the next day, the router (namely, the hub device) learns, based on the whole-house sensing system, that the user is in a bedroom, and indicates a smart speaker (namely, the second device) in the bedroom to perform the wake-up alarm. The user selects re-ringing after 10 minutes, the second device disables an alarm. The user may wash up, gargle, prepare breakfast, and the like. At 7:10 AM, the router (namely, the hub device) learns, based on the whole-house sensing system, that the user is currently in a living room, and indicates a smart speaker (namely, the third device) in the living room to perform the wake-up alarm. In this case, the user may know that 10 minutes currently passed away, and may appropriately arrange time of the user.

It can be learned that, when an alarm clock task needs to be performed a plurality of times for a same alarm clock, according to a solution of the distributed alarm clock provided in this embodiment of this application, different devices are intelligently switched, based on a change of a position of the user, to perform the alarm clock task each time, so as to provide a short-distance reminder for the user at any time. In addition, in a process of switching the devices to perform the alarm clock task, the user does not need to be required to operate/wear a specific device, to bring immersive alarm clock experience to the user.

In still some embodiments of this embodiment of this application, when an alarm clock task is performed once for a same alarm clock, different devices may also be switched to perform the current alarm clock task.

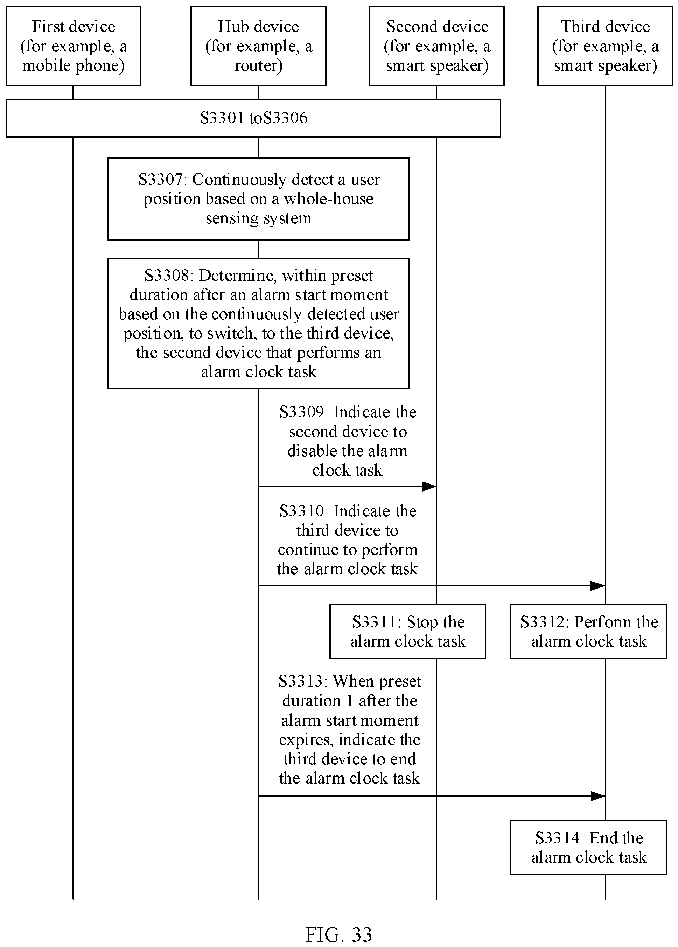

FIG. 33 is a schematic flowchart of still another method for implementing a distributed alarm clock according to an embodiment of this application. The procedure includes the following steps.

S3301 to S3306: For these steps, correspondingly refer to the descriptions of the related content in steps S3001 to S3006 in FIG. 30. Details are not described herein again.

S3307: The hub device continuously detects the user position based on the whole-house sensing system.

In some examples, after the hub device indicates the second device to perform the alarm clock task, the hub device continuously detects the user position based on the whole-house sensing system, so that the hub device determines whether the device that performs the alarm clock task needs to be switched. In some other examples, when determining that the second device executes the alarm clock task, the hub device may also learn a movement status (the stationary state or a moving state) of the user based on the whole-house sensing system. If the user is in the stationary state or a low-speed moving state (a moving speed is less than a threshold), the hub device may determine that the second device independently performs the alarm clock task without continuously detecting the user position. If a moving speed of the user is greater than or equal to a threshold, the hub device continuously detects the user position, so that the hub device determines whether the device that performs the alarm clock task needs to be switched.

S3308: The hub device determines, within preset duration 1 after the alarm start moment based on the detected user position, to switch, to the third device, the second device that performs the alarm clock task.

Within the preset duration 1 after the alarm start moment, the hub device determines, based on a real-time position of the user, a device that meets a requirement of performing the alarm clock task. For a specific determining method, refer to the related content in S3004 in FIG. 30. Details are not described herein again. When the device that meets the requirement of performing the alarm clock task changes, for example, changes to the third device, the hub device performs step S3309.

S3309: The hub device indicates the second device to disable the alarm clock task.

S3310: The hub device indicates the third device to continue to perform the alarm clock task.

S3311: After receiving the instruction of the hub device for disabling the alarm clock task, the second device stops the alarm clock task.

Step S3311 may be performed after or before step S3310, or may be simultaneously performed with step S3310.

S3312: The third device performs the alarm clock task.

S3313: When the preset duration 1 after the alarm start moment expires, the hub device indicates the third device to end the alarm clock task.

S3314: The third device ends the alarm clock task.

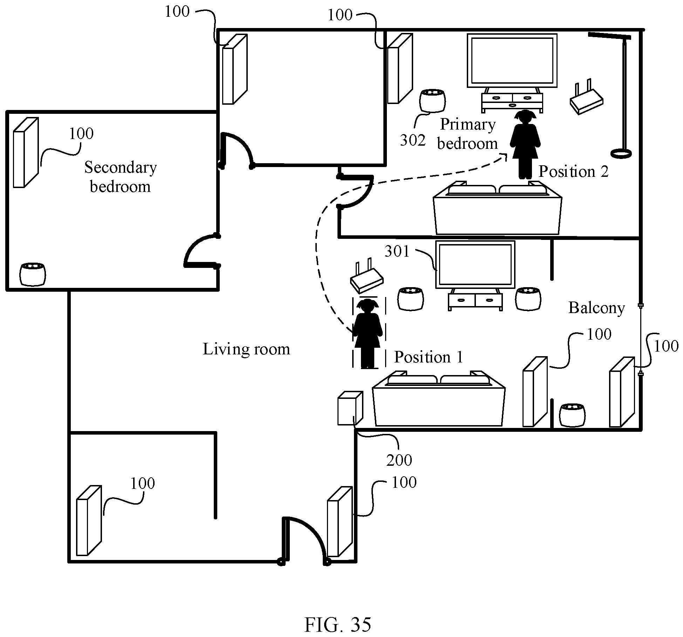

For example, as shown in FIG. 35, when the hub device obtains, based on the whole-house sensing system, that the human body is located in a position 1 of a living room at the alarm start moment, a device closest to the human body is a large-screen device 301. In this case, the hub device may select the large-screen device 301 to perform the alarm clock task. Further, if the hub device further determines that the human body is in the moving state, the hub device continuously detects the human body position. When detecting that the human body moves to a position 2, the hub device detects, based on the whole-house sensing system, that the device that is closest to the human body at this time has changed from the large-screen device 301 in the living room to the smart speaker 302 in a primary bedroom. In this case, the hub device switches the alarm clock task of the alarm clock to the smart speaker 302 for performing.

It can be learned that, in a process of switching the alarm clock, no operation of the user is required, and the hub device intelligently switches different devices, based on a movement track of the human body, to continue the alarm clock task, to bring immersive alarm clock experience to the user.

In still some embodiments of this application, in a scenario in which the whole-house scenario includes a plurality of users, when different users set alarm clocks, the hub device may add user tags to the different alarm clocks.

Specifically, the hub device may maintain identity information table of each user based on the identity information of the user collected by each device in the whole-house scenario. The identity information of the user includes but is not limited to information (for example, a MAC address of a private device held by the user, an IMEI of the device, and an account logged in to the device) of the private device (where the private device is usually used by one user, for example, a mobile phone or a smartwatch) and a biometric feature (for example, a voiceprint feature, a facial feature, or a body feature) of the user collected by the private device or a public device (where, the public device is usually used by two or more users, for example, a large-screen device, a smart speaker, a doorbell, a camera, or a millimeter-wave radar). Then, when receiving alarm clock information reported by another device, the hub device further determines, based on the maintained identity information table of the user, user information corresponding to the alarm clock information, that is, establishes a correspondence between an alarm clock and a user, that is, adds a user tag to each alarm clock.

For example, when a user 1 sets an alarm clock 1 by using a mobile phone of the user 1, and the mobile phone of the user 1 uploads alarm start information of the alarm clock 1 to the hub device, the hub device may determine an identity of the user 1 based on information (for example, a MAC address of the mobile phone, an IMEI of the mobile phone, a mobile number of the mobile phone, or an account logged in to the mobile phone) of the mobile phone and the identity information table of the user maintained by the hub device, and establish a correspondence between the alarm clock 1 and the user 1. For another example, when a user 2 sets an alarm clock 2 by using the smart speaker, and the smart speaker uploads alarm start information of the alarm clock 2 to the hub device, the hub device may determine an identity of the user 2 based on a voiceprint feature of the user 2 collected by the smart speaker and the identity information table of the user maintained by the hub device, and establish a correspondence between the alarm clock 2 and the user 2. For another example, when a user 3 sets an alarm clock 3 by using the large-screen device, and the large-screen device uploads alarm start information of the alarm clock 3 to the hub device, the hub device may determine an identity of the user 3 based on a facial feature of the user 3 collected by the large-screen device and the identity information table of the user maintained by the hub device, and establish a correspondence between the alarm clock 3 and the user 3.

Then, when alarm start of an alarm clock is performed, the hub device may determine, based on the correspondence between the alarm clock and the user, a specific user corresponding to the alarm clock. Then, position information of the specific user is obtained based on the whole-house sensing system, and a device is determined, based on the position information of the specific user, for performing the alarm clock. During specific implementation, the different users may be recognized based on a millimeter-wave radar in the whole-house sensing system. For example, the different users are recognized based on height features and posture features of the different users. Alternatively, facial recognition may be performed based on a human body image collected by a camera in the whole-house sensing system, to recognize a specific user, and then a position of the specific user and the like are measured based on the millimeter-wave radar in the whole-house sensing system. In conclusion, how to recognize the position of the specific user is not specifically limited in embodiments of this application.

**Embodiment 2: Device registration and control**

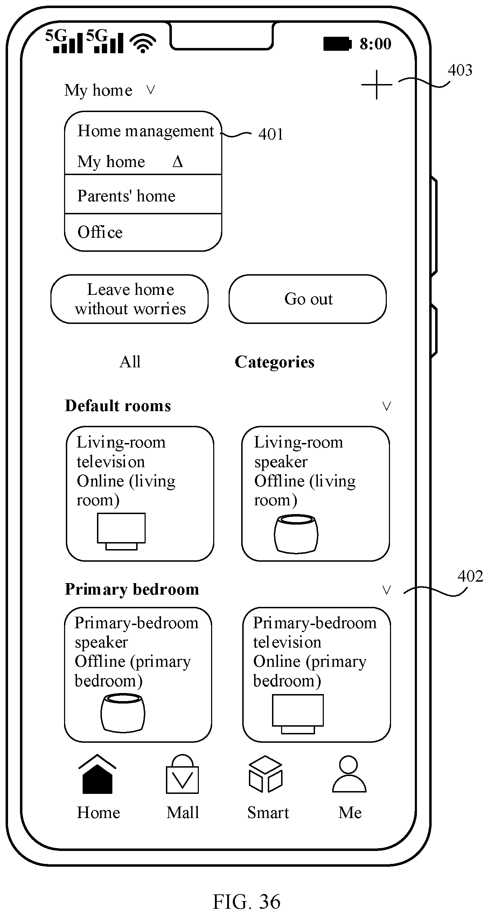

With rapid development of an internet of things, there are more types and quantities of devices disposed in a home environment. Usually, a user may bind a device and control the device by using a smart home application (for example, a smart life application) installed on a control device (for example, a mobile phone). When the user need to manage a plurality of homes, and each home has devices in a plurality of rooms, the user need to manually add attributes of the homes and the rooms for the devices when binding the devices. In this way, when the user subsequently controls a device by using the control device, the user may obtain, through screening, the device in a room based on a home and the room. For example, FIG. 36 shows a main interface of the smart home application. The user may first select a device in a specified home by operating a control 401 on the main interface. Then, the device in the specified room is searched by turning pages up and down, and an expansion control corresponding to the specified room is operated to expand a list of a registered device in the room. For example, an expansion control 402 corresponding to a primary bedroom is operated, and the control device displays a device in the primary bedroom. Finally, the user may select a specific device in the device list to open a control interface of the specific device for manipulation. It can be learned that an operation procedure of registering and controlling the device by the user is very complex.

Therefore, embodiments of this application provide a device registration and control method based on automatically adding home and room information, to obtain, based on a whole-house sensing system, information about a home and a room in which a control device is located, and automatically adding corresponding home and room information for a device that is being registered or automatically obtain, through screening, a device in the home or the room.

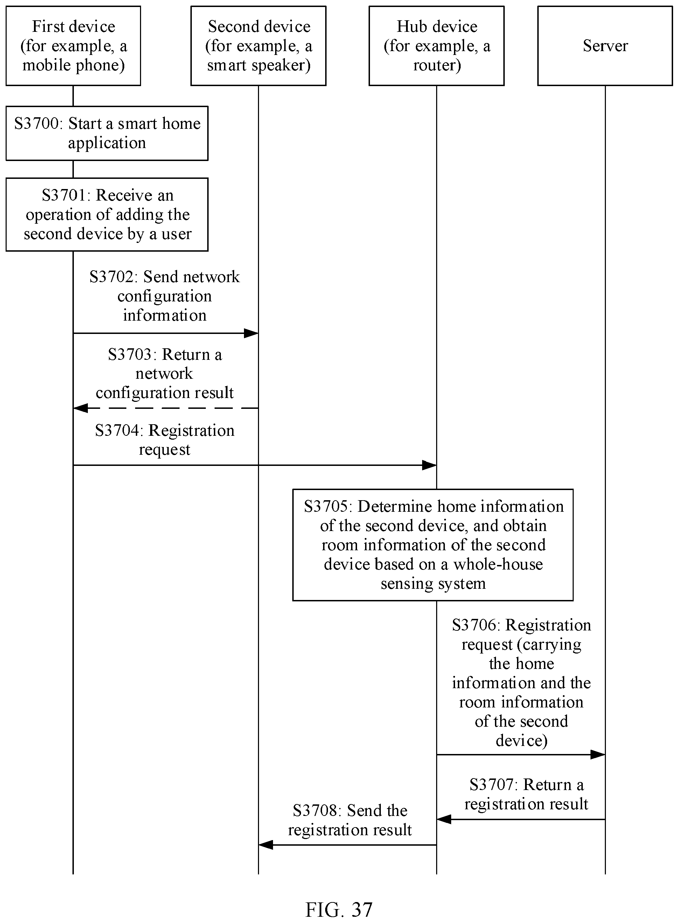

FIG. 37 is a schematic flowchart of a device network configuration and registration method according to an embodiment of this application. The procedure includes the following steps.

S3700: A first device starts a smart home application.

For example, when using the first device to configure a network for a second device (a device that is configured with no network and unregistered), the user may first trigger the second device to enter a network configuration mode (for example, press and hold a power button for 3 seconds). The first device is a control device for a device to be configured with a network, for example, a mobile phone, a tablet computer, or a smartwatch. Then, the user may start the smart home application (for example, an AI life application of Huawei) on the first device. In some examples, the first device accesses an internet by connecting to a home Wi-Fi network. In this case, when the smart home application on the first device includes a plurality of homes, the first device may determine, based on the currently connected Wi-Fi network, a home in which the user is currently located, and automatically display information about a device in the home.

S3701: The first device receives an operation of adding the second device by the user.

For example, FIG. 36 shows a main interface of the smart home application displayed by a first device. The user may start a network configuration and registration procedure of the second device by operating an adding control 403 on the main interface of the smart home application. In response to detecting that the user operating the adding control 403, the first device starts to search a surrounding device. After finding the second device, the first device establishes a connection (for example, a Bluetooth connection or a Wi-Fi connection) to the second device.

S3702: The first device sends a network configuration information to the second device.

For example, the first device sends the network configuration information to the second device by the Bluetooth connection or the Wi-Fi connection established in step S3701. The network configuration information is information about accessing the internet by the first device, and the network configuration information may also be used by the second device to access the internet. The network configuration information includes but is not limited to a service set identifier (service set identifier, SSID) and a password of the home Wi-Fi network, so that the second device is connected to the home Wi-Fi network based on the network configuration information.

S3703: The second device returns a network configuration result to the first device.

For example, after network configuration of the second device succeeds, the network configuration result may be returned to the first device. When network configuration of the second device fails, the network configuration result may alternatively be returned to the first device, so that the first device executes a corresponding policy, for example, retry, based on a cause of a network configuration failure. In the following step, description is provided in a case in which network configuration of the second device succeeds. In addition, it should be noted that this step is optional.

S3704: The first device sends a request to a hub device, to request to register the second device.

The hub device may be, for example, the hub device 200 in (a) in FIG. 1, and is configured to centrally manage all devices in a home network. In some examples, a device that is powered on for a long time and has a specific storage capability and computing capability may be selected as the hub device in the home network, for example, a router, a mobile phone, a smart speaker, or a set-top box. In some other embodiments, the hub device may alternatively be the first device or the second device. For example, the first device or the second device is equipped with the hub apparatus, and the hub apparatus is configured to centrally manage all devices in a home network. In this case, interaction between the first device and the hub device or interaction between the second device and the hub device may be considered as interaction between different modules in a same device.

For example, the first device may send a proxy registration instruction to the hub device, and request, by using the hub device, the server to register the second device.

S3705: The hub device determines home information of the second device, and obtains room information of the second device based on a whole-house sensing system.

It may be understood that, if network configuration and registration are performed on the second device by using the hub device, the second device and the hub device are devices in a same home, and therefore the hub device can determine home information of the hub device as home information of the second device.

The whole-house sensing system includes the first electronic device 100 in (a) in FIG. 1, and is configured to locate the user and/or obtain a position of each device in a whole house, and the like. As described above, some first electronic devices 100 each are equipped with a UWB module, and may detect a position (including information about a room in which each device is located) of the device in each room in the whole house by using the UWB module. Some first electronic devices 100 each are equipped with a millimeter-wave radar, and may further detect, by using the millimeter-wave radar, a position (including information about a room in which a human body is located) of the human body in any room in the whole house.

In some embodiments, the hub device may detect the human body position in the whole house based on the millimeter-wave radar in the whole-house sensing system, determine a position of a user of the first device based on the detected position of the human body, and determine the room information of the second device based on information about a direction in which the user of the first device is located.

In some examples of this embodiment, the hub device detects the human body position in the whole house based on the millimeter-wave radar in the whole-house sensing system, and determines the human body position as the position of the user of the first device. It may be understood that, when the user of the first device performs network configuration and registration on the second device, the user of the first device is usually located near the second device, that is, the user of the first device and the second device are located in a same room. Therefore, the hub device obtains information about a room in which the user of the first device is located, namely, the room information of the second device.

In some other examples of this embodiment, when the hub device detects, based on the whole-house sensing system, that positions of a plurality of human bodies are included in a whole-house scenario, that is, detects a plurality of users, the hub device may alternatively recognize a user of the first device based on a feature of each detected user, obtain information about a room in which the user of the first device is located, and determine the information as the room information of the second device.

For example, the hub device maintains a correspondence between each private device (for example, a mobile phone or a smartwatch) and a user of the private device, and the hub device may further record a feature of each user. The feature of the user includes any one or more of the following: a height feature, an identity attribute (for example, a child, a father, or a mother) corresponding to information about an account logged in to the private device, and a movement track in the room. When the first device is a private device, the hub device may determine a feature (for example, a height feature) of the user of the first device based on the identifier (for example, a MAC address, an IMEI, or login account) of the first device. Then, the hub device detects, based on the millimeter-wave radar in the whole-house sensing system, a human body position that is in the whole house and that is consistent with the feature of the user of the first device, and determines the human body position as the position of the user of the first device.

For another example, as described above, the whole-house sensing system may detect parameters such as an attitude, a breath, a heartbeat, and a height of the user. The user of the first device is operating the first device to perform network configuration for the second device. Therefore, the hub device may exclude a detected sleeping user (it is detected that the user is in a lie-down attitude, and a breath, a heartbeat, and the like conform to a sleep state), and exclude the child (based on the height feature) and the like. Optionally, a user in a specific room may also be selected based on a device type of the device to be configured with a network. For example, when the device to be configured with a network is a doorbell, a user located in a living room may be selected. For example, when the device to be configured with a network is a large screen or a smart speaker, a user in a kitchen can be excluded. In conclusion, a specific method for recognizing the user of the first device is not limited in embodiments of this application.

In some other embodiments, the hub device may detect, based on a UWB system in the whole-house sensing system, information about a room in which the first device is located, and determine the room information of the first device as the room information of the second device.

Specifically, the first device is equipped with a UWB tag. After the first device performs step S3701 and before the hub device performs step S3705, the UWB tag provided for the first device sends a locating request, to request to locate the first device. In this case, when the hub device obtains a position of the first device (including the room information of the first device) based on the whole-house sensing system, the user is usually also located near the second device when the user configures a network for the second device by using the first device, that is, the user, the first device, and the second device are located in a same room. Therefore, the hub device obtains the information about the room in which the first device is located, namely, the room information of the second device.

S3706: The hub device sends a registration request to the server, to request to register the second device.

The registration request carries the home information and the room information of the second device.

S3707: The server returns a registration result to the hub device.

S3708: The hub device sends the registration result to the second device.

It can be learned that, according to the device network configuration and registration method provided in this embodiment of this application, home information and room information can be automatically added to a device on which network configuration is performed, and a user does not need to manually enter the room information and the room information. This simplifies an operation procedure of the user, and shortens operation duration of the user, thereby improving network configuration efficiency of the device, and improving use experience of the device.

In addition, the home information and the room information are automatically added to the device on which network configuration is performed. This can also avoid a case in which the user adds an incorrect home or room because the user maintains devices in a plurality of homes or the user maintains a large quantity of devices, thereby improving accuracy of adding the home information and the room information to the device.

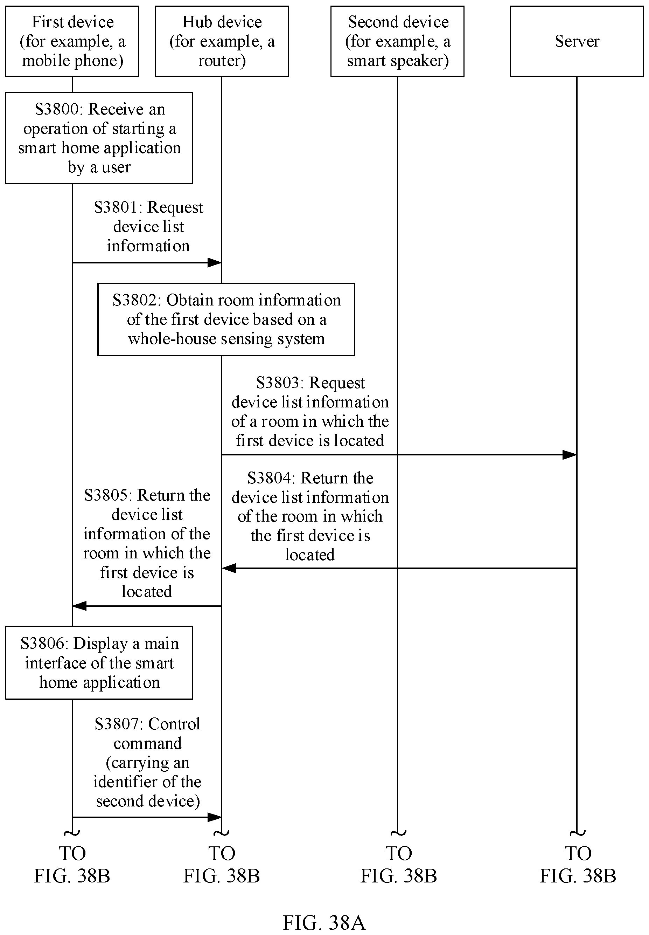

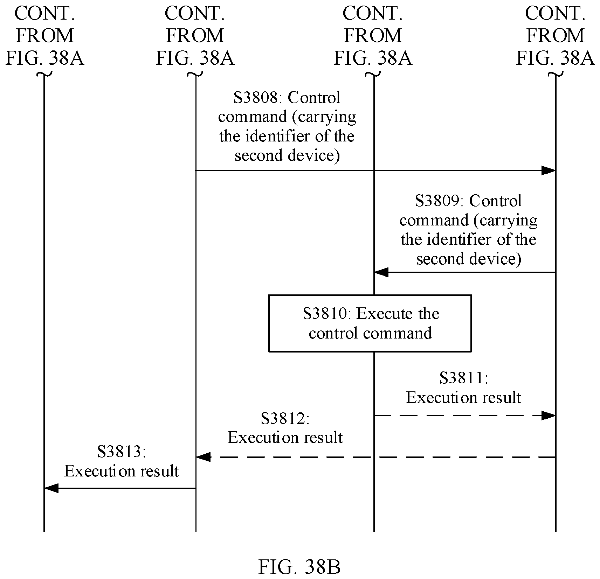

FIG. 38A and FIG. 38B are a schematic flowchart of a device control method according to an embodiment of this application. The procedure includes the following steps.

S3800: A first device receives an operation of starting a smart home application by a user.

For example, the user may implement manipulation such as control and management for a device in a home by using the smart home application (for example, an AI life application) installed on the first device.

S3801: The first device requests device list information from a hub device.

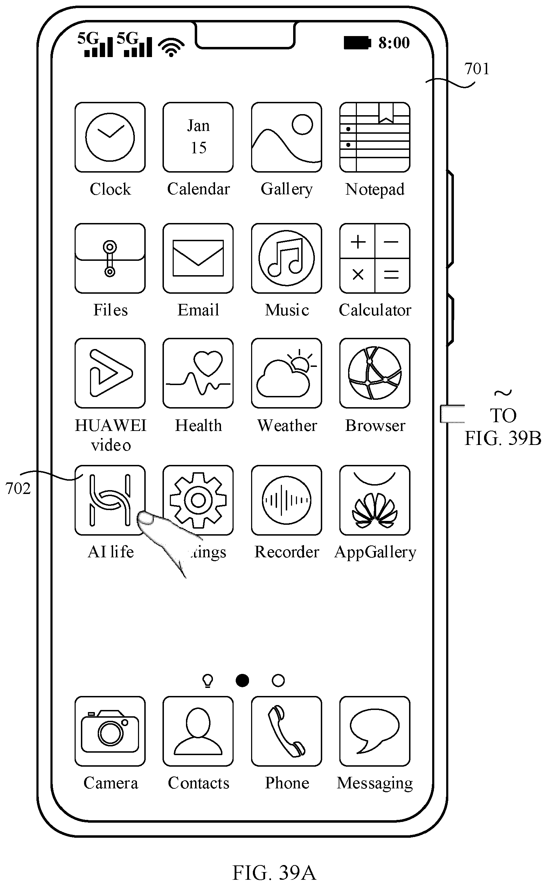

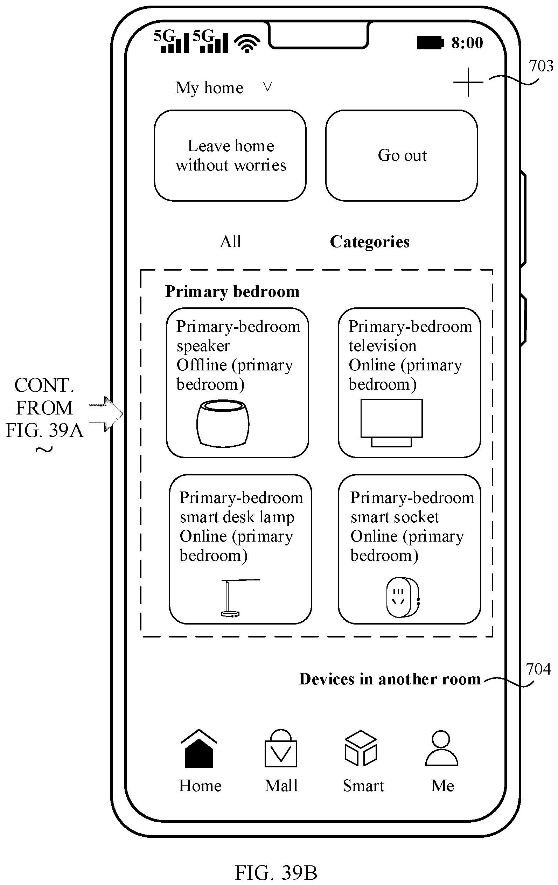

For example, as shown in FIG. 39A and FIG. 39B, in response to detecting that the user taps an application icon 702 of "AI life" on a desktop 701 of the first device, the first device requests the device list information from the hub device.

S3 802: The hub device obtains room information of the first device based on a whole-house sensing system.

In some embodiments, the hub device detects, based on a millimeter-wave radar of the whole-house notification system, a position of a human body in a whole house, and determines, based on the detected human body position, a position of the user of the first device, namely, a position (including the room information of the first device) of the first device. For a specific detection method, refer to the descriptions of the related content in step S3705. Details are not described herein again.

In some other embodiments, the hub device may detect a position (including information about a room in which the first device is located) of the first device based on a UWB system in the whole-house sensing system. For a specific detection method, refer to the descriptions of the related content in step S3705. Details are not described herein again.

S3 803: The hub device requests, from a server, device list information of a room in which the first device is located.

S3804: The server returns, to the hub device, the device list information of the room in which the first device is located.

S3805: The hub device returns, to the first device, the device list information of the room in which the first device is located.

S3806: The first device displays a main interface of the smart home application, where the main interface includes the device list information of the room in which the first device is located.

For example, as shown in FIG. 39A and FIG. 39B, the first device displays a main interface 703 of the smart home application based on the received device list information of the room in which the first device is located. It may be noted that the main interface 703 of the smart home application displays only a list of devices in the room (for example, a primary bedroom) in which the first device is located. In this case, the user may directly manipulate another device in the room in which the first device is located. Certainly, if the user may also view a device in another room by using a "devices in another room" control 704 on the main interface 703 of the smart home application, and may perform manipulation and the like on a device in the another room.

S3 807: The first device receives a control operation performed by the user on a second device, and sends a control command to the hub device. The control command carries an identifier of the second device and an operation instruction.

S3 808: The hub device forwards the control command to the server.

S3809: The server forwards the control command to the second device.

S3810: The second device executes the control command.

S3811: The second device returns an execution result to the server.

This step is optional.

S3812: The server returns the execution result to the hub device.

This step is optional.

S3813: The hub device returns the execution result to the first device.

This step is optional.

It should be noted that, in step S3807 to step S3813, an example in which the first device sends the control command to the second device by using the server is used for description. In some other embodiments, the first device may alternatively directly send the control command to the second device. This is not specifically limited herein.

Compared with that in the conventional technology, on the main interface of the smart home application, a device list of a default room is displayed, or device lists of all rooms are displayed. A quantity of displayed devices is large, and the user further needs to select a corresponding room, and then can obtain, through screening, a device that the user wants to manipulate. However, in this embodiment of this application, the hub device automatically obtains, through screening based on the whole-house sensing system, the device in the room in which the first device (namely, a control device) is located, and therefore a procedure of manually selecting the room by the user does not need to be performed. It may be understood that, when the user wants to operate a device, the user and the device are usually located in a same room. Therefore, in this embodiment of this application, the first device automatically obtains, through screening, the device in the room in which the first device is located. This satisfies an operation intention of the user, simplifies an operation procedure of the user, and improves user experience.

**Embodiment 3: Doorbell reminder or incoming call notification**

A smart doorbell may be connected to a home network in a wireless manner, for example, Bluetooth or Wi-Fi. When a guest triggers the doorbell, the smart doorbell may trigger a smart speaker or a device with a screen in the home network to send a reminder. Similarly, when there is an incoming call (audio call or video call), the smart speaker or the device with a screen in the home network is triggered to send a notification. In this embodiment of this application, an appropriate device may be intelligently selected based on a detected human body position and position of each smart device and based on a whole-house sensing system, to send the reminder, so as to prevent the user from missing the reminder, and improve user experience.

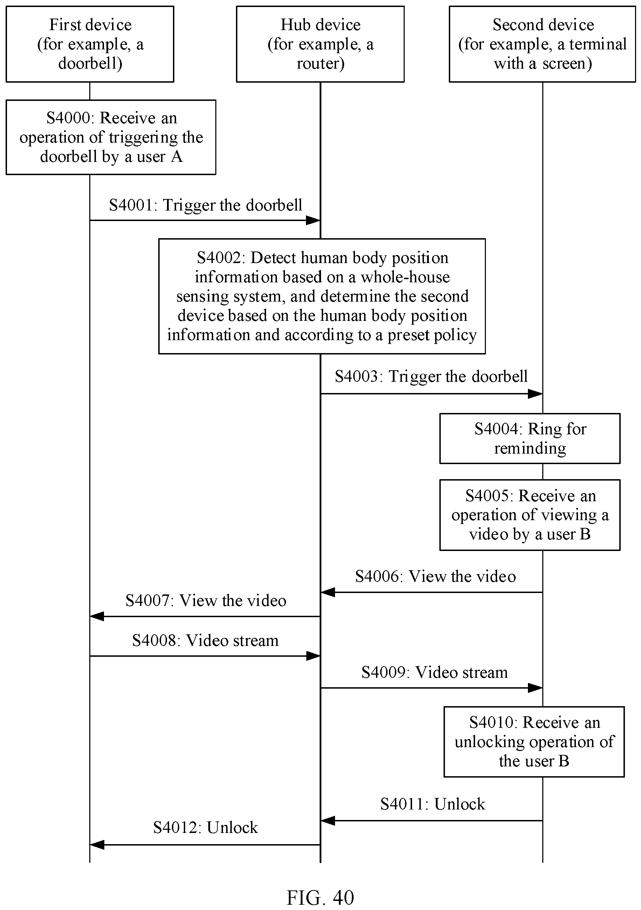

FIG. 40 is a schematic flowchart of a reminding method of a smart doorbell according to an embodiment of this application. The procedure includes the following steps.

S4000: A first device receives an operation of triggering the doorbell by a user A.

For example, the first device is the smart doorbell. The user A (for example, a guest) presses a doorbell button on the smart doorbell, or taps a control corresponding to the doorbell.

S4001: The first device sends a doorbell triggering message to a hub device.

For example, the hub device may be, for example, the hub device 200 in (a) in FIG. 1, and is configured to centrally manage all devices in the home network. In some examples, a device that is powered on for a long time and has a specific storage capability and computing capability may be selected as the hub device in the home network, for example, a router, a mobile phone, a smart speaker, or a set-top box. The first device accesses the home network by using the hub device. In this case, the first device sends, to the hub device, a message for triggering a doorbell reminder.

In some other embodiments, the hub device may alternatively be the first device or the second device. For example, the first device or the second device is equipped with the hub apparatus, and the hub apparatus is configured to centrally manage all devices in a home network. In this case, interaction between the first device and the hub device or interaction between the second device and the hub device may be considered as interaction between different modules in a same device.

S4002: The hub device detects a human body position based on a whole-house sensing system, and determines the second device based on the detected human body position and according to a preset policy.

The preset policy includes but is not limited to: preferably selecting, as the second device, a device that is closest to a human body; when a plurality of human bodies are detected, preferably selecting, as the second device, a device that is closest to the plurality of human bodies; preferably selecting, as the second device, a device that is in a room (for example, a living room) that is closest to the first device and in which there is a person; when there is a person resting in the plurality of detected human bodies, avoiding a device, that is near the resting person, when the second device is selected; and the like. It should be noted that specific content of the preset policy is not limited in embodiments of this application. In addition, the preset policy may be a system default policy, or may be set by the user.

For example, the whole-house sensing system includes the first electronic device 100 in (a) in FIG. 1, and is configured to locate the user and/or obtain a position of each device in a whole house, and the like. As described above, some first electronic devices 100 each are equipped with a UWB module, and may detect, by using the UWB module, information such as a position of a device in each room in the whole house, an orientation and a distance between devices, a display area of a display device (for example, a large-screen device), and an audio playing area of an audio playing device (for example, a smart speaker). Some first electronic devices 100 each are equipped with a millimeter-wave radar, and may further detect, by using the millimeter-wave radar, a position of a human body in any room in the whole house, a movement track of the human body in the whole house, a distance and an orientation between the human body and the device, and the like.

In some embodiments, after receiving the doorbell triggering message sent by the first device, the hub device detects the human body position in the whole house based on the whole-house sensing system, and determines, as the second device, a display device (for example, a large-screen device or a speaker with a screen) or an audio playing device (for example, a smart speaker) that is near the detected human body position. In this way, when the display device or the audio playing device that is located near the human body position (namely, a user position) is selected to ring, the user is reminded in time, and the user performs processing in time.

In some other embodiments, when detecting, based on the whole-house sensing system, that the whole house includes the plurality of human bodies, the hub device may obtain, as the second device through calculation, a display device or an audio playing device that is closer to all the plurality of human bodies. In this way, the plurality of users are conveniently reminded, so that an idle user in the plurality of users performs processing in time.

In still some embodiments, when detecting, based on the whole-house sensing system, that the whole house includes the plurality of human bodies, the hub device may further analyze a feature of each human body. For example, when it is determined, based on an attitude, a breath, and a heart rate of the human body, that a user is in a sleep state, a device that is near the user may be excluded when the second device is selected. For example, a device in a room in which the user is located is excluded. For another example, a device in a room in which an adult is located is preferably selected as the second device based on a height feature of the human body. For another example, the device in the room (for example, the living room) closest to the first device and in which there is a person is preferably selected as the second device based on a room in which the human body is located.

S4003: The hub device forwards the doorbell triggering message to the second device.

S4004: The second device rings for reminding.

S4005: The second device receives an operation of viewing a video by a user B.

In some embodiments, the second device is a device with a screen, and the first device is equipped with a camera. The camera may be configured to collect a guest scene outside a door. In this case, after the second device rings, the user B may also view, on the second device, an image collected by the camera of the first device.

Certainly, in some other embodiments, the second device may also automatically request the video from the first device when ringing (that is, performing step S4004). In other words, step S4006 is directly performed, without performing step S4005.

S4006: The second device sends a video viewing message to the hub device.

S4007: The hub device forwards the video viewing message to the first device.

S4008: The first device sends a collected video stream to the hub device.

S4009: The hub device forwards the collected video stream to the second device.

It should be noted that, in step S4006 to step S4009, an example in which the first device interacts with the second device by using the hub device is used for description. In some other examples, the first device may directly interact with the second device.

In addition, step S4005 to step S4009 are optional steps. In some examples, when the second device cannot play the video when being equipped with no screen.

S4010: The second device receives an unlocking operation of the user B.

In some embodiments, the user B may control, by using the second device, the first device to unlock in a manner of a GUI or a manner of VUI.

S4011: The second device sends an unlocking message to the hub device.

S4012: The hub device forwards the unlocking message to the first device.

In conclusion, compared with that in the conventional technology, the first device (for example, the smart doorbell) establishes a communication connection to a specific device (for example, the large-screen device or the smart speaker) in the home network. When the user triggers the doorbell on the first device, the specific device may collaboratively ring to remind the user. However, in this case, if the user is far away from the specific device, the user may miss the reminder, or the user needs to quickly move near the specific device to process the reminder, resulting in poor user experience. In addition, collaborative ringing of the smart doorbell and the specific device is limited to the communication connection between the smart doorbell and the specific device. When the communication connection between the smart doorbell and the specific device is broken or a signal is poor, collaboration between the smart doorbell and the specific device is affected.

However, in this embodiment of this application, when the user triggers the doorbell on the first device, the hub device detects the human body position based on the whole-house sensing system, and then selects an appropriate device, for example, the second device, based on the detected human body position, to collaborate with the first device. It can be learned that, in one aspect, the second device is selected based on the detected human body position, and therefore there is a user located near the second device. In this case, the user can receive the ringing reminder in time, which also facilitates processing by the user. In another aspect, the hub device selects the second device from all the devices in the whole house. Therefore, when a device in the whole house is disconnected from the hub device or the signal is poor, the hub device may select another device to collaborate with the first device.

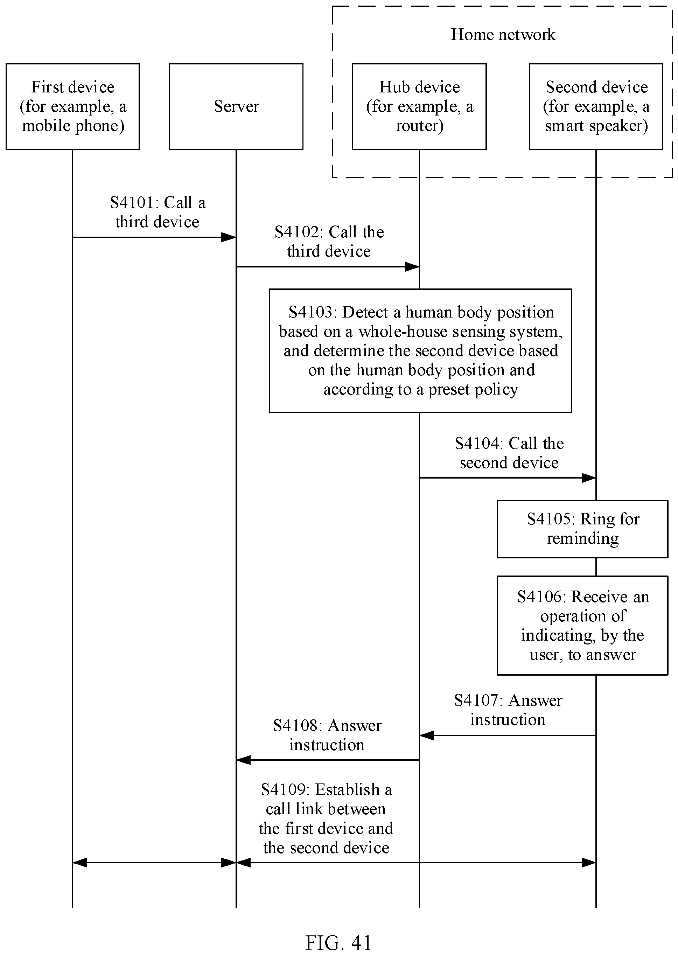

FIG. 41 is a schematic flowchart of an incoming call notification method according to an embodiment of this application. The procedure includes the following steps.

S4101: A first device sends, to a server, a request for calling a third device.

The request for calling the third device carries an identifier of the first device and an identifier of the third device. Optionally, the request may further carry a service identifier, for example, an audio call service or a video call service.

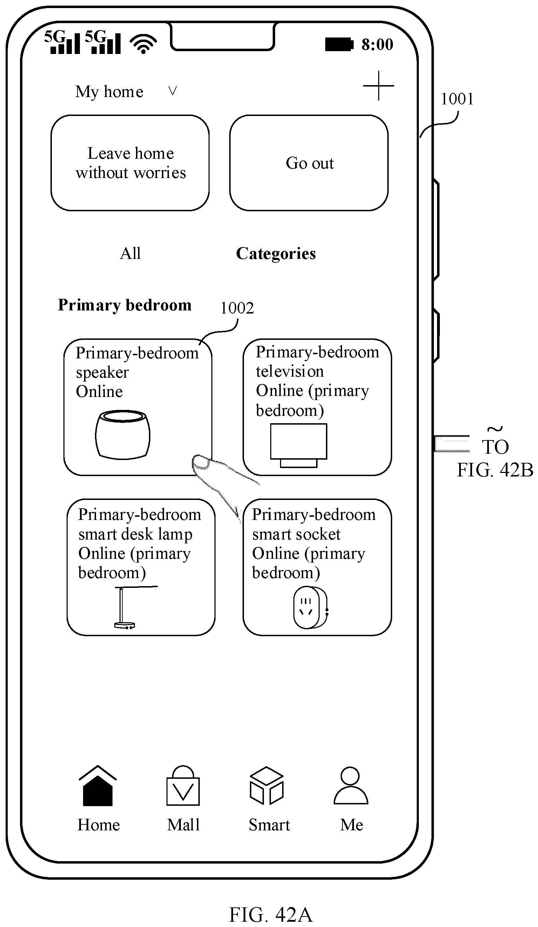

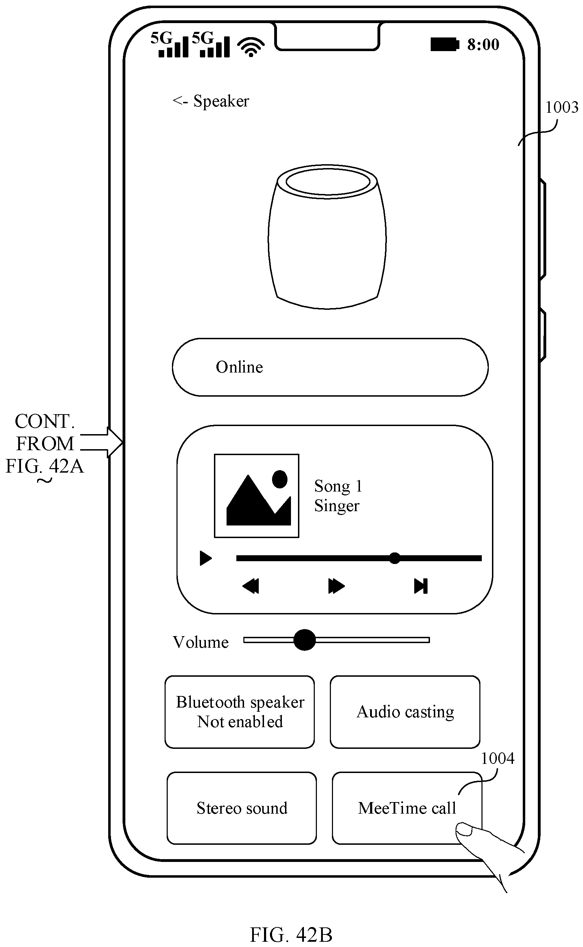

For example, the first device initiates, by using, for example, a smart home application, the request for calling the third device. For example, as shown in FIG. 42A and FIG. 42B, a mobile phone (namely, the first device) displays a device list interface 1001 of a smart home. In response to detecting that a user operates an icon 1002 of a smart speaker (namely, the third device) in a primary bedroom on the interface 1001, the mobile phone enters a control interface 1003 of the smart speaker. Further, in response to detecting that the user operates a control 1004 of "MeeTime call" on the control interface 1003 of the smart speaker, the mobile phone initiates, to a server for the smart home application, a request for calling the smart speaker.

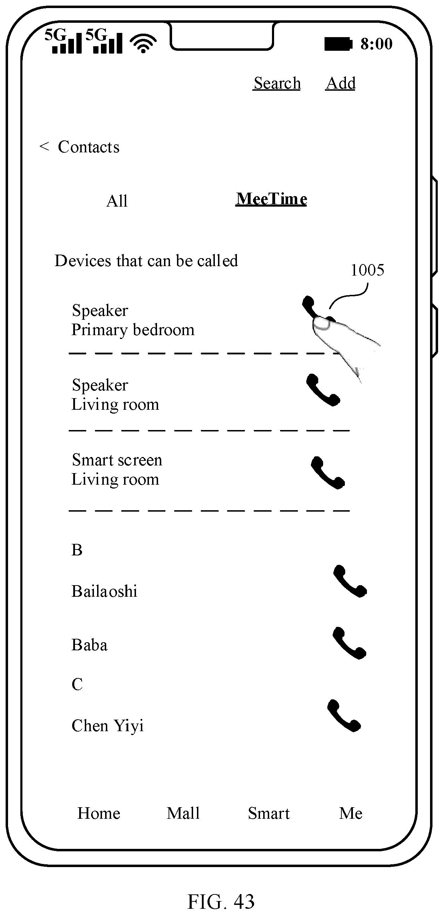

For another example, the first device may alternatively initiate, by using a MeeTime call function in a contact application, the request for calling the third device. For example, as shown in FIG. 43, a mobile phone (namely, the first device) displays a contact list interface of the contact application. In response to detecting that the user operates an icon 1005 of a smart speaker (namely, the third device) in a primary bedroom on the contact list interface, the mobile phone initiates, to a server for the smart home application, a request for calling the smart speaker.

S4102: The server sends, to the hub device, the request for calling the third device.

The hub device is a hub device, for example, a router, in a home network in which the third device is located.

It should be noted that the server herein may be one server or a plurality of servers. A quantity of servers is not limited in embodiments of this application. The server determines, based on the identifier of the third device carried in the request for calling the third device by the first device, the home network in which the third device is, and forwards, to the hub device in the home network, the call request for calling the third device by the first device.

S4103: The hub device detects a human body position based on a whole-house sensing system, and determines a second device based on the detected human body position and according to a preset policy.

The preset policy includes but is not limited to: preferably selecting, as the second device, a device that is closest to a human body; when a plurality of human bodies are detected, preferably selecting, as the second device, a device that is closest to the plurality of human bodies; preferably selecting, as the second device, a device that is in a room (for example, a living room) that is closest to the first device and in which there is a person; when there is a person resting in the plurality of detected human bodies, avoiding a device, that is near the resting person, when the second device is selected; and the like. It should be noted that specific content of the preset policy is not limited in embodiments of this application. In addition, the preset policy may be a system default policy, or may be set by the user.

For example, the whole-house sensing system includes the first electronic device 100 in (a) in FIG. 1, and is configured to locate the user and/or obtain a position of each device in a whole house, and the like. As described above, some first electronic devices 100 each are equipped with a UWB module, and may detect, by using the UWB module, information such as a position of a device in each room in the whole house, an orientation and a distance between devices, a display area of a display device (for example, a large-screen device), and an audio playing area of an audio playing device (for example, a smart speaker). Some first electronic devices 100 each are equipped with a millimeter-wave radar, and may further detect, by using the millimeter-wave radar, a position of a human body in any room in the whole house, a movement track of the human body in the whole house, a distance and an orientation between the human body and the device, and the like.

In some embodiments of this application, after receiving the request for calling the third device, when determining, based on the human body position detected by the whole-house sensing system, that there is no person within a preset distance range of the third device, the hub device may determine, according to the preset policy, another device in the home network as the second device, to perform incoming call notification. For a method for selecting the second device, refer to the descriptions of the related content in step S4002. Details are not described herein again. S4104: The hub device sends, to the second device, the request for calling the second device. S4105: After receiving the call request, the second device rings to give a prompt.

S4106: The second device receives an operation of indicating, by a user, to answer.

S4107: The second device sends an answer instruction to the hub device.

S4108: The hub device forwards the answer instruction to the server.

S4109: The server establishes a call link between the first device and the second device.

Then, the user of the first device and the user of the second device may make a call through the established call link.

It should be noted that, in some other embodiments, for example, when the user initiates a call to a group (for example, a home group) by using, for example, "MeeTime call", the foregoing technical solution may also be used to determine, based on human body position information and device position information that are detected by the whole-house sensing system, a device that eventually rings and performs a call task.

In conclusion, compared with that in the conventional technology, when a device (for example, the large-screen device or the smart speaker) in the home network receives an incoming call, only the device rings. In this case, if the user is far away from the device, the user may miss the notification, or the user needs to quickly move near the device to process the incoming call, resulting in poor user experience. In addition, if the device is offline, the current call fails.

However, in this embodiment of this application, when receiving a call to the device in the home network, the hub device can determine, based on the human body position detected by the whole-house sensing system, that there is no person around the called device, and may choose to switch the call to another appropriate device, that is located near a person, for ringing and the call with the first device. It can be learned that, in one aspect, the second device is selected based on the detected human body position, and therefore there is a user located near the second device. In this case, the user can receive the ringing reminder in time, which also facilitates processing by the user. In another aspect, the hub device selects the second device from all the devices in the whole house. Therefore, when a device in the whole house is disconnected from the hub device or the signal is poor, the hub device may select another device for ringing and the call with the first device.

**Embodiment 4: Calling between family members in a whole house**

In a home scenario, communication between family members in different rooms is a highfrequency scenario. A current voice call function based on social software and a call function provided by an operator may be used to implement point-to-point calling. However, if a user is far away from a specific device (for example, a mobile phone) on which the social software or call software is installed, there is still a problem that the user cannot answer the call. Therefore, embodiments of this application provide a method for calling a family member in a whole house, to detect a position of a called family member based on a whole-house sensing system, and select an appropriate device for a call based on the position of the called family member, so as to improve a calling success rate.

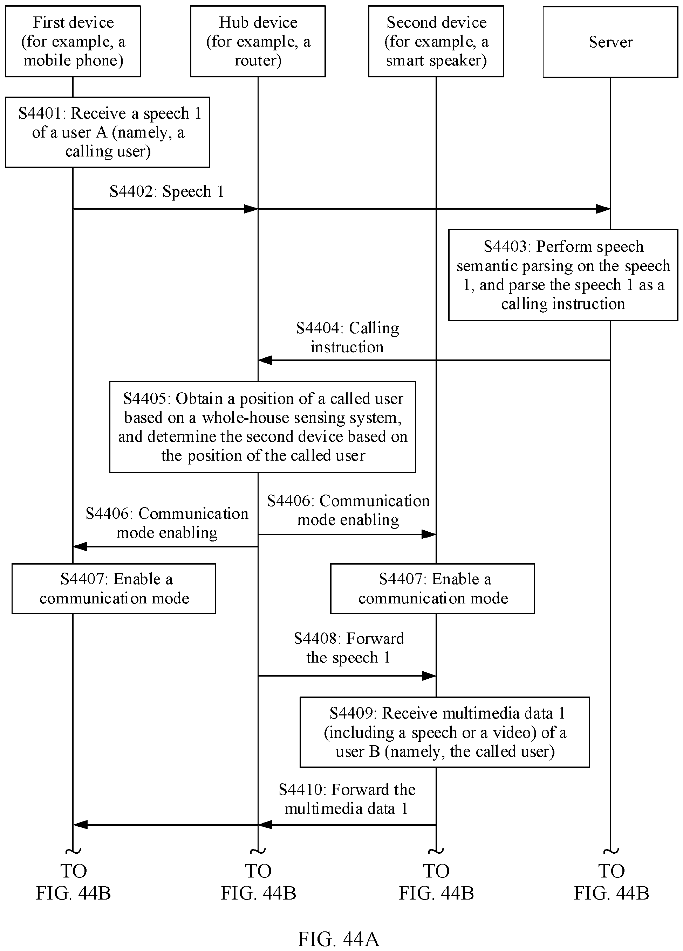

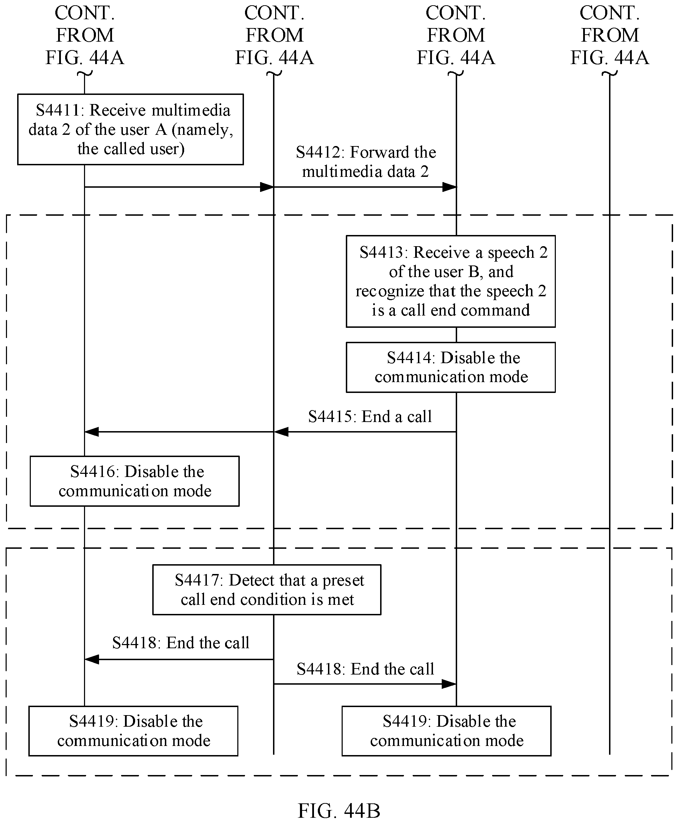

FIG. 44A and FIG. 44B are a schematic flowchart of a method for a call between family members in a whole-house scenario according to an embodiment of this application. The method includes the following steps.

S4401: A first device receives a speech 1 of a user A.

For example, when wanting to call another family member in a home, the user A may first speak a wake-up word (for example, "Hi, Celia"). The wake-up word may be used to wake up the first device. Then, the user A continues to speak the speech 1. The speech 1 includes a family member calling command (for example, "Xiao Ming, it is time to eat"). The first device collects the speech 1 spoken by the user A. Alternatively, the user A directly speaks the speech 1. The speech 1 includes a wake-up word and a family member calling command (for example, "Hi, Celia, call Xiao Ming to eat"). The family member calling command includes one or more specific called family members (for example, "Xiao Ming, it is time to eat" or "Mom and Dad, it is time to eat"), or a called family member who is currently at home (for example, "It is time to eat"), without specifying a specific family member.

In some embodiments, there are two or more devices near the user A that each have a voice function (a voice collection function and a voice playing function), and when the wake-up word spoken by the user A can wake up the two or more devices, one of the two or more woken-up devices may be determined as the first device based on a distributed collaboration technology, to continue collecting a speech of the user A. For example, a device whose sound signal has high strength and/or high clarity is selected, as the first device, from the two or more woken-up devices by comparing strength and/or clarity of collected sound signals, to improve quality of the speech 1 collected by the first device, and improve call quality.

S4402: The first device sends the speech 1 to a server by using a hub device.

The hub device may be, for example, the hub device 200 in (a) in FIG. 1, and is configured to centrally manage all devices in a home network. In some examples, a device that is powered on for a long time and has a specific storage capability and computing capability may be selected as the hub device in the home network, for example, a router, a mobile phone, a smart speaker, or a set-top box. In some other embodiments, the hub device may alternatively be the first device or the second device. For example, the first device or the second device is equipped with the hub apparatus, and the hub apparatus is configured to centrally manage all devices in a home network. In this case, interaction between the first device and the hub device or interaction between the second device and the hub device may be considered as interaction between different modules in a same device.

The first device sends the collected speech 1 to the hub device. The hub device forwards the speech 1 to the server, so that the server performs speech semantic recognition on the speech 1, to recognize a user intention.

S4403: The server performs speech semantic parsing on the speech 1, and parses the speech 1 as a calling instruction.

There may be one or more servers. After receiving the speech 1, the server performs speech recognition on the speech 1, and recognizes the speech 1 as a text. Then, semantic parsing is performed on the recognized text, to recognize the user intention corresponding to the speech 1, and then a speech skill corresponding to the user intention may be invoked.

For example, the server may specifically include an intention recognition server and a smart home application server. In this case, after performing speech semantic recognition on the speech 1, the intention recognition server determines that the user intention corresponding to the speech 1 is calling the family member. In this case, the intention recognition server sends the user intention corresponding to the speech 1 to the smart home application server. The smart home application server determines that the user intention is a family member calling instruction.

Optionally, the family member calling instruction includes an identifier of the called family member. The identifier of the called user may be a name, a nickname, an alias, a title, or the like of the called user.

Alternatively, the identifier of the called family member in the family member calling instruction may be set by default. This means that the calling instruction is to call all family members other than the calling user in the family.

S4404: The server forwards the calling instruction to the hub device.

S4405: The hub device obtains a position of the called user based on a whole-house sensing system, and determines the second device based on the position of the called user.

The whole-house sensing system includes the first electronic device 100 in (a) in FIG. 1, and is configured to locate a user and/or obtain a position of each device in a whole house, and the like. As described above, some first electronic devices 100 each are equipped with a UWB module, and may detect, by using the UWB module, information such as a position of a device in each room in the whole house, an orientation and a distance between devices, a display area of a display device (for example, a large-screen device), and an audio playing area of an audio playing device (for example, a smart speaker). Some first electronic devices 100 each are equipped with a millimeter-wave radar, and may further detect, by using the millimeter-wave radar, a position of a human body in any room in the whole house, a movement track of the human body in the whole house, a distance and an orientation between the human body and the device, and the like.

In addition, in this embodiment of this application, the hub device further maintains information about each family member in the family. The information about each family member includes a name, a nickname, an alias, a title, a facial feature (or an iris feature, or the like), a voiceprint feature, and the like of each family member. In an example, the user may preconfigure the information about each family member, for example, input the name, the nickname, the alias, the title, a face image (or an eye image), and a speech of the family member. The hub device or another device performs feature recognition on the input face image (or the eye image or the like) to obtain the facial feature (or an iris feature or the like), or performs feature recognition on the input speech to obtain the voiceprint feature or the like. In another example, the hub device may alternatively collect facial feature (or an iris feature) recognized from a face image (or an eye image) that is of the family member and that is collected by each device having a camera apparatus in the home, and collect a voiceprint feature recognized from a speech collected by each device having a microphone apparatus in the home, and user identity information (such as a name, a nickname, and a title like) extracted from the speech. Then, the hub device learns various types of collected information about each family member, to recognize the family member corresponding to the information about each family member, that is, establishes a correspondence between each family member and the family member information.

In some embodiments of this application, when the calling instruction received by the hub device includes a identifier of the specific called family member, the hub device may first obtain one or more human body positions in a whole house based on the whole-house sensing system, and then, recognize a position of the called family member based on a face image collected by a device having a camera apparatus in a room in which the one or more human body positions are located, or recognize a position of the called family member is based on a voice collected by a device having a microphone in a room in which the one or more human body positions are located. Then, a device having a voice function near the position of the called family member is recognized as the second device based on a UWB system. For example, a device closest to the position of the called family member may be selected and determined as the second device. Currently, the second device may alternatively be determined according to another policy. In other words, a specific method for selecting, as the second device, one device from a plurality of devices that have the speech function in a room is not specifically limited.

In some other embodiments of this application, when the calling instruction received by the hub device includes no identifier of the specific called family member, the hub device may first obtain one or more human body positions in the whole house based on the whole-house sensing system. Then, a device having the speech function near the one or more human body positions is recognized as the second device based on a UWB system. When the one or more human body positions are located in different rooms, one second device is determined in each room. When two or more human body positions in the one or more human body positions are located in a same room, one second device is determined in the room. For example, a device closest to one or more human body positions may be selected and determined as the second device. Currently, the second device may alternatively be determined according to another policy. In other words, a specific method for selecting, as the second device, a device from a plurality of devices that have a speech function in a room is not specifically limited.

S4406: The hub device sends a communication mode enabling message to the second device, and the hub device sends the communication mode enabling message to the first device.

For example, after determining the second device, the hub device separately sends the communication mode enabling message to the second device and the first device. The communication mode enabling message carries an identifier of the first device and an identifier of the second device. Optionally, the communication mode enabling message may further carry a call type, for example, a voice call or a video call. The identifier of the first device includes, for example, a MAC address and an IMEI of the first device. The identifier of the second device includes, for example, a MAC address and an IMEI of the second device.

It should be noted that the "communication mode enabling message" herein is mainly used to transfer the identifiers of the first device and the second device to each other, to facilitate subsequent transmission of multimedia data of the first device and the second device. Certainly, "communication mode enabling" herein may alternatively be expressed as another word, for example, "call mode establishing". This is not limited in embodiments of this application.

S4407: The first device enables a communication mode, and the second device enables the communication mode.

For example, after the first device enables the communication mode, the first device collects multimedia data (for example, a speech or a video) of the user in real time, and sends the collected multimedia data to the second device by using the hub device. In addition, after receiving the multimedia data from the second device, the first device directly plays the multimedia data. Similarly, after the second device enables the communication mode, the second device collects a speech or the multimedia data (for example, the speech or a video) of the user in real time, and sends the collected multimedia data to the first device by using the hub device. In addition, after receiving the multimedia data from the first device, the second device directly plays the multimedia data. To be specific, a local call between the first device and the second device is implemented, that is, the following step S4408 to step S4419 are performed.

S4408: The hub device forwards the speech 1 to the second device.

For example, when the speech 1 includes what the user A says to the user B, the hub device forwards the speech 1 to the second device. It may be understood that speech 1 may alternatively not include what the user A says to the user, and step S4408 may be omitted.

S4409: The first device receives multimedia data 1 of the user B.

For example, the first device starts to collect multimedia data (for example, a speech or an image) of the user B in real time.

S4410: The first device forwards the multimedia data 1 to the second device by using the hub device.

S4411: The second device receives multimedia data 2 of the user B.

S4412: The second device forwards the multimedia data 2 to the first device by using the hub device.

It should be noted that step S4409 and step S4410 are used to describe a process in which the second device sends the multimedia data of the user B to the first device. Step S4411 and step S4412 are used to describe a process in which the first device sends the multimedia data of the user A to the second device. An execution sequence of step S4409 and step S4410 and an execution sequence of step S4411 and step S4412 are not limited in embodiments of this application.

S4413: The second device receives a speech 2 of the user B, and recognizes that the speech 2 is a call end command.

The second device has a specific speech semantic recognition function, and may recognize whether a user speech includes the call end command. For example, when recognizing that speech 2 includes keywords such as "end", "stop", "exit", and "close", the second device may determine that the speech 2 is a call end command.

S4414: The second device disables the communication mode.

S4415: The second device sends a call end message to the first device by using the hub device.

Step S4415 may alternatively be performed before step S4414, or may be performed simultaneously. An execution sequence of step S4414 and step S4415 is not limited in embodiments of this application.

S4416: The first device disables the communication mode.

In step S4413 to step S4416, an example in which a called party (namely, the user B) initiates a call end instruction is used for description. In some other examples, a calling party (namely, the user A) may alternatively initiate a call end instruction. A method is similar. Details are not described again. In still some examples, the hub device may alternatively initiate a call end instruction. To be specific, step S4413 to step S4416 may be replaced with step S4417 to step S4419.

S4417: The hub device detects that a preset call end condition is met.

The preset condition includes but is not limited to: when the hub device detects that multimedia data that needs to be forwarded is not received again within preset duration after the multimedia data sent by the first device or the second device is forwarded; or the hub device receives a call end instruction sent by the first device or the second device, but still receives multimedia data sent by a peer end.

S4418: The hub device separately sends a call end message to the first device and the second device.

S4419: Both the first device and the second device disable the communication mode.

In step S4417 to step S4419, after detecting that the multimedia data that needs to be forwarded is not received again within the preset duration after the multimedia data sent by the first device or the second device is forwarded, the hub device can actively send the call end message to the first device and the second device. Alternatively, the hub device receives the call end instruction sent by the first device or the second device, but still receives the multimedia data sent by a peer end. In other words, if the peer end still does not end the call, the hub device can actively send the call end message to the first device and the second device. Alternatively, the hub device sends the call end message only to the peer that does not end the call.

In conclusion, when the user calls the another user in the home, the user can send a speech command to the hub device by using a nearby first device having the speech function. Then, the hub device can recognize, based on the whole-house sensing system, the position of the called user, and automatically determine, based on a position of another user, a nearby second device having the speech function. The second device and the first device jointly perform the local call between family members in the home. It can be learned that, in the technical solution provided in this embodiment of this application, the local call between the family members is no longer limited to a specific device on which social software or call software is installed. In addition, the hub device selects a nearby device for the called user based on the whole-house sensing system, to prevent the called user from missing the current call, and help the called user directly answer the call with the calling user. In addition, according to the technical solution provided in this embodiment of this application, the local call between the family members is implemented, and the server is prevented from forwarding multimedia data. This improves transmission efficiency of the multimedia data, and improves call experience of the user.

It should be noted that the methods in embodiments, the implementations, and the examples of embodiments of this application may be separately combined, and combined embodiments, implementations, or examples also fall within the protection scope of embodiments of this application. Steps or technical features in embodiments of this application may be randomly combined, and combined implementations also fall within the protection scope of embodiments of this application.

An embodiment of this application further provides an apparatus. The apparatus is included in an electronic device. The apparatus has functions of implementing behavior of the electronic device in any method in the foregoing embodiments. The function may be implemented by hardware, or may be implemented by hardware executing corresponding software. The hardware or the software includes at least one module or unit corresponding to the foregoing function, for example, a detection module or unit, a display module or unit, a determining module or unit, and a calculation module or unit.

An embodiment of this application further provides a computer storage medium, including computer instructions. When the computer instructions are run on an electronic device, the electronic device is enabled to perform any method in the foregoing embodiments.

An embodiment of this application further provides a computer program product. When the computer program product runs on a computer, the computer is enabled to perform any method in the foregoing embodiments.

An embodiment of this application further provides a graphical user interface on an electronic device. The electronic device includes a display, a camera, a memory, and one or more processors. The one or more processors are configured to execute one or more computer programs stored in the memory. The graphical user interface includes a graphical user interface displayed when the electronic device performs any method in the foregoing embodiments.

It may be understood that, to implement the foregoing functions, the foregoing device includes a corresponding hardware structure and/or software module for implementing each function. A person skilled in the art should easily be aware that, in combination with units and algorithm steps of the examples described in embodiments disclosed in this specification, embodiments of this application can be implemented by hardware or a combination of hardware and computer software. Whether a function is performed by hardware or hardware driven by computer software depends on particular applications and design constraints of the technical solutions. A person skilled in the art may use different methods to implement the described functions for each particular application, but it should not be considered that the implementation goes beyond the scope of embodiments of the present invention.

In embodiments of this application, the foregoing device may be divided into functional modules based on the foregoing method examples, for example, each functional module may be obtained through division based on each corresponding function, or two or more functions may be integrated into one processing module. The integrated module may be implemented in a form of hardware, or may be implemented in a form of a software functional module. It should be noted that, in embodiments of the present invention, division into the modules is an example, is merely logical function division, and may be other division during actual implementation.

A person skilled in the art may clearly learn from the foregoing description of the implementations that, for convenience and brevity of description, division into the foregoing functional modules is only used as an example for description. During actual application, the foregoing functions may be allocated to different function modules for implementation based on a requirement, that is, an inner structure of an apparatus is divided into different functional modules, to complete all or some of the functions described above. For a detailed working process of the foregoing system, apparatus, and unit, refer to a corresponding process in the foregoing method embodiments. Details are not described herein again.

Functional units in embodiments of this application may be integrated into one processing unit, or each of the units may exist alone physically, or two or more units are integrated into one unit. The integrated unit may be implemented in a form of hardware, or may be implemented in a form of a software functional unit.

When being implemented in the form of the software functional unit and sold or used as an independent product, the integrated unit may be stored in a computer-readable storage medium. Based on such an understanding, the technical solutions of embodiments of this application essentially, or the part contributing to the conventional technology, or all or some of the technical solutions may be implemented in a form of a software product. The computer software product is stored in a storage medium and includes several instructions for instructing a computer device (which may be a personal computer, a server, or a network device) or a processor to perform all or some of the steps of the methods described in embodiments of this application. The foregoing storage medium includes any medium that can store program code, for example, a flash memory, a removable hard disk, a read-only memory, a random access memory, a magnetic disk, or an optical disc.

The foregoing descriptions are merely specific implementations of this application, but are not intended to limit the protection scope of this application. Any variation or replacement within the technical scope disclosed in this application shall fall within the protection scope of this application. Therefore, the protection scope of this application shall be subject to the protection scope of the claims.

**Claims**

1.  A human sensing-based distributed alarm clock system, comprising a hub device, a first electronic device, and R second electronic devices, wherein any two of the hub device, the first electronic device, and any one of the R second electronic devices communicate in a wired communication or wireless communication manner; the first electronic device comprises an ultra-wide band module and a millimeter-wave radar module, and is configured to measure a human body position and positions of the R second electronic devices; and the R second electronic devices comprise a first device and a second device, and R is a positive integer greater than or equal to 1;

    the first device sends alarm start information of a first alarm clock to the hub device, and the alarm start information comprises an alarm start moment;
    after receiving the alarm start information of the first alarm clock, the hub device determines the second device from the R second electronic devices based on a human body position that is measured by the first electronic device within first preset duration before the alarm start moment of the first alarm clock and the positions of the R second electronic devices;
    the hub device sends, to the second device, a first message for performing the first alarm clock; and

the second device performs a task of the first alarm clock in response to receiving the first message.

2. The system according to claim 1, wherein that the first device sends alarm start information of a first alarm clock to the hub device comprises:
   the first device sends the alarm start information of the first alarm clock to the hub device within second preset duration before the alarm start moment of the first alarm clock.

3. The system according to claim 1 or 2, wherein the alarm start information further comprises ringing duration;

   the hub device sends, to the second device after the ringing duration starting after the hub device sends, to the second device, the first message for performing the first alarm clock, a second message for stopping performing the first alarm clock; and
   the second device stops, in response to receiving the second message, performing the task of the first alarm clock.

4. The system according to claim 1 or 2, wherein

   when the second device performs the task of the first alarm clock, the second device receives a first operation of disabling the first alarm clock by a user; and
   the second device stops, in response to receiving the first operation, performing the task of the first alarm clock.

5. The system according to claim 4, wherein
   after stopping performing the task of the first alarm clock, the second device sends, to the hub device, a third message for disabling the first alarm clock.

6. The system according to claim 5, wherein
   the hub device updates an execution status of the first alarm clock in response to receiving the third message.

7. The system according to claim 1 or 2, wherein the alarm start information further comprises a re-ringing interval, and the R second electronic devices further comprise a third device;

   when the second device performs the task of the first alarm clock, the second device receives a second operation of performing alarm clock re-ringing by a user;
   in response to receiving the second operation, the second device stops performing the task of the first alarm clock, and sends a fourth message for alarm clock re-ringing to the hub device;
   after receiving the fourth message, the hub device calculates a re-ringing moment of the first alarm clock based on the re-ringing interval;
   the hub device determines the third device from the R second electronic devices based on a human body position that is measured by the first electronic device within first preset duration before the re-ringing moment of the first alarm clock and the positions of the R second electronic devices;
   the hub device sends, to the third device, a fifth message for performing the first alarm clock; and
   the third device performs the task of the first alarm clock in response to receiving the fifth message.

8. The system according to claim 1 or 2, wherein the alarm start information further comprises ringing duration, and the R second electronic devices further comprise a fourth device;

   the hub device determines, based on a real-time human body position that is measured by the first electronic device and the positions of the R second electronic devices, the fourth device from the R second electronic devices within the ringing duration starting after the hub device sends, to the second device, the first message for performing the first alarm clock;
   the hub device sends, to the second device, a sixth message for stopping performing the first alarm clock, and sends, to the fourth device, a seventh message for performing the first alarm clock; and
   the second device stops, in response to receiving the sixth message, performing the task of the first alarm clock, and the fourth device performs the task of the first alarm clock in response to receiving the seventh message.

9. The system according to claim 8, wherein

   the hub device sends, to the fourth device after the ringing duration starting after the first message for performing the first alarm clock is sent to the second device, an eighth message for stopping performing the first alarm clock; and
the fourth device stops, in response to receiving the eighth message, performing the task of the first alarm clock.

10. A human sensing-based distributed alarm clock execution method, applied to a hub device, wherein the hub device separately communicates with a first electronic device and R second electronic devices in a wired communication or wireless communication manner; the first electronic device comprises an ultra-wide band module and a millimeter-wave radar module, and is configured to measure a human body position and positions of the R second electronic devices; and the R second electronic devices comprise a first device and a second device, and R is a positive integer greater than or equal to 1;

the hub device receives alarm start information of a first alarm clock sent by the first device, and the alarm start information comprises an alarm start moment;
the hub device determines the second device from the R second electronic devices based on a human body position that is measured by the first electronic device within first preset duration before the alarm start moment of the first alarm clock and the positions of the R second electronic devices; and
the hub device sends, to the second device, a first message for performing the first alarm clock.

11. The method according to claim 10,wherein the ringing information further comprises ringing duration; and
the hub device sends, to the second device after the ringing duration starting after the hub device sends, to the second device, the first message for performing the first alarm clock, a second message for stopping performing the first alarm clock.

12. The method according to claim 10, wherein
the hub device receives a third message that is for disabling the first alarm clock and that is sent by the second device, and the hub device updates an execution status of the first alarm clock.

13. The method according to claim 10, wherein the alarm start information further comprises a re-ringing interval, and the R second electronic devices further comprise a third device;

the hub device receives a fourth message for alarm clock re-ringing sent by the second device;
the hub device calculates a re-ringing moment of the first alarm clock based on the re-ringing interval;
the hub device determines the third device from the R second electronic devices based on a human body position that is measured by the first electronic device within first preset duration before the re-ringing moment of the first alarm clock and the positions of the R second electronic devices; and
the hub device sends, to the third device, a fifth message for performing the first alarm clock.

14. The method according to claim 10, wherein the ringing information further comprises ringing duration, and the R second electronic devices further comprise a fourth device;

the hub device determines, based on a real-time human body position that is measured by the first electronic device and the positions of the R second electronic devices, the fourth device from the R second electronic devices within the ringing duration starting after the hub device sends, to the second device, the first message for performing the first alarm clock; and
the hub device sends, to the second device, a sixth message for stopping performing the first alarm clock, and sends, to the fourth device, a seventh message for performing the first alarm clock.

15. The method according to claim 14, wherein
the hub device sends, to the fourth device after the ringing duration starting after the first message for performing the first alarm clock is sent to the second device, an eighth message for stopping performing the first alarm clock.

16. A human sensing-based device registration system, comprising a hub device, a first electronic device, R second electronic devices, and a server, wherein the first electronic device comprises an ultra-wide band module and a millimeter-wave radar module, and is configured to measure a human body position and positions of the R second electronic devices; and the R second electronic devices comprise a first device and a second device, and R is a positive integer greater than or equal to 1;

the first device receives a first operation of adding the second device;
the first device sends network configuration information to the second device, and sends a first registration request for the second device to the hub device;

after receiving the first registration request for the second device, the hub device determines room information or area information of the second device based on the human body position and the positions of the R second electronic devices that are measured by the first electronic device;

the hub device sends a second registration request for the second device to the server, and the second registration request comprises the room information or the area information of the second device; and

the server registers the second device based on the second registration request.

17. The system according to claim 16, wherein that the hub device determines room information or area information of the second device based on the human body position and the positions of the R second electronic devices that are measured by the first electronic device comprises:

the hub device determines, as the room information or the area information of the second device, a room or an area in which the human body position measured by the first electronic device is located; or

the first device comprises an ultra-wide band module, and the hub device determines the room information or the area information of the second device based on a room or an area in which a position that is of the first device and that is measured by the first electronic device is located.

18. A human sensing-based device registration method, applied to a hub device, wherein the hub device separately communicates with a first electronic device, R second electronic devices, and a server in a wired communication or wireless communication manner; the first electronic device comprises an ultra-wide band module and a millimeter-wave radar module, and is configured to measure a human body position and positions of the R second electronic devices; and the R second electronic devices comprise a first device and a second device, and R is a positive integer greater than or equal to 1;

the hub device receives a first registration request for the second device sent by the first device;

the hub device determines room information or area information of the second device based on the human body position and the positions of the R second electronic devices that are measured by the first electronic device; and

the hub device sends a second registration request for the second device to the server, and the second registration request comprises the room information or the area information of the second device.

19. The method according to claim 18, wherein that the hub device determines room information or area information of the second device based on the human body position and the positions of the R second electronic devices that are measured by the first electronic device comprises:

the hub device determines, as the room information or the area information of the second device, a room or an area in which the human body position measured by the first electronic device is located; or

the first device comprises an ultra-wide band module, and the hub device determines the room information or the area information of the second device based on a room or an area in which a position that is of the first device and that is measured by the first electronic device is located.

20. A human sensing-based device control system, comprising a hub device, a first electronic device, R second electronic devices, and a server, wherein the first electronic device comprises an ultra-wide band module and a millimeter-wave radar module, and is configured to measure a human body position and positions of the R second electronic devices; and the R second electronic devices comprise a first device, and R is a positive integer greater than or equal to 1;

the first device sends a first request to the hub device, and the first request is for searching for device list information controlled by the first device;

after receiving the first request, the hub device determines room information of the first device based on the human body position and the positions of the R second electronic devices that are measured by the first electronic device;

the hub device sends a second request to the server, and the second request is for requesting device list information of a room in which the first device is located;

after receiving the second request, the server returns, to the hub device, the device list information of the room in which the first device is located; and

after receiving the device list information that is of the room in which the first device is located and that is returned by the server, the first device displays a device list of the room in which the first device is located.

21. The system according to claim 20, wherein before the first device sends the first request to the hub device, the first device receives a first operation of starting a smart home application or enabling a device search function by a user.

22. A human sensing-based device control method, applied to a hub device, wherein the hub device separately communicates with a first electronic device, R second electronic devices, and a server in a wired communication or wireless communication manner; the first electronic device comprises an ultra-wide band module and a millimeter-wave radar module, and is configured to measure a human body position and positions of the R second electronic devices; and the R second electronic devices comprise a first device, and R is a positive integer greater than or equal to 1;

   the hub device receives a first request sent by the first device, and the first request is for searching for device list information controlled by the first device;
   the hub device determines room information of the first device based on the human body position and the positions of the R second electronic devices that are measured by the first electronic device;
   the hub device sends a second request to the server, and the second request is for requesting device list information of a room in which the first device is located;
   the hub device receives the device list information that is of the room in which the first device is located and that is returned by the server; and
   the hub device returns, to the first device, the device list information of the room in which the first device is located.

23. A human sensing-based intelligent reminding system, comprising a hub device, a first electronic device, and R second electronic devices, wherein the first electronic device comprises an ultra-wide band module and a millimeter-wave radar module, and is configured to measure a human body position and positions of the R second electronic devices; and the R second electronic devices comprise a first device and a second device, and R is a positive integer greater than or equal to 1;

   the first device sends a first message of a first reminder to the hub device;
   after receiving the first message of the first reminder, the hub device determines the second device from the R second electronic devices based on the human body position and the positions of the R second electronic devices that are measured by the first electronic device;
   the hub device sends a second message of the first reminder to the second device; and
   the second device performs the first reminder after receiving the second message.

24. The system according to claim 23, wherein
   before the first device sends the first message of the first reminder to the hub device, the first device receives an operation of triggering a doorbell by a user, and the first message of the first reminder is for triggering a doorbell reminder.

25. The system according to claim 23, wherein that the first device sends a first message of a first reminder to the hub device comprises:
   the first device sends the first message of the first reminder to the hub device by using a server, and the first message of the first reminder is for calling any one of the R second electronic devices.

26. The system according to any one of claims 23 to 25, wherein that the hub device determines the second device from the R second electronic devices based on the human body position and the positions of the R second electronic devices that are measured by the first electronic device comprises:

   the hub device determines, as the second device from the R second electronic devices based on the human body position and the positions of the R second electronic devices that are measured by the first electronic device, a device that is closest to the human body position; or
   when there are a plurality of detected human body positions, the hub device determines, as the second device from the R second electronic devices based on the human body positions and the positions of the R second electronic devices that are measured by the first electronic device, a device that is closest to a central position of the plurality of human body positions; or
   the hub device determines, as the second device from the R second electronic devices based on the human body position and the positions of the R second electronic devices that are measured by the first electronic device, a device that is in a room in which there is a person and that is closest to the first device; or
   the hub device determines, as the second device from the R second electronic devices based on the human body position and the positions of the R second electronic devices that are measured by the first electronic device, a device in a room in which no person rests.

27. A human sensing-based intelligent reminding method, applied to a hub device, wherein the hub device separately communicates with a first electronic device and R second electronic devices in a wired communication or wireless communication manner; the first electronic device comprises an ultra-wide band module and a millimeter-wave radar module, and is configured to measure a human body position and positions of the R second electronic devices; and the R second electronic devices comprise a first device and a second device, and R is a positive integer greater than or equal to 1;

   after receiving a first message that is of a first reminder and that is sent by the first device, the hub device determines the second device from the R second electronic devices based on the human body position and the positions of the R second electronic devices that are measured by the first electronic device; and the hub device sends a second message of the first reminder to the second device, and the second message indicates the second device to perform the first reminder.

28. The method according to claim 27, wherein the first message of the first reminder is for triggering a doorbell reminder.

29. The method according to claim 27, wherein that the hub device receives the first message that is of the first reminder and that is sent by the first device comprises:
   the hub device receives the first message that is of the first reminder and that is sent by the first device by using a server, and the first message of the first reminder is for calling any one of the R second electronic devices.

30. The method according to any one of claims 27 to 29, wherein that the hub device determines the second device from the R second electronic devices based on the human body position and the positions of the R second electronic devices that are measured by the first electronic device comprises:

   the hub device determines, as the second device from the R second electronic devices based on the human body position and the positions of the R second electronic devices that are measured by the first electronic device, a device that is closest to the human body position; or
   when there are a plurality of detected human body positions, the hub device determines, as the second device from the R second electronic devices based on the human body positions and the positions of the R second electronic devices that are measured by the first electronic device, a device that is closest to a central position of the plurality of human body positions; or
   the hub device determines, as the second device from the R second electronic devices based on the human body position and the positions of the R second electronic devices that are measured by the first electronic device, a device that is in a room in which there is a person and that is closest to the first device; or
   the hub device determines, as the second device from the R second electronic devices based on the human body position and the positions of the R second electronic devices that are measured by the first electronic device, a device in a room in which no person rests.

31. A human sensing-based indoor calling system, comprising a hub device, a first electronic device, R second electronic devices, and a server, wherein the first electronic device comprises an ultra-wide band module and a millimeter-wave radar module, and is configured to measure a human body position and positions of the R second electronic devices; and the R second electronic devices comprise a first device and a second device, and R is a positive integer greater than or equal to 1;

   the first device receives a first speech of a first user, and the first speech is for calling a second user;
   the first device sends the first speech of the first user to the server by using the hub device;
   the server performs speech semantic recognition on the first speech of the first user, and recognizes the first speech as a first calling instruction;
   the server sends the first calling instruction to the hub device;
   after receiving the first calling instruction, the hub device determines, as the second device from the R second electronic devices based on the human body position and the positions of the R second electronic devices that are measured by the first electronic device, a device located near the second user; and
   the hub device forwards, to the second device, a second speech or video of the first user sent by the first device, and forwards, to the first device, a third speech or video of the second user sent by the second device.

**32.** The system according to claim 31, wherein

when detecting that a call end condition is met, the hub device sends a call end message to the first device and/or the second device; and
the call end condition comprises that the call end message sent by the first device or the second device is received or the hub device receives no new speech or video after preset duration starting after a speech or a video of a user is forwarded.

**33.** The system according to claim 31 or 32, wherein
when the first speech does not comprise information about the second user, that the hub device determines, from the R second electronic devices based on the human body position and the positions of the R second electronic devices that are measured by the first electronic device, that the second user is located near the second device comprises:
the hub device determines, based on the human body position that is measured by the first electronic device, the detected human body position as a position of the second user, and determines, as the second user from the R second electronic devices based on the positions of the R second electronic devices, a device located near the second user.

**34.** A human sensing-based indoor calling method, applied to a hub device, wherein the hub device separately communicates with a first electronic device, R second electronic devices, and a server in a wired communication or wireless communication manner; the first electronic device comprises an ultra-wide band module and a millimeter-wave radar module, and is configured to measure a human body position and positions of the R second electronic devices; and the R second electronic devices comprise a first device and a second device, and R is a positive integer greater than or equal to 1;

the hub device receives a first calling instruction sent by the server, the first calling instruction is obtained by the server based on a first speech sent by the first device, and the first speech is used by a first user to call a second user;
the hub device determines, as the second device from the R second electronic devices based on the human body position and the positions of the R second electronic devices that are measured by the first electronic device, a device located near the second user; and
the hub device forwards, to the second device, a second speech or video of the first user sent by the first device, and forwards, to the first device, a third speech or video of the second user sent by the second device.

**35.** The method according to claim 34, wherein

when detecting that a call end condition is met, the hub device sends a call end message to the first device and/or the second device; and
the call end condition comprises that the call end message sent by the first device or the second device is received or the hub device receives no new speech or video after preset duration starting after a speech or a video of a user is forwarded.

**36.** The method according to claim 34 or 35, wherein

when the first speech does not comprise information about the second user, that the hub device determines, from the R second electronic devices based on the human body position and the positions of the R second electronic devices that are measured by the first electronic device, that the second user is located near the second device comprises:
the hub device determines, based on the human body position that is measured by the first electronic device, the detected human body position as a position of the second user, and determines, as the second user from the R second electronic devices based on the positions of the R second electronic devices, a device located near the second user.

**37.** An electronic device, comprising a processor, a memory, and a communication module, wherein the memory and the communication module are coupled to the processor, the memory is configured to store computer program code, the computer program code comprises computer instructions, and when the processor reads the computer instructions from the memory, the electronic device is enabled to perform the method according to any one of claims 10 to 15, 18, 19, 22, 27 to 30, and 34 to 36.

38. A computer-readable storage medium, comprising computer instructions, wherein when the computer instructions are run on an electronic device, the electronic device is enabled to perform the method according to any one of claims 10 to 15, 18, 19, 22, 27 to 30, and 34 to 36.

(a)

(b)

FIG. 1

First electronic device 100

FIG. 2

Hub device 200

FIG. 3

Second electronic device 300

Antenna                              Antenna

| Wireless communication module 360 (Wi-Fi/Bluetooth/ZigBee or the like) | | UWB module 350 |

| Loudspeaker 370A | | | | Memory 320 |
| Receiver 370B | Audio module 370 | Processor 310 | | USB interface 330 |
| Microphone 370C | | | | Display 380 |
| Headset jack 370D | | | | IMU module 390 |

| Power supply module 340 |

FIG. 4

FIG. 5A

FIG. 5B

FIG. 6

FIG. 7(a)

FIG. 7(b)

FIG. 7(c)

FIG. 7(d)

FIG. 7(e)

Transmit antenna 1

Receive     Receive
antenna 0   antenna 2

Receive     Receive     Transmit           Transmit
antenna 1   antenna 3   antenna 0          antenna 2

(a)

Receive antenna 0

Receive antenna 1

Receive antenna 2

Receive antenna 3

Transmit
antenna 0

Transmit
antenna 1

Transmit
antenna 2

(b)

FIG. 8

$Z_e$ axis

Antenna 2

$X_e$ axis

$O_e$

Antenna 0  Antenna 1

$Y_e$ axis    L-shaped antenna structure

(a)

$Z_e$ axis

Antenna 2

$X_e$ axis

$O_e$

Antenna 0  Antenna 1

$Y_e$ axis    Triangular antenna structure

(b)

(c)

FIG. 9

FIG. 10

FIG. 11

FIG. 12(a)

FIG. 12(b)

FIG. 12(c)

FIG. 12(d) (CONTINUED)

FIG. 12(e) (CONTINUED)

FIG. 12(f) (CONTINUED)

FIG. 13(a)

FIG. 13(b)

FIG. 13(c) (CONTINUED)

$Z_e$ axis

100

$Y_e$ axis

$X_e$ axis

300 B

300 C

300

A

300

(a)

$Z_e$ axis

100

300

300

300

$O_e$ $Y_e$ axis

300

$X_e$ axis

300

300

300

300

300

(b)

FIG. 14

(a)

(b)

FIG. 15

(a)

(b)

FIG. 16

FIG. 17

FIG. 18

(c)

(d)

(e)

FIG. 18 (CONTINUED)

FIG. 19(a)

Two transmit antennas and four receive antennas

FIG. 19(b)-1

$Z_m$ axis

CONT.
FROM ⟹ $X_m$ axis
FIG. 19(b)-1
~

$O_m$ Receive Receive Receive Receive Receive Receive Receive Receive
antenna antenna antenna antenna antenna antenna antenna antenna
0      1      2      3      4      5      6      7

$\dfrac{\lambda_L}{2}$

$Y_m$ axis

One transmit antenna and eight receive antennas

FIG. 19(b)-2

FIG. 19(c)-1 (CONTINUED)

~
TO
FIG. 19(c)-2

FIG. 19(c)-2 (CONTINUED)

(a)

(b)

FIG. 20

S2100: A millimeter-wave radar module receives a reflected signal

S2101: The millimeter-wave radar module performs a two-dimensional fast Fourier transform on a digital difference frequency signal

Range FFT

↓

Doppler FFT

S2102: The millimeter-wave radar module obtains a distance between a reflection point and the millimeter-wave radar and a radial speed

S2103: The millimeter-wave radar module determines a signal coming direction of the reflected signal

S2104: The millimeter-wave radar module determines coordinates of the reflection point in a fourth coordinate system

S2105: The millimeter-wave radar module determines coordinates of a smart device or a user in the fourth coordinate system

S2106: The millimeter-wave radar module tracks the smart device or the user

FIG. 21

Target tracking result → S2201: Extract phase information

Range FFT (Range FFT) result →

S2201: Extract phase information → S2202: Unwrap a phase → S2203: Calculate a phase difference → S2204: Perform band-pass filtering → S2205: Estimate a range → S2206: Perform determining

FIG. 22

(a)

(b)

FIG. 23

First electronic device 100

$Z_m$ axis

Transmit antenna 1

Receive
antenna 0

Receive
antenna 2

$X_m$ axis

Receive
antenna 1

Receive
antenna 3

Transmit
antenna 0

Transmit
antenna 2

$Y_m$
axis

dz

$Z_e$ axis

Antenna 2

dy

$X_e$ axis

Antenna
0

Antenna
1

$Y_e$ axis

dx

FIG. 24

(a)

(b)

FIG. 25

S1: Establish a first coordinate system, a second coordinate system, a third coordinate system, a fourth coordinate system, and a fifth coordinate system, and obtain position information of a device, an area, and a user in the fifth coordinate system through conversion from the first coordinate system, the second coordinate system, the third coordinate system, and the fourth coordinate system to the fifth coordinate system

S2: A second electronic device performs a preset operation based on the position information of the user and position information of the second electronic device

(a)

Hub device 200
Determine, based on the coordinates of the second electronic device and the coordinates of the user, that the second electronic device executes a preset instruction

Coordinates of the second electronic device and a room or an area in which the second electronic device is located

Report coordinates of a user in a whole house and a room or an area in which the user is located

Deliver the preset instruction

First electronic device 100

| UWB module | Millimeter-wave radar module |

Second electronic device 300

(b)

FIG. 26

FIG. 27(a)

FIG. 27(b)

FIG. 27(c) (CONTINUED)

$$E(W,G)=\frac{1}{n}\sum_{i=1}^{n}\left\|q_i^{t1}-(Wq_i^{t3}+G)\right\|^2$$

FIG. 28

2901

Locate smart devices

2904

Select all

| Living room | ☑ | Dining room | ☑ | Kitchen | ☐ |

| Toilet | ☐ | Primary bedroom | ☐ | Secondary bedroom | ☐ |

2902

OK

2903

Cancel

(a)

$Y_h$ axis

C

B

Q

A

D

$O_h$

$X_h$ axis

(b)

FIG. 29

```
┌─────────────────────┐        ┌─────────────────────┐        ┌─────────────────────┐
│    First device     │        │     Hub device      │        │    Second device    │
│ (for example, a mobile│      │ (for example, a router)│     │ (for example, a smart│
│       phone)        │        │                     │        │       speaker)      │
└─────────────────────┘        └─────────────────────┘        └─────────────────────┘
```

S3001: Receive an operation A of setting an alarm clock by a user

S3002: Determine alarm start information based on the alarm clock set by the user

S3003: Report the alarm start information

S3004: Obtain position information of the user based on a whole-house sensing system, and determine the second device based on the position information of the user

S3005: When a current time point is an alarm start moment, indicate the second device to perform an alarm clock task

S3006: Perform the alarm clock task

S3007: When preset duration 1 after the alarm start moment expires, indicate the second device to end the alarm clock task

S3008: End the alarm clock task

FIG. 30

| First device (for example, a mobile phone) | Hub device (for example, a router) | Second device (for example, a smart speaker) |
|---|---|---|

S3101 to S3106

S3107: Receive an operation B of disabling an alarm clock by a user

S3108: Disable an alarm clock task

S3109: Report the operation B performed by the user

S3110: Forward the operation B performed by the user

S3111: Update a status of the alarm clock

FIG. 31

| First device (for example, a mobile phone) | Hub device (for example, a router) | Second device (for example, a smart speaker) | Third device (for example, a large-screen device) |
|---|---|---|---|

S3201 to S3206

S3207: Receive an operation C of performing alarm clock re-ringing by a user

S3208: Stop performing an alarm start task

S3209: Report the operation C performed by the user

S3210: Update a status of the alarm clock, and calculate a re-ringing moment

S3211: When a current time point is the re-ringing moment, obtain position information of the user based on a whole-house sensing system, and determine the third device based on the position information of the user

S3212: Indicate the third device to perform an alarm clock task

S3213: Perform the alarm clock task

S3214: When preset duration 2 after the re-ringing moment expires, indicate the third device to end the alarm clock task

S3215: End the alarm clock task

FIG. 32

| First device (for example, a mobile phone) | Hub device (for example, a router) | Second device (for example, a smart speaker) | Third device (for example, a smart speaker) |

S3301 toS3306

S3307: Continuously detect a user position based on a whole-house sensing system

S3308: Determine, within preset duration after an alarm start moment based on the continuously detected user position, to switch, to the third device, the second device that performs an alarm clock task

S3309: Indicate the second device to disable the alarm clock task

S3310: Indicate the third device to continue to perform the alarm clock task

S3311: Stop the alarm clock task

S3312: Perform the alarm clock task

S3313: When preset duration 1 after the alarm start moment expires, indicate the third device to end the alarm clock task

S3314: End the alarm clock task

FIG. 33

FIG. 34

(1)

(2)

FIG. 35

FIG. 36

| First device (for example, a mobile phone) | Second device (for example, a smart speaker) | Hub device (for example, a router) | Server |
|---|---|---|---|

S3700: Start a smart home application

S3701: Receive an operation of adding the second device by a user

S3702: Send network configuration information

S3703: Return a network configuration result

S3704: Registration request

S3705: Determine home information of the second device, and obtain room information of the second device based on a whole-house sensing system

S3706: Registration request (carrying the home information and the room information of the second device)

S3707: Return a registration result

S3708: Send the registration result

FIG. 37

| First device (for example, a mobile phone) | Hub device (for example, a router) | Second device (for example, a smart speaker) | Server |
|---|---|---|---|

S3800: Receive an operation of starting a smart home application by a user

S3801: Request device list information

S3802: Obtain room information of the first device based on a whole-house sensing system

S3803: Request device list information of a room in which the first device is located

S3804: Return the device list information of the room in which the first device is located

S3805: Return the device list information of the room in which the first device is located

S3806: Display a main interface of the smart home application

S3807: Control command (carrying an identifier of the second device)

| TO FIG. 38B | TO FIG. 38B | TO FIG. 38B | TO FIG. 38B |
|---|---|---|---|

FIG. 38A

CONT.
FROM
FIG. 38A

CONT.
FROM
FIG. 38A

CONT.
FROM
FIG. 38A

CONT.
FROM
FIG. 38A

S3808: Control
command (carrying
the identifier of the
second device)

S3809: Control
command (carrying
the identifier of the
second device)

S3810: Execute the
control command

S3811:
Execution result

S3812:
Execution result

S3813:
Execution result

FIG. 38B

FIG. 39A

FIG. 39B

| First device (for example, a doorbell) | Hub device (for example, a router) | Second device (for example, a terminal with a screen) |
|---|---|---|

S4000: Receive an operation of triggering the doorbell by a user A

S4001: Trigger the doorbell

S4002: Detect human body position information based on a whole-house sensing system, and determine the second device based on the human body position information and according to a preset policy

S4003: Trigger the doorbell

S4004: Ring for reminding

S4005: Receive an operation of viewing a video by a user B

S4006: View the video

S4007: View the video

S4008: Video stream

S4009: Video stream

S4010: Receive an unlocking operation of the user B

S4011: Unlock

S4012: Unlock

FIG. 40

FIG. 41

FIG. 42A

<- Speaker

1003

Online

CONT.
FROM
FIG. 42A

Song 1
Singer

Volume

Bluetooth speaker
Not enabled

Audio casting

1004

Stereo sound

MeeTime call

FIG. 42B

Search   Add

< Contacts

All                **MeeTime**

Devices that can be called

1005

Speaker
Primary bedroom

– – – – – – – – – –

Speaker
Living room

– – – – – – – – – –

Smart screen
Living room

– – – – – – – – – –

B

Bailaoshi

Baba

C

Chen Yiyi

Home      Mall      Smart      Me

FIG. 43

| First device (for example, a mobile phone) | Hub device (for example, a router) | Second device (for example, a smart speaker) | Server |
|---|---|---|---|

S4401: Receive a speech 1 of a user A (namely, a calling user)

S4402: Speech 1

S4403: Perform speech semantic parsing on the speech 1, and parse the speech 1 as a calling instruction

S4404: Calling instruction

S4405: Obtain a position of a called user based on a whole-house sensing system, and determine the second device based on the position of the called user

S4406: Communication mode enabling

S4406: Communication mode enabling

S4407: Enable a communication mode

S4407: Enable a communication mode

S4408: Forward the speech 1

S4409: Receive multimedia data 1 (including a speech or a video) of a user B (namely, the called user)

S4410: Forward the multimedia data 1

TO FIG. 44B

TO FIG. 44B

TO FIG. 44B

TO FIG. 44B

FIG. 44A

128

CONT.
FROM
FIG. 44A

CONT.
FROM
FIG. 44A

CONT.
FROM
FIG. 44A

CONT.
FROM
FIG. 44A

S4411: Receive multimedia data 2 of the user A (namely, the called user)

S4412: Forward the multimedia data 2

S4413: Receive a speech 2 of the user B, and recognize that the speech 2 is a call end command

S4414: Disable the communication mode

S4415: End a call

S4416: Disable the communication mode

S4417: Detect that a preset call end condition is met

S4418: End the call

S4418: End the call

S4419: Disable the communication mode

S4419: Disable the communication mode

FIG. 44B

## INTERNATIONAL SEARCH REPORT

| | |
|---|---|
| International application No. | |
| | **PCT/CN2022/111863** |

**A. CLASSIFICATION OF SUBJECT MATTER**

G05B 15/02(2006.01)i; G05B 19/418(2006.01)i

According to International Patent Classification (IPC) or to both national classification and IPC

**B. FIELDS SEARCHED**

Minimum documentation searched (classification system followed by classification symbols)

G05B; H04L; HO4W

Documentation searched other than minimum documentation to the extent that such documents are included in the fields searched

Electronic data base consulted during the international search (name of data base and, where practicable, search terms used)

CNPAT, CNKI, EPODOC, WPI: 智能, 家居, 人, 用户, 位置, 定位, 距离, 毫米波, 超宽带, 闹铃, 提醒, 注册, smart, intelligent, home, household, human, body, position, locat+, distance, millimeter w wave, UWB, alart, warning, registration

**C. DOCUMENTS CONSIDERED TO BE RELEVANT**

| Category* | Citation of document, with indication, where appropriate, of the relevant passages | Relevant to claim No. |
|---|---|---|
| A | CN 105049329 A (HANGZHOU MUSHAO SCIENCE & TECHNOLOGY CO., LTD.) 11 November 2015 (2015-11-11)<br>description, paragraphs [0028]-[0041], and figures 1-2 | 1-38 |
| A | CN 106909396 A (YULONG COMPUTER TELECOMMUNICATION SCIENTIFIC (SHENZHEN) CO., LTD.) 30 June 2017 (2017-06-30)<br>entire document | 1-38 |
| A | CN 107395469 A (HISENSE GROUP CO., LTD.) 24 November 2017 (2017-11-24)<br>entire document | 1-38 |
| A | CN 105208510 A (XIAOMI INC.) 30 December 2015 (2015-12-30)<br>entire document | 1-38 |
| A | CN 105785945 A (MIDEA GROUP CO., LTD.) 20 July 2016 (2016-07-20)<br>entire document | 1-38 |
| A | KR 20180035292 A (DONG YEON SYSTEM CO., LTD.) 06 April 2018 (2018-04-06)<br>entire document | 1-38 |

☐ Further documents are listed in the continuation of Box C. ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **09 October 2022** | **20 October 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2022/111863**

| Patent document cited in search report | | | Publication date (day/month/year) | Patent family member(s) | | | Publication date (day/month/year) |
|---|---|---|---|---|---|---|---|
| CN | 105049329 | A | 11 November 2015 | None | | | |
| CN | 106909396 | A | 30 June 2017 | None | | | |
| CN | 107395469 | A | 24 November 2017 | None | | | |
| CN | 105208510 | A | 30 December 2015 | CN | 105208510 | B | 30 November 2018 |
| CN | 105785945 | A | 20 July 2016 | None | | | |
| KR | 20180035292 | A | 06 April 2018 | KR | 101859715 | B1 | 29 June 2018 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202111243352

- CN 202110872916

**Non-patent literature cited in the description**

- **QIN YONGYUAN.** book Inertial Navigation. Beijing: Science Press, May 2006